(19) Europäisches Patentamt
European Patent Office
Office européen des brevets

(11) EP 2 688 556 B1

(12) # EUROPEAN PATENT SPECIFICATION

(45) Date of publication and mention of the grant of the patent:
**06.05.2015 Bulletin 2015/19**

(21) Application number: **12717468.8**

(22) Date of filing: **22.03.2012**

(51) Int Cl.:
***A61K 9/20*** *(2006.01)* ***A61K 9/28*** *(2006.01)*
***A61K 47/34*** *(2006.01)* ***A61K 47/10*** *(2006.01)*
***A61K 31/485*** *(2006.01)*

(86) International application number:
**PCT/IB2012/000595**

(87) International publication number:
**WO 2012/131463 (04.10.2012 Gazette 2012/40)**

(54) **CONTROLLED RELEASE PHARMACEUTICAL DOSAGE FORMS**

RETARD-DARREICHUNGSFORMEN

FORMES PHARMACEUTIQUES À LIBÉRATION CONTRÔLÉE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(30) Priority: **25.03.2011 US 201161467824 P**

(43) Date of publication of application:
**29.01.2014 Bulletin 2014/05**

(73) Proprietor: **Purdue Pharma L.P.**
**Stamford, CT 06901-3431 (US)**

(72) Inventor: **HUANG, Haiyong Hugh**
**Princeton, Junction, NJ 08550 (US)**

(74) Representative: **Ehlich, Eva Susanne**
**Maiwald Patentanwalts GmbH**
**Elisenhof**
**Elisenstraße 3**
**80335 München (DE)**

(56) References cited:
**EP-A1- 1 897 545** **WO-A1-02/36099**
**WO-A1-2006/002884**

- **HARIHARAN M AND GUPTA V K: "A novel compression-coated tablet dosage form", PHARMACEUTICAL TECHNOLOGY, ADVANSTAR COMMUNICATIONS,INC, US , no. YEARBOOK 2001 1 January 2001 (2001-01-01), pages 14-19, XP002678748, ISSN: 1543-2521 Retrieved from the Internet: URL:http: //www.pharmtech.com/pharmtech/dat a/ articlestandard/pharmtech/512001/5072/ar ticle.pdf**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 688 556 B1

## Description

### TECHNICAL FIELD OF THE INVENTION

The present invention relates to controlled release pharmaceutical dosage forms, for example to a tamper resistant controlled release dosage form including an opioid analgesic, essentially following a zero order release rate. The present invention further relates to processes of manufacture of these dosage forms, uses thereof as well as methods of treatment.

### BACKGROUND OF THE INVENTION

Controlled release formulations aim at achieving a release of an active agent contained therein starting at a predetermined time-point and extending over a necessary period of time in order to provide for a preferred concentration of the active agent in the plasma of patients and to achieve a therapeutic effect for an extended period of time. There are medical conditions requiring the release of the active agent at a constant rate to maintain plasma levels of said active agent in the therapeutic range, thereby avoiding plasma level fluctuations characteristic of conventionally administered dosage forms in a multidose regimen. Therefore, a need exists in the art for pharmaceutical oral dosage forms releasing active agents essentially according to a zero order mode. This is in particular true for certain dosage forms comprising an opioid analgesic as an active agent.

Furthermore pharmaceutical products, in particular pharmaceutical products comprising an opioid analgesic, are sometimes the subject of abuse. For example, a particular dose of opioid agonist may be more potent when administered parenterally as compared to the same dose administered orally. Some formulations can be tampered with to provide the opioid agonist contained therein for illicit use. Controlled release opioid agonist formulations are sometimes crushed, or subject to extraction with solvents (e.g., ethanol) by drug abusers to provide the opioid contained therein for immediate release upon oral or parenteral administration. Controlled release opioid agonist dosage forms which can liberate a portion of the opioid upon exposure to ethanol, can also result in a patient receiving the dose more rapidly than intended if a patient disregards instructions for use and concomitantly uses alcohol with the dosage form.

There continues to exist a need in the art for pharmaceutical oral dosage forms comprising an active agent, in particular an opioid analgesic, without significantly changed release properties when in contact with alcohol and/or with resistance to crushing and which provide essentially zero order release.

### OBJECTS AND SUMMARY OF THE INVENTION

It is an object of the present invention to provide solid oral extended release pharmaceutical dosage forms comprising an active agent, wherein the active agent is released essentially following a zero order mode.

It is a further object of the present invention to provide solid oral extended release pharmaceutical dosage forms comprising an active agent such as an opioid analgesic which are tamper resistant.

It is a further object of the present invention to provide solid oral extended release pharmaceutical dosage forms comprising an active agent such as an opioid analgesic which are resistant to crushing.

It is a further object of the present invention to provide solid oral extended release pharmaceutical dosage forms comprising an active agent such as an opioid analgesic which are resistant to alcohol extraction and dose dumping when concomitantly used with or in contact with alcohol.

The above objectives and others are attained by virtue of the present invention as in particular described by the following embodiments relating to dosage forms and the respective manufacturing processes as well as the uses thereof.

In one embodiment, the invention concerns a solid oral extended release pharmaceutical dosage form comprising a multi-layered extended release matrix formulation, the extended release matrix formulation comprising

(1) a first composition forming a first active agent containing layer of the extended release matrix formulation comprising:

(a) at least one polyethylene oxide having, based on rheological measurements, an approximate molecular weight of at least 1,000,000; and
(b) at least one active agent; and

(2) a second composition forming an active agent-free second layer of the extended release matrix formulation comprising at least one polyethylene oxide.

In one particular embodiment the second composition comprises at least one polyethylene oxide having, based on rheological measurements, an approximate molecular weight of at least 1,000,000.

In one particular embodiment the second composition comprises at least one polyethylene oxide having, based on rheological measurements, an approximate molecular weight of less than 1,000,000.

In one particular embodiment, the active agent in the solid oral extended release pharmaceutical dosage form is selected from opioid analgesics.

In one particular embodiment, the multi-layered extended release matrix formulation is a bilayer formulation.

In one particular embodiment, the multi-layered extended release matrix formulation is thermoformed or subjected to a curing step.

## BRIEF DESCRIPTION OF THE DRAWINGS

Figure 1 is a graphic illustration of multi-layered structures.

Fig. 1A) to 1E) show sandwich-type extended release matrix formulations, which comprise at least three layers, and half-sandwich-type structures comprising two layers.

Fig. 1 F) and G) show structures not covered by the present invention.

Figure 2 is a flow chart of the treatment periods of Example 7.

Figure 3 shows the mean plasma concentration versus time following the administration of Examples 1A, 1 B and 1C in the fasted state in Example 7.

Figure 4 shows the mean plasma concentration versus time following the administration of Examples 2A, 2B and 2C in the fasted state in Example 7.

Figure 5 shows the mean plasma concentration versus time following the administration of Example 1B in the fasted and fed state in Example 7.

Figure 6 shows the mean plasma concentration versus time following the administration of Example 2B in the fasted and fed state in Example 7.

## DETAILED DESCRIPTION

Herein below, the present invention will be described in more detail. At the onset various terms used herein are explained.

The term "extended release" is defined for purposes of the present invention to refer to multilayer dosage forms containing active agent, which are formulated to make the active agent available over an extended period after ingestion, thereby allowing a reduction in dosing frequency compared to a conventional dosage form (e.g. as a solution or an immediate release dosage form) containing the active agent.

The term "immediate release" is defined for the purposes of the present invention to refer to dosage forms containing active agent which are formulated to allow the active agent to be released in the gastrointestinal tract with no delay or prolongation of the dissolution or absorption of the active agent.

The term "zero-order release rate" refers to the rate of active agent release from a dosage form which is independent of the amount of active agent remaining in the dosage form, such that the rate is relatively constant over a period of time. A dosage form exhibiting zero order release rate would exhibit a relatively straight line in a graphical representation of percent active agent released versus time during that period of time. In accordance with the present invention, "a release rate essentially according to zero order release mode" is defined as a rate of release of active agent from a dosage form which is proportional within 50%, 40% or 30% to elapsed time from 2 to 12 hours, as measured by an in-vitro dissolution in a USP Apparatus 1 (basket) at 100 rpm in 900 ml simulated gastric fluid without enzymes (SGF) at 37° C. In one embodiment, the release rate is proportional within 20% to elapsed time from 2 to 12 hours. In another embodiment, the release is proportional within 50%, 40% or 30% to elapsed time from 2 to 18 hours. In another embodiment, the release rate is proportional within 20% to elapsed time from 2 to 18 hours. Proportional within a certain % (e.g. 20%) to elapsed time (e.g. 2 to 12 hours) means that such certain % (e.g. 20%) difference from the mean hourly release rate, to be calculated using the release rates during said elapsed time (e.g. 2 to 12 hours), is acceptable. The term "multilayer" means that the extended release formulations of the present invention have sandwich-type structures with at least three layers, or half-sandwich-type structures with two layers.

In the present context, the term "sandwich-type structure" designates any three-dimensional structure comprising more than two layers (see Figure 1, A) and B)). However, "sandwich-type" structures do not relate to those wherein one of the layers is completely covered, encased or surrounded by one or more other layer(s) (see, for example, Figure 1G). Also, a structure with a core encased by a shell is not within the meaning of a "sandwich-type structure".

A "half sandwich-type structure" is an arrangement of two layers (see, e.g., Figure 1 C to E), provided that one of the layers is not completely covered or surrounded by the second layer (as in Figure 1 F). Also, a structure with a core encased by a shell is not within the meaning of a "sandwich-type structure."

It should be noted that the term "layer" when used in the context of the present invention not only refers to essentially planar forms, but includes any form or shape. Figure 1D depicts two layers in a non-planar orientation in relation to each other.

The term "solid oral extended release pharmaceutical dosage form" refers to the administrable form of a pharmaceutical comprising a unit dose of active agent in extended release form such as an "extended release matrix formulation" and optionally one or more other excipients, adjuvants and/or additives conventional in the art, such as a protective coating or a capsule and the like, and optionally any other additional features or components that are useful in the dosage form.

The term "extended release matrix formulation" is defined for purposes of the present invention as a shaped solid form comprising a first and a second composition forming at least a first and a second layer, respectively, either one or both compositions comprising at least a high weight molecular weight polyethylene oxide. The "multi-layered extended release matrix formulation" comprises a first layer that comprises at least one active agent (hereinafter also referred to as an "active agent-containing layer" or "active layer"). The first layer is in direct contact with at least one other layer, e.g., at least a second layer not containing the at least one active agent of the first layer (hereinafter also referred to as "active agent-free layer" or "blocking layer"). The "active agent-containing layer" comprises the at least one active agent; the "active-agent free layer" is free of said at least one active agent. Both layers can optionally comprise one or more other active agents, retardants and/or other materials, including but not limited to low molecular weight polyethylene oxide and other adjuvants and additives conventional in the art. The active agent-containing layer is exposed to the surrounding medium, except for surface areas thereof covered by the active agent-free layer(s). Where the surface of the active-agent-containing layer is covered by an active agent-free layer, the active agent-free layer(s) prevent(s) direct access of the surrounding medium to the active agent-containing layer. The entire surface area of the active agent-containing layer will be completely exposed to the surrounding medium only once the active agent-free layer has completely dissolved. The active agent can dissolve from the surface of the active agent-containing layer exposed to the surrounding medium and, once the active agent-free layer hydrates, the active agent may also pass by diffusion through the active agent-free layer from the surface of said layer.

Unless otherwise indicated, all numerical values of molecular weights are in Daltons.

The term "high molecular weight polyethylene oxide" is defined for proposes of the present invention as having an approximate molecular weight of at least 1,000,000. For the purpose of this invention, the approximate molecular weight is based on rheological measurements. Polyethylene oxide is considered to have an approximate molecular weight of 1,000,000 when a 2% (by wt) aqueous solution of said polyethylene oxide using a Brookfield viscometer Model RVF, spindle No. 1, at 10 rpm, at 25°C shows a viscosity range of 400 to 800 mPa s (cP). Polyethylene oxide is considered to have an approximate molecular weight of 2,000,000 when a 2% (by wt) aqueous solution of said polyethylene oxide using a Brookfield viscometer Model RVF, spindle No. 3, at 10 rpm, at 25°C shows a viscosity range of 2000 to 4000 mPa s (cP). Polyethylene oxide is considered to have an approximate molecular weight of 4,000,000 when a 1% (by wt) aqueous solution of said polyethylene oxide using a Brookfield viscometer Model RVF, spindle No. 2, at 2 rpm, at 25°C shows a viscosity range of 1650 to 5500 mPa s (cP). Polyethylene oxide is considered to have an approximate molecular weight of 5,000,000 when a 1% (by wt) aqueous solution of said polyethylene oxide using a Brookfield viscometer Model RVF, spindle No. 2, at 2 rpm, at 25°C shows a viscosity range of 5500 to 7500 mPa s (cP). Polyethylene oxide is considered to have an approximate molecular weight of 7,000,000 when a 1% (by wt) aqueous solution of said polyethylene oxide using a Brookfield viscometer Model RVF, spindle No. 2, at 2 rpm, at 25°C shows a viscosity range of 7500 to 10,000 mPa s (cP). Polyethylene oxide is considered to have an approximate molecular weight of 8,000,000 when a 1% (by wt) aqueous solution of said polyethylene oxide using a Brookfield viscometer Model RVF, spindle No. 2, at 2 rpm, at 25°C shows a viscosity range of 10,000 to 15,000 mPa s (cP).

Regarding the lower molecular weight polyethylene oxides, polyethylene oxide is considered to have an approximate molecular weight of 100,000 when a 5% (by wt) aqueous solution of said polyethylene oxide using a Brookfield viscometer Model RVT, spindle No. 1, at 50 rpm, at 25°C shows a viscosity range of 30 to 50 mPa s (cP). Polyethylene oxide is considered to have an approximate molecular weight of 900,000 when a 5% (by wt) aqueous solution of said polyethylene oxide using a Brookfield viscometer Model RVF, spindle No. 2, at 2 rpm, at 25°C shows a viscosity range of 8800 to 17,600 mPa s (cP).

The term "low molecular weight polyethylene oxide" is defined for purposes of the present invention as having, based on the rheological measurements outlined above, an approximate molecular weight of less than 1,000,000.

The term "curing" or "temperature curing" is defined for the purposes of the present invention as referring to a process step wherein an elevated temperature is applied to the shaped extended release matrix formulation at atmospheric pressure.

The term "thermoforming" is defined for the purposes of the present invention as referring to a process wherein elevated temperature is applied before and/or during the shaping of the extended release matrix formulation, e.g., pressure and heat are simultaneously applied during process steps such as extrusion, injection molding, or heating during tablet pressing, e.g. by using a heated tabletting tool.

The term "direct compression" is defined for purposes of the present invention as referring to a tabletting process wherein the tablet or any other compressed dosage form is made by a process comprising the steps of dry blending the components of the formulation and compressing the dry blend to form the formulation, e.g. by using a diffusion blend and/or convection mixing process (e.g. Guidance for Industry, SUPAC-IR/MR: Immediate Release and Modified Release Solid Oral Dosage Forms, Manufacturing Equipment Addendum).

The term "bed of free flowing tablets" is defined for the purposes of the present invention as referring to a batch of tablets that are kept in motion with respect to each other as, e.g., in a coating pan set at a suitable rotation speed or in a fluidized bed of tablets. The bed of free flowing tablets preferably reduces or prevents the sticking of tablets to one another.

The term "flattening" and related terms as used in the context of the flattening of tablets or other dosage forms in accordance with the present invention means that a tablet or other dosage form is subjected to a force applied from a direction substantially perpendicular to the widest diameter of the dosage form, e.g. by applying pressure to the flat face of a tablet. The force may be applied with a carver style bench press (unless expressly mentioned otherwise) to the extent necessary to achieve the target flatness/reduced thickness. According to certain embodiments of the invention, the flattening does not result in breaking the tablet into pieces; however, edge spits and cracks may occur. The flatness may be described in terms of the thickness of the flattened tablet compared to the thickness of the non-flattened tablet, as expressed in % thickness, based on the thickness of the non flattened tablet. Apart from tablets, the flattening can be applied to any shape of a solid oral dosage form, wherein the force is applied from a direction substantially perpendicular to the widest diameter of the dosage form when the shape is other than spherical, and applied from any direction when the shape is spherical. The flatness may then be described in terms of the thickness of the flattened shape compared to the thickness of the non-flattened shape expressed in % thickness, based on the thickness of the non flattened shape. The thickness may be measured using a thickness gauge (e.g., a digital thickness gauge or digital caliper).

In certain embodiments of the invention, apart from using a bench press, a hammer can be used for flattening tablets/dosage forms. In such a flattening process, hammer strikes may be manually applied from a direction substantially perpendicular to the widest diameter of the tablet. The flatness may then be described in terms of the thickness of the flattened shape compared to the thickness of the non-flattened shape expressed in % thickness, based on the thickness of the non-flattened shape. The thickness is measured using a thickness gauge (e.g., digital thickness gauge or digital caliper).

By contrast, when conducting the breaking strength or tablet hardness test as described in Remington's Pharmaceutical Sciences, 18th edition, 1990, Chapter 89 "Oral Solid Dosage Forms", pages 1633-1665, which is incorporated herein by reference, using the Schleuniger Apparatus the tablet/dosage form is put between a pair of flat plates arranged in parallel, and pressed by means of the flat plates, such that the force is applied substantially perpendicular to the thickness and substantially in line with the diameter of the tablet, thereby reducing the diameter in that direction. This reduced diameter is described in terms of % diameter, based on the diameter of the tablet before conducting the breaking strength test. The breaking strength or tablet hardness is defined as the force at which the tested tablet/dosage form breaks. Tablets/dosage forms that do not break, but which are deformed due to the force applied are considered to be break-resistant at that particular force.

A further test to quantify the strength of tablets/dosage forms is the indentation test using a Texture Analyzer, such as the TA-XT2 Texture Analyzer (Texture Technologies Corp., 18 Fairview Road, Scarsdale, NY 10583). In this method, the tablets/dosage forms are placed on top of a stainless steel stand with slightly concave surface, and subsequently penetrated by the descending probe of the Texture Analyzer, such as a TA-8A 1/8 inch diameter stainless steel ball probe. Before starting the measurement, the tablet is aligned directly under the probe, such that the descending probe will penetrate the tablet pivotally, i.e. in the center of the tablet, and such that the force of the descending probe is applied substantially perpendicular to the diameter and substantially in line with the thickness of the tablet. First, the probe of the Texture Analyzer starts to move towards the tablet sample at a pre-test speed. When the probe contacts the tablet surface and the trigger force set is reached, the probe continues its movement with the test speed and penetrates the tablet. For each penetration depth of the probe, which will hereinafter be referred to as "distance", the corresponding force is measured, and the data are collected. When the probe has reached the desired maximum penetration depth, it changes direction and moves back at the post-test speed, while further data can be collected. The cracking force is defined to be the force of the first local maximum that is reached in the corresponding force/distance diagram and is calculated using, for example, the Texture Analyzer software "Texture Expert Exceed, Version 2.64 English". Without wishing to be bound by any theory, it is believed that at this point, some structural damage to the tablet/dosage form occurs in the form of cracking. However, the cracked tablets/dosage forms according to certain embodiments of the present invention remain cohesive, as evidenced by the continued resistance to the descending probe. The corresponding distance at the first local maximum is hereinafter referred to as the "penetration depth to crack" distance.

For the purposes of certain embodiments of the present invention, the term "breaking strength" refers to the hardness of the tablets/dosage forms that may preferably be measured using the Schleuniger apparatus, whereas the term "cracking force" reflects the strength of the tablets/dosage forms that may preferably be measured in the indentation test using a Texture Analyzer.

A further parameter of the extended release matrix formulations that can be derived from the indentation test as described above is the work the extended release matrix formulation is subjected to in an indentation test as described above. The work value corresponds to the integral of the force over the distance.

The phrase "resistant to crushing" is defined for the purposes of certain embodiments of the present invention as referring to dosage forms that can at least be flattened with a bench press as described above without breaking. In certain embodiments, the dosage form can be flattened to no more than about 60% thickness, no more than about 50% thickness, no more than about 40% thickness, no more than about 30% thickness, no more than about 20% thickness, no more than about 10% thickness, or no more than about 5% thickness without breaking.

For the purpose of certain embodiments of the present invention, dosage forms of the present invention are regarded as being "resistant to alcohol extraction" when the respective dosage form provides an in-vitro dissolution rate, when measured in a USP Apparatus 1 (basket) at 100 rpm in 900 ml simulated gastric fluid without enzymes (SGF) comprising 40% ethanol at 37° C, characterized by the percent amount of active released at 0.5 hours, or at 0.5 and 0.75 hours, or at 0.5, 0.75 and 1 hour, or at 0.5, 0.75, 1 and 1.5 hours or at 0.75, 1, 1.5 and 2 hours of dissolution that deviates no more than about 30 % points, no more than about 20 % points or no more than about 15 % points at each of said time points from the corresponding in-vitro dissolution rate of a reference dosage form measured in a USP Apparatus 1 (basket) at 100 rpm in 900 ml simulated gastric fluid without enzymes (SGF) at 37° C without ethanol.

The term "tamper-resistant" for the purposes of the present invention refers to dosage forms which at least provide resistance to crushing or resistance to alcohol extraction, or both, as defined above and may have further tamper-resistant characteristics.

For purposes of the present invention the term "active agent" is defined as a pharmaceutically active substance useful for a therapeutic purpose. In certain embodiments, the term "active agent" refers to an opioid analgesic.

For purposes of the present invention, the term "opioid analgesic" includes single compounds and combinations of compounds selected from the group of opioids and which provide an analgesic effect such as one single opioid agonist or a combination of opioid agonists, one single mixed opioid agonist-antagonist or a combination of mixed opioid agonist-antagonists, or one single partial opioid agonist or a combination of partial opioid agonists and combinations of an opioid agonists, mixed opioid agonist-antagonists and partial opioid agonists with one or more opioid antagonists, stereoisomers, ethers, esters, salts, hydrates and solvates thereof, compositions of any of the foregoing, and the like.

The present invention disclosed herein is specifically meant to encompass the use of the active agents, as e.g. opioid analgesics, in their base form or in the form of any pharmaceutically acceptable salt thereof.

Pharmaceutically acceptable salts include, but are not limited to, inorganic acid salts such as hydrochloride, hydrobromide, sulfate, phosphate and the like; organic acid salts such as formate, acetate, trifluoroacetate, maleate, tartrate and the like; sulfonates such as methanesulfonate, benzenesulfonate, p-toluenesulfonate, and the like; amino acid salts such as arginate, asparginate, glutamate and the like, and metal salts such as sodium salt, potassium salt, cesium salt and the like; alkaline earth metals such as calcium salt, magnesium salt and the like; organic amine salts such as triethylamine salt, pyridine salt, picoline salt, ethanolamine salt, triethanolamine salt, dicyclohexylamine salt, N,N'-dibenzylethylenediamine salt and the like.

Active agents, as e.g. opioid analgesics, used according to the present invention may contain one or more asymmetric centers and may give rise to enantiomers, diastereomers, or other stereoisomeric forms. The present invention is meant to encompass the use of all such possible forms, as well as their racemic and resolved forms and compositions thereof. When the active agent contains olefinic double bonds or other centers of geometric asymmetry, it is intended to include both E and Z geometric isomers. All tautomers are intended to be encompassed by the present invention as well.

As used herein, the term "stereoisomers" is a general term for all isomers of individual molecules that differ only in the orientation of their atoms in space. It includes enantiomers and isomers of compounds with more than one chiral center that are not mirror images of one another (diastereomers).

The term "chiral center" refers to a carbon atom to which four different groups are attached.

The term "enantiomer" or "enantiomeric" refers to a molecule that is non-superimposeable on its mirror image and hence optically active wherein the enantiomer rotates the plane of polarized light in one direction and its mirror image rotates the plane of polarized light in the opposite direction.

The term "racemic" refers to a mixture of equal parts of enantiomers and which is optically inactive.

The term "resolution" refers to the separation or concentration or depletion of one of the two enantiomeric forms of a molecule.

Pharmacokinetic parameters such as $C_{max}$, $T_{max}$, $AUC_t$, $AUC_{inf}$, etc. describing the plasma drug concentration versus time curve can be obtained in clinical trials, first by single-dose administration of the active agent, e.g. oxycodone, to a number of test persons, such as healthy human subjects. The pharmacokinetic parameter values of the individual test persons are then averaged, e.g. mean AUC, mean $C_{max}$ and mean $T_{max}$ values are each obtained. In the context of the present invention, unless otherwise explicitly indicated, pharmacokinetic parameters such as AUC, $C_{max}$ and $T_{max}$ refer to mean values. Further, in the context of the present invention, *in vivo* parameters such as values for AUC, $C_{max}$, $T_{max}$, and analgesic efficacy refer to parameters or values obtained after administration at steady state or of a single dose to human subjects.

The $C_{max}$ value indicates the maximum observed plasma concentration of the active agent. The $T_{max}$ value indicates the time point at which the $C_{max}$ value is reached. In other words, $T_{max}$ is the time point of the maximum observed plasma concentration.

The AUC (Area Under the Curve) value corresponds to the area of the plasma drug concentration versus time curve. The AUC value is proportional to the amount of active agent absorbed into the blood circulation in total and hence is a measure for the bioavailability.

The $AUC_t$ value corresponds to the area under the plasma drug concentration versus time curve from the time of administration to the last measurable plasma drug concentration and is calculated by the linear up/log down trapezoidal rule.

$AUC_{inf}$ is the area under the plasma drug concentration versus time curve extrapolated to infinity and is calculated using the formula:

$$AUC_{inf} = AUC_t + \frac{C_t}{\lambda_Z}$$

where $C_t$ is the last measurable plasma concentration and $\lambda_Z$ is the apparent terminal phase rate constant.

$\lambda_Z$ is the apparent terminal phase rate constant, where $\lambda_Z$ is the magnitude of the slope of the linear regression of the log concentration versus time profile during the terminal phase.

$t_{1/2Z}$ is the apparent plasma terminal phase half-life and is commonly determined as $t_{1/2Z} = (\ln 2)/\lambda_Z$.

The lag time $t_{lag}$ is estimated as the timepoint immediately prior to the first measurable plasma drug concentration value.

The $C_{24}/C_{max}$ ratio corresponds to the ratio between the plasma drug concentration at hour 24 and $C_{max}$.

The term "healthy human subject" refers to a male or female with average values as regards height, weight and physiological parameters, such as blood pressure, etc. Healthy human subjects for the purposes of the present invention are selected according to inclusion and exclusion criteria which are based on and in accordance with recommendations of the International Conference for Harmonization of Clinical Trials (ICH).

Thus, inclusion criteria comprise males and females aged between 18 to 50 years, inclusive, a body weight ranging from 50 to 100 kg (110 to 220 lbs) and a Body Mass Index (BMI) $\geq 18$ and $\leq 34$ (kg/$m^2$), that subjects are healthy and free of significant abnormal findings as determined by medical history, physical examination, vital signs, and electrocardiogram, that females of child-bearing potential must be using an adequate and reliable method of contraception, such as a barrier with additional spermicide foam or jelly, an intra-uterine device, hormonal contraception (hormonal contraceptives alone are not acceptable), that females who are postmenopausal must have been postmenopausal $\geq 1$ year and have elevated serum follicle stimulating hormone (FSH), and that subjects are willing to eat all the food supplied during the study.

A further inclusion criterion may be that subjects will refrain from strenuous exercise during the entire study and that they will not begin a new exercise program nor participate in any unusually strenuous physical exertion.

Exclusion criteria comprise that females who are pregnant (positive beta human chorionic gonadotropin test) or lactating, any history of or current drug or alcohol abuse for five years, a history of or any current conditions that might interfere with drug absorption, distribution, metabolism or excretion, use of an opioid-containing medication in the past thirty (30) days, a history of known sensitivity to hydrocodone, naltrexone, or related compounds, any history of frequent nausea or emesis regardless of etiology, any history of seizures or head trauma with current sequelae, participation in a clinical drug study during the thirty (30) days preceding the initial dose in this study, any significant illness during the thirty (30) days preceding the initial dose in this study, use of any medication including thyroid hormone replacement therapy (hormonal contraception is allowed), vitamins, herbal, and/or mineral supplements, during the 7 days preceding the initial dose, abnormal cardiac conditions, refusal to abstain from food for 10 hours preceding and 4 hours following administration or for 4 hours following administration of the study drugs and to abstain from caffeine or xanthine entirely during each confinement, consumption of alcoholic beverages within forty-eight (48) hours of initial study drug administration (Day 1) or anytime following initial study drug administration, history of smoking or use of nicotine products within 45 days of study drug administration or a positive urine cotinine test, blood or blood products donated within 60 days prior to administration of the study drugs or anytime during the study and for 30 days after completion of the study, except as required by the clinical study protocol, plasma donated within 14 days prior to administration of the study drug or anytime during the study, except as required by the study, positive results for urine drug screen, alcohol screen at check-in of each period, and hepatitis B surface antigen (HBsAg), hepatitis C antibody (anti-HCV), a positive Naloxone HCl challenge test, presence of Gilbert's Syndrome or any known hepatobiliary abnormalities and that the Investigator believes the subject to be unsuitable for reason(s) not specifically stated above.

Subjects meeting all the inclusion criteria and none of the exclusion criteria will be randomized into the study.

The enrolled population is the group of subjects who sign informed consent.

The randomized safety population is the group of subjects who are randomized, receive study drug, and have at least one post-dose safety assessment.

The full analysis population for PK metrics will be the group of subjects who are randomized, receive study drug, and have at least one valid PK metric. Subjects experiencing emesis within 24 hours after dosing might be excluded based on visual inspection of the PK profiles prior to database lock. Subjects and profiles/metrics excluded from the analysis set will be documented in the Statistical Analysis Plan.

For the Naloxone HCl challenge test, vital signs and pulse oximetry ($SPO_2$) are obtained prior to the Naloxone HCl challenge test. The Naloxone HCl challenge may be administered intravenously or subcutaneously. For the intravenous route, the needle or cannula should remain in the arm during administration. 0.2 mg of Naloxone HCl (0.5 mL) are administered by intravenous injection. The subject is observed for 30 seconds for evidence of withdrawal signs or symptoms. Then 0.6 mg of Naloxone HCl (1.5 mL) are administered by intravenous injection. The subject is observed for 20 minutes for signs and symptoms of withdrawal. For the subcutaneous route, 0.8 mg of Naloxone HCl (2.0 mL) are administered and the subject is observed for 20 minutes for signs and symptoms of withdrawal. Following the 20-minute observation, post- Naloxone HCl challenge test vital signs and $SPO_2$ are obtained.

Vital signs include systolic blood pressure, diastolic blood pressure, pulse rate, respiratory rate, and oral temperature.

For the "How Do You Feel?" (HDYF?) Inquiry, subjects will be asked a non-leading "How Do You Feel?" question such as "Have there been any changes in your health status since screening/since you were last asked?" at each vital sign measurement. Subject's response will be assessed to determine whether an adverse event is to be reported. Subjects will also be encouraged to voluntarily report adverse events occurring at any other time during the study.

Each subject receiving a fed treatment will consume a standard high-fat content meal in accordance with the "Guidance for Industry: Food-Effect Bioavailability and Fed Bioequivalence Studies" (US Department of Health and Human Services, Food and Drug Administration, Center for Drug Evaluation and Research, December 2002). The meal will be provided 30 minutes before dosing and will be eaten at a steady rate over a 25-minute period so that it is completed by 5 minutes before dosing.

Clinical laboratory evaluations performed in the course of clinical studies include biochemistry (fasted at least 10 hours), hematology, serology, urinalysis, screen for drugs of abuse, and further tests.

Biochemistry evaluations (fasted at least 10 hours) include determination of albumin, Alkaline Phosphatase, alanine aminotransferase (alanine transaminase, ALT), aspartate aminotransferase (aspartate transaminase, AST), calcium, chloride, creatinine, glucose, inorganic phosphate, potassium, sodium, total bilirubin, total protein, urea, lactate dehydrogenase (LDH), direct bilirubin and $CO_2$.

Hematology evaluations include determination of hematocrit, hemoglobin, platelet count, red blood cell count, white blood cell count, white blood cell differential (% and absolute): basophils, eosinophils, lymphocytes, monocytes and neutrophils. Serology evaluations include determination of hepatitis B surface antigen (HBsAg), hepatitis B surface antibody (HBsAb) and hepatitis C antibody (anti-HCV).

Urinalysis evaluations include determination of color, appearance, pH, glucose, ketones, urobilinogen, nitrite, occult blood, protein, leukocyte esterase, microscopic and macroscopic evaluation, specific gravity.

Screen for drugs of abuse includes urin screen with respect to opiates, amphetamines, cannabinoids, benzodiazepines, cocaine, cotinine, barbiturates, phencyclidine, methadone and propoxyphene and alcohol tests, such as blood alcohol and breathalyzer test.

Further tests for females only include serum pregnancy test, urine pregnancy test and serum follicle stimulating hormone (FSH) test (for self reported postmenopausal females only).

The invention will now be described in more detail.

In one embodiment, the invention concerns a solid oral extended release pharmaceutical dosage form comprising a multi-layered extended release matrix formulation, the extended release matrix formulation comprising

(1) a first composition forming a first active agent-containing layer of the extended release matrix formulation comprising:

(a) at least one polyethylene oxide having, based on rheological measurements, an approximate molecular weight of at least 1,000,000; and
(b) at least one active agent; and

(2) a second composition forming an active agent-free second layer of the extended release matrix formulation comprising at least one polyethylene oxide.

In one particular embodiment the second composition comprises at least one polyethylene oxide having, based on rheological measurements, an approximate molecular weight of at least 1,000,000.
In one particular embodiment the second composition comprises at least one polyethylene oxide having, based on rheological measurements, an approximate molecular weight of less than 1,000,000.

THE ACTIVE AGENT

In one particular embodiment, the active agent in the solid oral extended release pharmaceutical dosage form is selected from opioid analgesics. The opioid analgesic may comprise or consist of one or more opioid agonists.
Opioid agonists useful in the present invention include, but are not limited to, alfentanil, allylprodine, alphaprodine, anileridine, benzylmorphine, bezitramide, buprenorphine, butorphanol, clonitazene, codeine, desomorphine, dextromoramide, dezocine, diampromide, diamorphone, dihydrocodeine, dihydromorphine, dimenoxadol, dimepheptanol, dimethylthiambutene, dioxaphetyl butyrate, dipipanone, eptazocine, ethoheptazine, ethylmethylthiambutene, ethylmorphine, etonitazene, etorphine, dihydroetorphine, fentanyl and derivatives, hydrocodone, hydromorphone, hydroxypethidine, isomethadone, ketobemidone, levorphanol, levophenacylmorphan, lofentanil, meperidine, meptazinol, metazocine, methadone, metopon, morphine, myrophine, narceine, nicomorphine, norlevorphanol, normethadone, nalorphine, nalbuphene, normorphine, norpipanone, opium, oxycodone, oxymorphone, papaveretum, pentazocine, phenadoxone, phenomorphan, phenazocine, phenoperidine, piminodine, piritramide, propheptazine, promedol, properidine, propoxyphene, sufentanil, tilidine, tramadol, and the pharmaceutically acceptable salts, hydrates and solvates thereof, mixtures of any of the foregoing, and the like.
Opioid antagonists useful in combination with opioid agonists as described above include, e.g. naloxone, naltrexone and nalmephene, and the pharmaceutically acceptable salts, hydrates and solvates thereof, mixtures of any of the foregoing, and the like.
In certain embodiments, the opioid analgesic is selected from hydrocodone, hydromorphone and the pharmaceutically acceptable salts, hydrates and solvates thereof, mixtures of any of the foregoing, and the like.
In certain embodiments, the opioid analgesic is hydromorphone, hydrocodone, or a pharmaceutically acceptable salt thereof such as e.g. the hydromorphone hydrochloride salt or the hydrocodone bitartrate salt. The dosage form comprises from about 1 mg to about 100 mg hydromorphone hydrochloride, or from about 0.5 mg to about 1250 mg hydrocodone bitartrate, or from about 2 mg to about 200 mg hydrocodone bitartrate. If other salts, derivatives or forms are used, equimolar amounts of any other pharmaceutically acceptable salt or derivative or form including but not limited to hydrates and solvates or the free base may be used. The dosage form may comprise, e.g., 5 mg, 7.5 mg, 10 mg, 15 mg, 20 mg, 30 mg, 40 mg, 45 mg, 60 mg, 80 mg, 90 mg, 100 mg, 120 or 150 mg hydrocodone bitartrate, or an equimolar amount of the free base or any other pharmaceutically acceptable salt, derivative or form thereof (including but not limited to hydrates and solvates thereof). The dosage form comprises, e.g. 2 mg, 5 mg, 7.5 mg, 10 mg, 15 mg, 20 mg, 25 mg, 30 mg, 32 mg or 64 mg hydromorphone hydrochloride or an equimolar amount of the free base or any other pharmaceutically acceptable salt, derivative or form thereof (including but not limited to hydrates and solvates thereof).
In certain embodiments, other active agents may be selected for use in accordance with the present invention either as the sole active agent or in combination with an opioid analgesic. Examples of such other active agents include antihistamines (e.g., dimenhydrinate, diphenhydramine, chlorpheniramine and dexchlorpheniramine maleate), non -steroidal anti-inflammatory agents (e.g., naproxen, diclofenac, indomethacin, ibuprofen, sulindac, Cox-2 inhibitors) and acetaminophen, anti-emetics (e.g., metoclopramide, methylnaltrexone), anti-epileptics (e.g., phenytoin, meprobmate and nitrazepam), vasodilators (e.g., nifedipine, papaverine, diltiazem and nicardipine), anti-tussive agents and expectorants (e.g. codeine phosphate), antiasthmatics (e.g. theophylline), antacids, anti-spasmodics (e.g. atropine, scopolamine), antidiabetics (e.g., insulin), diuretics (e.g., ethacrynic acid, bendrofluthiazide), anti-hypotensives (e.g., propranolol, clonidine), antihypertensives (e.g., clonidine, methyldopa), bronchodilatiors (e.g., albuterol), steroids (e.g., hydrocortisone, triamcinolone, prednisone), antibiotics (e.g., tetracycline), antihemorrhoidals, hypnotics, psychotropics, antidiarrheals, mucolytics, sedatives, decongestants (e.g. pseudoephedrine), laxatives, vitamins, stimulants (including appetite suppressants such as phenylpropanolamine) and cannabinoids, as well as pharmaceutically acceptable salts, hydrates, and solvates thereof.

In certain embodiments, the invention is directed to the use of Cox-2 inhibitors as active agents, by themselves or in combination with opioid analgesics, such as, e.g., the use of Cox-2 inhibitors such as meloxicam (4-hydroxy-2-methyl-*N*-(5-methyl-2-thiazolyl)-2*H*-1,2-benzothiazine-3-carboxamide-1,1-dioxide), as disclosed in U.S. Serial No. 10/056,347 and 11/825,938; nabumetone (4-(6-methoxy-2-naphthyl)-2-butanone), as disclosed in U.S. Serial No. 10/056,348; celecoxib (4-[5-(4-methylphenyl)-3-(trifluoromethyl)-1H-pyrazol-1-yl]benzenesulfonamide), as disclosed in U.S. Serial No. 11/698,394; nimesulide (N-(4-Nitro-2-phenoxyphenyl)methane sulfonamide), as disclosed in U.S. Serial No. 10/057,630, and N-[3-(formylamino)-4-oxo-6-phenoxy-4H-1-benzopyran-7-yl]methanesulfonamide (T-614), as disclosed in U.S. Serial No. 10/057,632.

The present invention is also directed to dosage forms utilizing active agents such as, e.g., benzodiazepines, barbiturates or amphetamines.

The term "benzodiazepines" refers to benzodiazepines and drugs that are derivatives of benzodiazepine that are able to depress the central nervous system. Benzodiazepines include, but are not limited to, alprazolam, bromazepam, chlordiazepoxide, clorazepate, diazepam, estazolam, flurazepam, halazepam, ketazolam, lorazepam, nitrazepam, oxazepam, prazepam, quazepam, temazepam, triazolam, methylphenidate as well as pharmaceutically acceptable salts, hydrates, solvates, and mixtures thereof. Benzodiazepine antagonists that can be used in the present invention include, but are not limited to, flumazenil as well as pharmaceutically acceptable salts, hydrates, solvates and mixtures thereof.

Barbiturates refer to sedative-hypnotic drugs derived from barbituric acid (2, 4, 6,-trioxohexahydropyrimidine). Barbiturates include, but are not limited to, amobarbital, aprobarbotal, butabarbital, butalbital, methohexital, mephobarbital, metharbital, pentobarbital, phenobarbital, secobarbital as well as pharmaceutically acceptable salts, hydrates, solvates, and mixtures thereof. Barbiturate antagonists that can be used in the present invention include, but are not limited to, amphetamines as well as pharmaceutically acceptable salts, hydrates, solvates and mixtures thereof.

Stimulants include, but are not limited to, amphetamines, such as amphetamine, dextroamphetamine resin complex, dextroamphetamine, methamphetamine, methylphenidate, as well as pharmaceutically acceptable salts, hydrates, and solvates and mixtures thereof. Stimulant antagonists that can be used in the present invention include, but are not limited to, benzodiazepines, as well as pharmaceutically acceptable salts, hydrates, solvates and mixtures thereof.

## THE HALF-SANDWICH TYPE OR SANDWICH TYPE STRUCTURE

Except for the shapes shown in Figures IF and 1G, the multi-layer extended release matrix formulation of the invention may have any physical shape, e.g. a cubic shape, a rectangular shape, an oval shape, a globular shape, etc., provided that at least two distinct layers are present. The two layers may have the same volume dimensions (in Vol.-%) or may have different volume dimensions. Examples of physical shapes contemplated by the invention are depicted in Figures 1 A) to E). Figures 1F and 1G depict physical shapes that are not encompassed by the present invention.

In certain embodiments of the invention, the active agent-containing layer and the active agent-free layer are visually indistinguishable from each other, thereby presenting an obstacle to abuse of the active agent. One measurement that can be utilized in order to evaluate the visual indistinguishability of the active agent-containing layer and the active agent-free layer is determining the color of the two layers by the CIE L*A*B* value. Preferably, the CIE L*A*B* values of the two layers are within 10% of each other. Another measurement to evaluate color is the use of a RYB or RGB color wheel, where the two layers preferably correspond to the same hue or adjacent hues.

The weight ratio of the active agent-containing layer : active agent free layer or blocking layer may range from about 1 to about 5 or from about 1.5 to about 3, or is about 2 or is about 2.5.

## THE COMPOSITIONS

The composition of the active agent-containing layer comprises at least one polyethylene oxide having, based on rheological measurements, an approximate molecular weight of at least 1,000,000, and at least one active agent.

The composition of the active agent-free layer does not comprise any active agent present in the active agent-containing layer. The composition of the active agent-free layer comprises at least one polyethylene oxide. In certain embodiments, the polyethylene oxide, based on rheological measurements, has an approximate molecular weight of at least 1,000,000. In certain other embodiments, the polyethylene oxide, based on rheological measurements, has an approximate molecular weight of less than 1,000,000.

In a further particular embodiment, the composition of each of the active agent-containing layer and the active agent free layer comprises less than 25 % lactose.

In a further particular embodiment, the composition of each of the active agent-containing layer and the active agent free layer comprises essentially no lactose.

In a further particular embodiment, the composition of each of the active agent-containing layer and the active agent free layer comprises essentially no hydrogenated castor oil.

In a further particular embodiment, the composition of each of the active agent-containing layer and the active agent free layer of the extended release matrix formulation comprises essentially no hydroxypropylmethylcellulose.

In certain embodiments, an antioxidant, e.g. BHT (butylated hydroxytoluene), is added to the composition.

In a further particular embodiment, the first composition (of the active agent-containing layer) comprises at least about 60 % (by wt.), or 70 % (by wt.), or 80 % (by wt.), or 90 % (by wt.) of polyethylene oxide.

In a further particular embodiment, the second composition (of the active agent free layer) comprises at least about 90 % (by wt.) of polyethylene.

In certain embodiments of the above embodiment, the composition forming the active agent-free layer comprises at least about 91 % (by wt.), at least about 92 % (by wt.), at least about 95 % (by wt.), at least about 97 % (by wt.) or at least about 99 % (by wt.) of polyethylene oxide.

According to certain embodiments, the composition forming the active agent-free layer does not contain any active agent. According to other embodiments, the composition forming the active agent-free layer does not contain any opioid antagonist, emetic or bitter substance.

According to certain embodiments, the layers of the multi-/ or bilayer dosage forms are macroscopically indistinguishable, thereby preventing a separation of the at least two layers on the basis of their visual appearance.

In certain embodiments, the layers of the multi-/ or bilayer dosage form strongly bond to each other, thereby preventing the easy separation of the layers from each other, and hindering abuse of opioid analgesic present in the active agent-containing layer of the dosage form.

In certain embodiments of the invention, the compositions comprise at least about 60 % (by wt) polyethylene oxide in the active agent-containing layer, and at least about 90 % (by wt.) of polyethylene oxide in the active agent-free layer.

In further particular embodiments, the high molecular weight polyethylene oxide has, based on rheological measurements, an approximate molecular weight of from 2,000,000 to 8,000,000.

In further particular embodiments, the high molecular weight polyethylene oxide has, based on rheological measurements, an approximate molecular weight of 2,000,000, 4,000,000, 7,000,000 or 8,000,000.

In certain embodiments of the invention, the layered extended release matrix formulation as described herein may be over-coated with a polyethylene oxide powder layer by applying to the cured or uncured formulation a powder layer of polyethylene oxide surrounding the layered core and optionally curing the powder-layered formulation as described herein. Such an outer polyethylene oxide layer may provide a lag time before the release of the active agent starts and/or a slower overall release rate. Such an outer layer may or may not comprise a certain amount of the active agent of the active agent-containing layer.

According to a further aspect of the invention, the density of the extended release matrix formulation in the solid oral extended release pharmaceutical dosage form, preferably in a dosage form containing hydromorphone HCl or hydrocodone bitartrate as active agent, is equal to or less than about 1.20 g/cm$^3$. Preferably, the density is equal to or less than about 1.19 g/cm$^3$, equal to or less than about 1.18 g/cm$^3$, or equal to or less than about 1.17 g/cm$^3$. For example, the density of the extended release matrix formulation may be in the range of from about 1.10 g/cm$^3$ to about 1.20 g/cm$^3$, or from about 1.11 g/cm$^3$ to about 1.20 g/cm$^3$, or from about 1.11 g/cm$^3$ to about 1.19 g/cm$^3$. Preferably, it is in the range of from about 1.12 g/cm$^3$ to about 1.19 g/cm$^3$ or from about 1.13 g/cm$^3$ to about 1.19 g/cm$^3$, more preferably from about 1.13 g/cm$^3$ to about 1.18 g/cm$^3$.

The density of the extended release matrix formulation is preferably determined by Archimedes Principle using a liquid of known density ($\rho 0$). The extended release matrix formulation is first weighed in air and then immersed in a liquid and weighed. From these two weights, the density of the extended release matrix formulation $\rho$ can be determined by the equation:

$$\rho = \frac{A}{A - B} \cdot \rho_0$$

wherein $\rho$ is the density of the extended release matrix formulation, A is the weight of the extended release matrix formulation in air, B is the weight of the extended release matrix formulation when immersed in a liquid and $\rho_0$ is the density of the liquid at a given temperature. A suitable liquid of known density $\rho_0$ is for example hexane.

Preferably, the density of an extended release matrix formulation is measured using a Top-loading Mettler Toledo balance Model # AB 135-S/FACT, Serial # 1127430072 and a density determination kit 33360. Preferably, hexane is used as liquid of known density $\rho 0$.

The density values throughout this document correspond to the density of the extended release matrix formulation at room temperature.

In those embodiments wherein the dosage form comprises the extended release matrix formulation coated with a cosmetic coating, the density of the extended release matrix formulation is preferably measured prior to performing the coating step, or by removing the coating from a coated extended release matrix formulation and subsequently measuring the density of the uncoated extended release matrix formulation.

## THE DISSOLUTION PROFILE

In a particular embodiment, the solid oral extended release matrix formulation provides a dissolution rate which, when measured in a USP Apparatus 1 (basket) at 100 rpm in 900 ml simulated gastric fluid without enzymes (SGF) at 37° C, releases the active agent essentially according to a zero order mode.
In a further particular embodiment, the solid oral extended release matrix formulation provides a dissolution rate which, when measured in a USP Apparatus 1 (basket) at 100 rpm in 900 ml simulated gastric fluid without enzymes (SGF) at 37° C, ranges from about 5% to about 15% (by wt.) active agent released after 1 hour and additionally may release the active agent essentially according to a zero order mode.
In a further particular embodiment, the solid oral extended release matrix formulation provides a dissolution rate which, when measured in a USP Apparatus 1 (basket) at 100 rpm in 900 ml simulated gastric fluid without enzymes (SGF) at 37° C, ranges from about 10% to about 30% (by wt.) active agent released after 2 hours and additionally may release the active agent essentially according to a zero order mode.
In a further particular embodiment, the solid oral extended release matrix formulation provides a dissolution rate which, when measured in a USP Apparatus 1 (basket) at 100 rpm in 900 ml simulated gastric fluid without enzymes (SGF) at 37° C, ranges from about 20% to about 60% (by wt.) active agent released after 4 hours and additionally may release the active agent essentially according to a zero order mode.
In a further particular embodiment, the solid oral extended release matrix formulation provides a dissolution rate which, when measured in a USP Apparatus 1 (basket) at 100 rpm in 900 ml simulated gastric fluid without enzymes (SGF) at 37° C, ranges from about 40% to about 100% (by wt.) active agent released after 8 hours and additionally may release the active agent essentially according to a zero order mode.
In a further particular embodiment, the solid oral extended release matrix formulation provides a dissolution rate which, when measured in a USP Apparatus 1 (basket) at 100 rpm in 900 ml simulated gastric fluid without enzymes (SGF) at 37° C, ranges from about 5% to about 15% (by wt.) active agent released per hour and additionally may release the active agent essentially according to a zero order mode.

## SPECIFIC HYDROCODONE AND HYDROMORPHONE COMPOSITIONS

In a further particular embodiment, the solid oral extended release pharmaceutical dosage form comprises the opioid analgesic hydrocodone bitartrate or hydromorphone hydrochloride, and the first composition comprises at least about 5 % (by wt.) of hydrocodone bitartrate or at least about 2 % (by wt.) of hydromorphone hydrochloride.
In a further particular embodiment, the first composition comprises at least about 65 % (by wt.) polyethylene oxide having, based on rheological measurements, an approximate molecular weight of at least 1,000,000.
In a further particular embodiment, the solid oral extended release pharmaceutical dosage form of the present invention comprises an extended release matrix formulation, the extended release matrix formulation comprising:

(1) a first composition forming an active agent-containing layer of said extended release matrix formulation comprising at least 65 % (by wt.) of at least one polyethylene oxide having, based on rheological measurements, an approximate molecular weight of at least 1,000,000; and
(2) about 5 mg hydrocodone bitartrate.

In a further particular embodiment, the solid oral extended release pharmaceutical dosage form of the present invention comprises an extended release matrix formulation, the extended release matrix formulation comprising:

(1) a first composition forming an active agent-containing layer of said extended release matrix formulation comprising at least 90 % (by wt.) of at least one polyethylene oxide having, based on rheological measurements, an approximate molecular weight of at least 1,000,000; and
(2) about 5 mg hydrocodone bitartrate.

In a further particular embodiment, the solid oral extended release pharmaceutical dosage form of the present invention comprises an extended release matrix formulation, the extended release matrix formulation comprising:

(1) a first composition forming an active agent-containing layer of said extended release matrix formulation comprising at least 65 % (by wt.) of at least one polyethylene oxide having, based on rheological measurements, an approximate molecular weight of at least 1,000,000; and

(2) about 10 mg hydrocodone bitartrate.

In a further particular embodiment, the solid oral extended release pharmaceutical dosage form of the present invention comprises an extended release matrix formulation, the extended release matrix formulation comprising:

(1) a first composition forming an active agent-containing layer of said extended release matrix formulation comprising at least 65 % (by wt.) of at least one polyethylene oxide having, based on rheological measurements, an approximate molecular weight of at least 1,000,000; and
(2) about 15 mg or 20 mg hydrocodone bitartrate.

In a further particular embodiment, the solid oral extended release pharmaceutical dosage form of the present invention comprises an extended release matrix formulation, the extended release matrix formulation comprising:

(1) a first composition forming an active agent-containing layer of said extended release matrix formulation comprising at least 65 % (by wt.) of at least one polyethylene oxide having, based on rheological measurements, an approximate molecular weight of at least 1,000,000; and
(2) about 40 mg hydrocodone bitartrate.

In a further particular embodiment, the solid oral extended release pharmaceutical dosage form of the present invention comprises an extended release matrix formulation, the extended release matrix formulation comprising:

(1) a first composition forming an active agent-containing layer of said extended release matrix formulation comprising at least 65 % (by wt.) of at least one polyethylene oxide having, based on rheological measurements, an approximate molecular weight of at least 1,000,000; and
(2) about 60 mg, 80 mg, 100 mg or 120 mg hydrocodone bitartrate.

In a further particular embodiment, the solid oral extended release pharmaceutical dosage form of the present invention comprises an extended release matrix formulation, the extended release matrix formulation comprising:

(1) a first composition forming an active agent-containing layer of said extended release matrix formulation comprising at least 90 % (by wt) of at least one polyethylene oxide having, based on rheological measurements, an approximate molecular weight of at least 1,000,000; and
(2) about 5 mg hydromorphone hydrochloride.

In a further particular embodiment, the solid oral extended release pharmaceutical dosage form of the present invention comprises an extended release matrix formulation, the extended release matrix formulation comprising:

(1) a first composition forming an active agent-containing layer of said extended release matrix formulation comprising at least 90 % (by wt) of at least one polyethylene oxide having, based on rheological measurements, an approximate molecular weight of at least 1,000,000; and
(2) about 6 mg or 7 mg hydromorphone hydrochloride.

In a further particular embodiment, the solid oral extended release pharmaceutical dosage form of the present invention comprises an extended release matrix formulation, the extended release matrix formulation comprising:

(1) a first composition forming an active agent-containing layer of said extended release matrix formulation comprising at least 90 % (by wt) of at least one polyethylene oxide having, based on rheological measurements, an approximate molecular weight of at least 1,000,000; and
(2) about 8 mg or 10 mg hydromorphone hydrochloride.

In a further particular embodiment, the solid oral extended release pharmaceutical dosage form of the present invention comprises an extended release matrix formulation, the extended release matrix formulation comprising:

(1) a first composition forming an active agent-containing layer of said extended release matrix formulation comprising at least 90 % (by wt) of at least one polyethylene oxide having, based on rheological measurements, an approximate molecular weight of at least 1,000,000; and
(2) about 12 mg hydromorphone hydrochloride.

In a further particular embodiment, the solid oral extended release pharmaceutical dosage form of the present invention comprises an extended release matrix formulation, the extended release matrix formulation comprising:

(1) a first composition forming an active agent-containing layer of said extended release matrix formulation comprising at least 90 % (by wt) of at least one polyethylene oxide having, based on rheological measurements, an approximate molecular weight of at least 1,000,000; and
(2) about 15 mg or 20 mg hydromorphone hydrochloride.

In a further particular embodiment, the solid oral extended release pharmaceutical dosage form of the present invention comprises an extended release matrix formulation, the extended release matrix formulation comprising:

(1) a first composition forming an active agent-containing layer of said extended release matrix formulation comprising at least 90 % (by wt) of at least one polyethylene oxide having, based on rheological measurements, an approximate molecular weight of at least 1,000,000; and
(2) about 25 mg or 30 mg hydromorphone hydrochloride.

In a further particular embodiment, the solid oral extended release pharmaceutical dosage form according to invention comprises an extended release matrix formulation, the extended release matrix formulation comprising:

(1) a first composition forming an active agent-containing layer of said extended release matrix formulation comprising at least 90 % (by wt) of at least one polyethylene oxide having, based on rheological measurements, an approximate molecular weight of at least 1,000,000; and
(2) 32 mg hydromorphone hydrochloride.

THERMOFORMING AND CURING

This section describes thermoforming or curing of the extended release matrix formulation comprising the above compositions.
In certain embodiments of the invention, the extended release matrix formulation is thermoformed or subject to a temperature curing step.
In a further particular embodiment, the extended release matrix formulation is cured at a temperature which is at least the softening temperature of at least one polyethylene oxide included in the formulation.
In a further particular embodiment, the extended release matrix formulation is cured at a temperature of at least about 60 °C for a time period of at least about 1 minute.
In a further particular embodiment, the extended release matrix formulation is cured in accordance with any procedure as described with respect to the process of preparation described herein.

PROCESS OF PREPARATION

In a further embodiment, the present invention relates to a process of preparing a solid oral extended release pharmaceutical dosage form comprising an extended release matrix formulation according to any of the preceding embodiments, comprising at least the steps of:

(a) combining at least

(1) an active agent, and
(2) at least one polyethylene oxide having, based on rheological measurements, an approximate molecular weight of at least 1,000,000, to yield a first composition forming an active agent-containing layer;

(b) providing at least one further composition comprising at least one polyethylene oxide having, based on rheological measurements, an approximate molecular weight of at least 1,000,000 or less than 1,000,000, to yield a further composition forming at least one active agent-free layer,
(c) shaping the compositions from (a) and (b) to form at least a bilayer extended release formulation; and
(d) curing said extended release matrix formulation comprising at least a curing step at a temperature which is at least the softening temperature of said at least one polyethylene oxide.

The curing step is generally conducted at atmospheric pressure. Said curing step may be conducted at the softening temperature of said at least one polyethylene oxide, e.g. for an appropriate time period, such as, e.g., for at least about 1, about 2, about 3, about 4, about 5, about 6, about 7, about 8, about 9, about 10, about 11, about 12, about 13, about 14, or about 15 minutes.

In a further particular embodiment, said curing step is conducted for a time period of at least about 5 minutes.

In a further particular embodiment, said curing step is conducted for a time period of at least about 15 minutes.

In further particular embodiments of the processes of any one of the previous embodiments, the curing step (d) takes place in a bed of free flowing extended release matrix formulations.

In further particular embodiments of the processes of any one of the previous embodiments, the curing step takes place in a coating pan. The coating pan allows an efficient batch-wise curing step which allows conducting a coating step before curing and a subsequent coating step without the need to transfer the dosage forms, e.g. the tablets.

In a further particular embodiment of the processes referred to above, the compositions in step (c) are shaped in the form of a tablet.

In a further particular embodiment of the processes referred to above, steps (a) to (c) provide the extended release matrix formulation by direct compression.

In further embodiments of the processes of any one of the previous embodiments, the curing step (d) includes coating and curing. The extended release matrix formulations, e.g. in the form of tablets, are initially coated to a first target weight gain, then cured at a temperature from about 60 °C to about 90 °C for a time period of at least about 1 minute, cooled down to a temperature of below about 50 °C, and coated to a second target weight gain. Curing step (d) is preferably conducted in a coating pan. In particular embodiments, the first target weight gain is the weight gain obtained in a first coating step, e.g. the first target weight gain may be at least 0.5 %, at least 1.0 %, or at least 1.5 % of the final tablet weight. In the second target weight gaining step, the coating results in a final target weight gain of at least 2.0 %, at least 2.5 %, at least 3.0 %, at least 3.5 %, at least 4.0 %, at least 4.5 %, or at least 5.0 % of the tablet weight.

In further particular embodiments of the processes of the invention, step (d) is performed at a temperature of at least about 60 °C or at least about 62 °C, preferably at least about 68 °C, at least about 70 °C, at least about 72 °C, or at least about 75 °C.

In further particular embodiments of the processes of the invention, the extended release matrix formulation in step (d) is subjected to a curing temperature of from about 60 °C to about 90 °C, or from about 65 °C to about 90 °C, or from about 68 °C to about 90 °C.

In further particular embodiments of the processes of the invention, the extended release matrix formulation in step (d) is subjected to a temperature of at least about 62 °C or at least about 68 °C for a time period of from about 1 minute to about 5 hours, or from about 5 minutes to about 3 hours.

In further particular embodiments of the processes of the invention, the extended release matrix formulation in step (d) is subjected to a curing temperature of at least about 62 °C or at least about 68 °C for a time period of at least about 15 minutes.

In further particular embodiments of the processes of the invention, the extended release matrix formulation in step (d) is subjected to a curing temperature of at least about 60 °C, or at least about 62 °C, or at least about 68 °C, or at least about 70 °C, or at least about 72 °C, or at least about 75 °C, or from about 62 °C to about 85 °C, for a time period of at least about 15 minutes, at least about 30 minutes, at least about 60 minutes, or at least about 90 minutes.

In further particular embodiments of the processes of any one of the previous embodiments, the extended release matrix formulation in step (d) is subjected to a curing temperature of at least about 60 °C or at least about 62 °C, but less than about 90 °C or less than about 80 °C.

In particular embodiments of the above processes of the invention, curing of the extended release matrix formulation in step d) comprises at least a curing step wherein the high molecular weight polyethylene oxide in the extended release matrix formulation at least partially melts. For example, at least about 20%, or at least about 30%, or at least about 40%, or at least about 50%, or at least about 60%, or at least about 75%, or at least about 90%, or about 100% of the high molecular weight polyethylene oxide melts.

In other embodiments, the curing of the extended release matrix formulation comprises at least a curing step wherein the extended release matrix formulation is subjected to an elevated temperature for a certain period of time. In such embodiments, the curing temperature, is at least as high as the softening temperature of the high molecular weight polyethylene oxide. Without wanting to be bound by any theory, it is believed that curing at a temperature that is at least as high as the softening temperature of the high molecular weight polyethylene oxide causes the polyethylene oxide particles to at least adhere to each other or even to fuse together. According to some embodiments, the curing temperature is at least about 60 °C, or at least about 62 °C, or ranges from about 62 °C to about 90 °C, or from about 62 °C to about 85 °C, or from about 62 °C to about 80 °C, or from about 65 °C to about 90 °C, or from about 65 °C to about 85 °C, or from about 65 °C to about 80 °C. The curing temperature preferably ranges from about 68 °C to about 90 °C, or from about 68 °C to about 85 °C, or from about 68 °C to about 80 °C, or from about 70 °C to about 90 °C, or from about 70 °C to about 85 °C, or from about 70 °C to about 80 °C, or from about 72 °C to about 90 °C, or from about 72 °C to about 85 °C, or from about 72 °C to about 80 °C. The curing temperature may be at least about 60 °C, or at least about 62 °C, but less than about 90 °C or less than about 80 °C. Preferably, it is in the range of from about 62 °C to about 72 °C, in particular from about 68 °C to about 72 °C. Preferably, the curing temperature is at least as high as the lower limit of the softening temperature range of the high molecular weight polyethylene oxide or at least about 62 °C or at least about 68 °C. More preferably, the curing temperature is within the softening temperature range of the high molecular weight polyethylene oxide or at least about 70 °C. Even more preferably, the curing temperature is at least as high as the upper limit of the softening temperature range of the high molecular weight polyethylene oxide or at least about 72 °C. In a further embodiment, the curing temperature is higher than the upper limit of the softening temperature range of the high molecular weight polyethylene oxide, for example the curing temperature is at least about 75 °C or at least about 80 °C.

In those embodiments where the curing of the extended release matrix formulation comprises at least a curing step wherein the extended release matrix formulation is subjected to an elevated temperature for a certain period of time, this period of time is hereinafter referred to as the "curing time". For the measurement of the curing time a starting point and an end point of the curing step is defined. For the purposes of the present invention, the starting point of the curing step is defined to be the point in time when the curing temperature is reached.

In certain embodiments, the temperature profile during the curing step shows a plateau-like form between the starting point and the end point of the curing. In such embodiments, the end point of the curing step is defined to be the point in time when the heating is stopped or at least reduced, e.g. by terminating or reducing the heating and/or by starting a subsequent cooling step, and the temperature subsequently drops below the curing temperature by more than about 10 °C and/ or below the lower limit of the softening temperature range of high molecular weight polyethylene oxide, for example below about 62 °C. When the curing temperature is reached and the curing step is thus started, deviations from the curing temperature in the course of the curing step can occur. Such deviations are tolerated as long as they do not exceed a value of about ± 10 °C, preferably about ±6 °C, and more preferably about ±3 °C. For example, if a curing temperature of at least about 75 °C is to be maintained, the measured temperature may temporarily increase to a value of about 85 °C, preferably about 81 °C and more preferably about 78 °C, and the measured temperature may also temporarily drop down to a value of about 65 °C, preferably about 69 °C and more preferably about 72 °C. In the cases of a larger decrease of the temperature and/or in the case that the temperature drops below the lower limit of the softening temperature range of high molecular weight polyethylene oxide, for example below about 62 °C, the curing step is discontinued, i.e., an end point is reached. Curing can be restarted by again reaching the curing temperature.

In other embodiments, the temperature profile during the curing step shows a parabolic or triangular form between the starting point and the end point of the curing. This means that after the starting point, i.e., the point in time when the curing temperature is reached, the temperature further increases to reach a maximum, and then decreases. In such embodiments, the end point of the curing step is defined to be the point in time when the temperature drops below the curing temperature.

In this context, it has to be noted that depending on the apparatus used for the curing, which will hereinafter be called the curing device, different kinds of temperatures within the curing device can be measured to characterize the curing temperature.

In certain embodiments, the curing step may take place in an oven. In such embodiments, the temperature inside the oven is measured. Based thereon, when the curing step takes place in an oven, the curing temperature is defined to be the target inside temperature of the oven and the starting point of the curing step is defined to be the point in time when the inside temperature of the oven reaches the curing temperature. The end point of the curing step is defined to be (1) the point in time when the heating is stopped or at least reduced and the temperature inside the oven subsequently drops below the curing temperature by more than about 10 °C and/ or below the lower limit of the softening temperature range of high molecular weight polyethylene oxide, for example below about 62 °C, in a plateau-like temperature profile, or (2) the point in time when the temperature inside the oven drops below the curing temperature in a parabolic or triangular temperature profile. Preferably, the curing step starts when the temperature inside the oven reaches a curing temperature of at least about 62 °C, at least about 68 °C or at least about 70 °C, more preferably of at least about 72 °C or at least about 75 °C. In preferred embodiments, the temperature profile during the curing step shows a plateau-like form, wherein the curing temperature, i.e. the inside temperature of the oven, is preferably at least about 68 °C, or about 70 °C or about 72 °C or about 73 °C, or lies within a range of from about 70 °C to about 75 °C, and the curing time is preferably in the range of from about 30 minutes to about 20 hours, more preferably from about 30 minutes to about 15 hours, or from about 30 minutes to about 4 hours, or from about 30 minutes to about 2 hours. Most preferably, the curing time is in the range of from about 30 minutes to about 90 minutes.

In certain other embodiments, the curing takes place in curing devices that are heated by an air flow and comprise a heated air supply (inlet) and an exhaust, like for example a coating pan or fluidized bed. Such curing devices will hereinafter be called convection curing devices. In such curing devices, it is possible to measure the temperature of the inlet air, i.e., the temperature of the heated air entering the convection curing device and the temperature of the exhaust air, i.e., the temperature of the air leaving the convection curing device. It is also possible to determine, or at least estimate, the temperature of the formulations inside the convection curing device during the curing step, e.g., by using infrared temperature measurement instruments, such as an IR gun, or by measuring the temperature using a temperature probe that is placed inside the curing device near the extended release matrix formulations. Based thereon, when the curing step takes place in a convection curing device, the curing temperature can be defined and the curing time can be measured as the following.

In one embodiment, wherein the curing time is measured according to method 1, the curing temperature is defined to be the target inlet air temperature and the starting point of the curing step is defined to be the point in time when the inlet air temperature reaches the curing temperature. The end point of the curing step is defined to be (1) the point in time when the heating is stopped or at least reduced and the inlet air temperature subsequently drops below the curing temperature by more than about 10 °C or below the lower limit of the softening temperature range of high molecular weight polyethylene oxide, for example below about 62 °C, in a plateau-like temperature profile, or (2) the point in time when the inlet air temperature drops below the curing temperature in a parabolic or triangular temperature profile. Preferably, the curing step starts according to method 1, when the inlet air temperature reaches a curing temperature of at least about 62 °C, at least about 68 °C, at least about 70 °C, at least about 72 °C, or at least about 75 °C. In a preferred embodiment, the temperature profile during the curing step shows a plateau-like form, wherein the curing temperature, i.e., the target inlet air temperature, is preferably at least about 72 °C, for example about 75 °C, and the curing time which is measured according to method 1 is preferably in the range of from about 15 minutes to about 2 hours, for example about 30 minutes or about 1 hour.

In another embodiment, wherein the curing time is measured with respect to the target exhaust air, the curing temperature is defined to be the target exhaust air temperature and the starting point of the curing step is defined to be the point in time when the exhaust air temperature reaches the curing temperature. The end point of the curing step is defined to be (1) the point in time when the heating is stopped or at least reduced and the exhaust air temperature subsequently drops below the curing temperature by more than about 10 °C and/ or below the lower limit of the softening temperature range of high molecular weight polyethylene oxide, for example below about 62 °C, in a plateau-like temperature profile or (2) the point in time when the exhaust air temperature drops below the curing temperature in a parabolic or triangular temperature profile. Preferably, the curing step starts according to method 2, when the exhaust air temperature reaches a curing temperature of at least about 62 °C, at least about 68 °C, at least about 70 °C, at least about 72 °C, or at least about 75 °C. In preferred embodiments, the temperature profile during the curing step shows a plateau-like form, wherein the curing temperature, i.e. the target exhaust air temperature, is at least about 68 °C, at least about 70 °C, or at least about 72 °C. For example, the target exhaust air temperature is about 68 °C, about 70 °C, about 72 °C, about 75 °C or about 78 °C, and the curing time which is measured according to method 2 is preferably in the range of from about 1 minute to about 2 hours, or from about 5 minutes to about 90 minutes. For example, the curing time is about 5 minutes, about 10 minutes, about 15 minutes, about 30 minutes, about 60 minutes, about 70 minutes, about 75 minutes or about 90 minutes. In a more preferred embodiment, the curing time, which is measured according to method 2, is in the range of from about 15 minutes to about 1 hour.

In a further embodiment, wherein the curing time is measured according to method 3, the curing temperature is defined to be the target temperature of the extended release matrix formulations and the starting point of the curing step is defined to be the point in time when the temperature of the extended release matrix formulations, which can be measured for example by an IR gun, reaches the curing temperature. The end point of the curing step is defined to be (1) the point in time when the heating is stopped or at least reduced and the temperature of the extended release matrix formulations subsequently drops below the curing temperature by more than about 10 °C and/ or below the lower limit of the softening temperature range of high molecular weight polyethylene oxide, for example below about 62 °C, in a plateau-like temperature profile, or (2) the point in time when the temperature of the extended release matrix formulations drops below the curing temperature in a parabolic or triangular temperature profile. Preferably, the curing step starts according to method 3, when the temperature of the extended release matrix formulations reaches a curing temperature of at least about 62 °C, at least about 68 °C, at least about 70 °C, at least about 72 °C, or at least about 75 °C.

In still another embodiment, wherein the curing time is measured according to method 4, the curing temperature is defined to be the target temperature measured using a temperature probe, such as a wire thermocouple, that was placed inside the curing device near the extended release matrix formulations and the starting point of the curing step is defined to be the point in time when the temperature measured using a temperature probe that was placed inside the curing device near the extended release matrix formulations reaches the curing temperature. The end point of the curing step is defined to be (1) the point in time when the heating is stopped or at least reduced and the temperature measured using the temperature probe subsequently drops below the curing temperature by more than about 10 °C and/ or below the lower limit of the softening temperature range of high molecular weight polyethylene oxide, for example below about 62 °C, in a plateau-like temperature profile or (2) the point in time when the temperature measured using the temperature probe drops below the curing temperature in a parabolic or triangular temperature profile. Preferably, the curing step starts according to method 4, when the temperature measured using a temperature probe that was placed inside the curing device near the extended release matrix formulations reaches a curing temperature of at least about 62 °C, at least about 68 °C, at least about 70 °C, at least about 72 °C, or at least about 75 °C. In a preferred embodiment, the temperature profile during the curing step shows a plateau-like form, wherein the curing temperature, i.e. the target temperature measured using a temperature probe that was placed inside the curing device near the extended release matrix formulations, is preferably at least about 68 °C, for example it is about 70 °C, and the curing time which is measured according to method 4 is preferably in the range of from about 15 minutes to about 2 hours, for example the curing time is about 60 minutes or about 90 minutes.

If curing takes place in a convection curing device, the curing time can be measured by any one of methods 1, 2, 3 or 4. In a preferred embodiment, the curing time is measured according to method 2.

In certain embodiments, the curing temperature is defined as a target temperature range, for example the curing temperature is defined as a target inlet air temperature range or a target exhaust air temperature range. In such embodiments, the starting point of the curing step is defined to be the point in time when the lower limit of the target temperature range is reached, and the end point of the curing step is defined to be the point in time when the heating is stopped or at least reduced, and the temperature subsequently drops below the lower limit of the target temperature range by more than about 10 °C and/or below the lower limit of the softening temperature range of high molecular weight polyethylene oxide, for example below about 62 °C.

The curing time, i.e. the time period the extended release matrix formulation is subjected to the curing temperature, which can for example be measured according to method 1, 2 , 3 or 4 as described above, is at least about 1 minute or at least about 5 minutes. The curing time may vary from about 1 minute to about 24 hours, or from about 5 minutes to about 20 hours, or from about 10 minutes to about 15 hours, or from about 15 minutes to about 10 hours, or from about 30 minutes to about 5 hours, depending on the specific composition and on the formulation and the curing temperature. The parameters of the composition, the curing time and the curing temperature are chosen to achieve the tamper resistance as described herein. According to certain embodiments, the curing time varies from about 15 minutes to about 30 minutes. According to further embodiments wherein the curing temperature is at least about 60 °C, at least about 62 °C, at least about 68 °C, at least about 70 °C, at least about 72 °C, or at least about 75 °C, or varies from about 62 °C to about 85 °C, or from about 65 °C to about 85 °C, the curing time is preferably at least about 15 minutes, at least about 30 minutes, at least about 60 minutes, at least about 75 minutes, at least about 90 minutes or about 120 minutes. In preferred embodiments, wherein the curing temperature is, for example, at least about 62°C, at least about 68 °C, at least about 70 °C, at least about 72 °C, or at least about 75 °C, or ranges from about 62 °C. to about 80 °C, from about 65 °C to about 80 °C, from about 68 °C to about 80 °C, from about 70 °C to about 80 °C or from about 72 °C to about 80 °C, the curing time is preferably at least about 1 minute or at least about 5 minutes. More preferably, the curing time is at least about 10 minutes, at least about 15 minutes, or at least about 30 minutes. In certain embodiments, the curing time can be chosen to be as short as possible while still achieving the desired tamper resistance. For example, the curing time preferably does not exceed about 5 hours, or does not exceed about 3 hours, or does not exceed about 2 hours. Preferably, the curing time is in the range of from about 1 minute to about 5 hours, from about 5 minutes to about 3 hours, from about 15 minutes to about 2 hours, or from about 15 minutes to about 1 hour. Any combination of curing temperature and curing time as disclosed herein lies within the scope of the present invention.

In certain embodiments, the composition is only subjected to the curing temperature until the high molecular weight polyethylene oxide present in the extended release matrix formulation has reached its softening temperature and/or at least partially melts. In certain such embodiments, the curing time may be less than about 5 minutes, for example the curing time may vary from about 0 minutes to about 3 hours, or from about 1 minute to about 2 hours, or from about 2 minutes to about 1 hour. Instant curing is possible by choosing a curing device which allows for instant heating of the high molecular weight polyethylene oxide in the extended release matrix formulation to at least its softening temperature, so that the high molecular weight polyethylene oxide at least partially melts. Such curing devices are, for example, microwave ovens, ultrasound devices, light irradiation apparatus such as UV-irradiation apparatus, ultra-high frequency (UHF) fields, or any method known to the person skilled in the art.

The skilled person is aware that the size of the extended release matrix formulation may determine the required curing time and curing temperature to achieve the desired tamper resistance. Without wishing to be bound by any theory, it is believed that in the case of a large extended release matrix formulation, such as a large tablet, a longer curing time will be necessary to conduct the heat into the interior of the formulation than in the case of a corresponding formulation with smaller size. Higher temperature increases the thermal conductivity rate and thereby decreases the required curing time.

In certain embodiments, after curing, the dosage form may be coated. An additional curing step can follow after coating the dosage form, and said additional curing step can be performed as described above. In certain such embodiments, the curing temperature of the additional curing step is preferably at least about 70 °C, at least about 72 °C or at least about 75 °C, and the curing time is preferably in the range of from about 15 minutes to about 1 hour, for example about 30 minutes.

In certain embodiments, the curing step leads to a decrease in the density of the extended release matrix formulation such that the density of the cured extended release matrix formulation is lower than the density of the extended release matrix formulation prior to the curing step. Preferably, the density of the cured extended release matrix formulation in comparison to the density of the uncured extended release matrix formulation decreases by at least about 0.5%. More preferably, the density of the cured extended release matrix formulation in comparison to the density of the uncured extended release matrix formulation decreases by at least about 0.7 %, at least about 0.8 %, at least about 1.0 %, at least about 2.0 % or at least about 2.5 %. Without wanting to be bound by any theory, it is believed that the extended release matrix formulation, due to the absence of elevated pressure during the curing step, expands, resulting in a density decrease.

According to a further aspect of the invention, the density of the extended release matrix formulation in the solid oral extended release pharmaceutical dosage form, preferably in a dosage form containing hydromorphone HCl or hydrocodone bitartrate as the active agent, is equal to or less than about 1.20 g/cm3. Preferably, it is equal to or less than about 1.19 g/cm3, equal to or less than about 1.18 g/cm3, or equal to or less than about 1.17 g/cm3. For example, the density of the extended release matrix formulation is in the range of from about 1.10 g/cm3 to about 1.20 g/cm3, from about 1.11 g/cm3 to about 1.20 g/cm3, or from about 1.11 g/cm3 to about 1.19 g/cm3. Preferably it is in the range of from about 1.12 g/cm3 to about 1.19 g/cm3 or from about 1.13 g/cm3 to about 1.19 g/cm3, more preferably from about 1.13 g/cm3 to about 1.18 g/cm3.

The density of the extended release matrix formulation is preferably determined as defined above.

In certain embodiments of the invention, the shaping of the extended release matrix formulation is performed in a tablet press, e.g., a bilayer tablet press. However, any other process for manufacturing tablets as known in the art may be used.

In certain embodiments, the present invention is directed to a process of preparing a solid oral extended release pharmaceutical dosage form, wherein step (a) above, may comprise wet granulation of the active agent and optionally other pharmaceutical additives or components of the first composition, such as microcrystalline cellulose, hydroxypropylcellulose, but not polyethylene oxide, in a granulator, e.g. a high-shear granulator. After wet granulating these components, the wet granulation material may be passed through a screen of a milling device. Thereafter, the screened material may be dried, e.g., using a fluid bed dryer. The dried granulation product may optionally be further screened through a fine screen of a milling device. Subsequently, the material is combined with the at least one polyethylene oxide of the first composition using a conventional blender (e.g. a "V" blender, Gemco 2 CU. FT.) to yield the first composition. Thereafter, further additives of the first layer composition, e.g. magnesium stearate may be added to the blended mixture.

In certain embodiments, the present invention is further directed to a process of preparing a solid oral extended release pharmaceutical dosage form, wherein a further (in case of a bilayer dosage form, a second) composition for use in preparing an "active agent-free" or "blocking layer" of the multi- or bilayer pharmaceutical dosage form of step (b) in the above process of the invention. This step may comprise a blending process of polyethylene oxide and optionally other components of the blocking layer, e.g., magnesium stearate, for example using a conventional "V" blender (e.g. a "V" blender, Gemco 2 CU. FT.).

In certain embodiments, the present invention is directed to a process of preparing a solid oral extended release pharmaceutical dosage form, wherein the compositions obtained in steps (a) and (b), respectively, are combined to form a multi- or bilayer. The compositions may be compressed in a press, e.g. a tablet press (for example a Karnavati bilayer tablet press), wherein the compositions forming the active layer and the blocking layer, respectively, may be charged in the respective sites of the hopper and the compression is then run. Subsequently, the obtained tablets may be coated to a first targeted weight gain, e.g. using spray coating with Opadry® coating suspensions. After coating to a first targeted weight gain, the tablets may be cured, e.g., using a pan coater. After curing, the products are sufficiently cooled for spray-coating with a coating suspension, e.g., in a pan coater to obtain a second targeted weight gain.

In the above described embodiments of the processes of the invention, high molecular weight polyethylene oxide having, based on rheological measurements, an approximate molecular weight of from 2,000;000 to 15,000,000 or from 2,000,000 to 8,000,000 may be used. In particular, polyethylene oxides having, based on rheological measurements, an approximate molecular weight of 2,000,000, 4,000,000, 7,000,000 or 8,000,000 may be used. In particular, polyethylene oxides having, based on rheological measurements, an approximate molecular weight of 7,000,000 or 4,000,000, may be used. Moreover, also at least one low molecular weight polyethylene oxide may be used having, based on rheological measurements, an approximate molecular weight of less than 1,000,000, such as polyethylene oxides having, based on rheological measurements, an approximate molecular weight of from 100,000 to 900,000 may be used. The addition of such low molecular weight polyethylene oxides may be used to specifically tailor the release rate, such as enhance the release rate of a formulation that otherwise provides a release rate too slow for the specific purpose. In such embodiments, at least one polyethylene oxide having, based on rheological measurements, an approximate molecular weight of 100,000 may be used. For example, in certain embodiments of the processes of the invention, compositions may be prepared that comprise at least one polyethylene oxide having, based on rheological measurements, an approximate molecular weight of at least 1,000,000 and at least one polyethylene oxide having, based on rheological measurements, an approximate molecular weight of less than 1,000,000, wherein the composition comprises at least about 10 % (by wt) or at least about 20 % (by wt) of the polyethylene oxide having, based on rheological measurements, an approximate molecular weight of less than 1,000,000. In certain such embodiments the curing temperature is less than about 80 °C or even less than about 77 °C

In certain embodiments the overall content of polyethylene oxide in the composition of the first "active agent-containing" layer prepared in the processes of the invention is at least about 60 % (by wt). Without wishing to be bound to any theory, it is believed that high contents of polyethylene oxide provide for the tamper resistance as described herein, such as high breaking strength and the resistance to alcohol extraction. According to certain such embodiments, the active agent is either hydrocodone bitartrate or hydromorphone hydrochloride and the composition comprises more than about 5% (by wt) of the hydrocodone bitartrate or more than about 2 % (by wt.) of the hydromorphone hydrochloride.

In certain such embodiments in the composition prepared in the processes of the invention, the content of the at least one polyethylene oxide in the "active agent-containing" layer having, based on rheological measurements, an approximate molecular weight of at least 1,000,000 is at least about 60 % (by wt). In certain embodiments, the content in the composition of the at least one polyethylene oxide having, based on rheological measurements, an approximate molecular weight of at least 1,000,000 is at least about 65 %, 70 %, 75 %, 80 %, 85% or at least about 90 % (by wt). In such embodiments, a polyethylene oxide having, based on rheological measurements, an approximate molecular weight of at least 4,000,000 or at least 7,000,000 may be employed. In certain such embodiments, the active agent is hydrocodone bitartrate or hydromorphone hydrochloride, although other active agents can also be used according to this aspect of the invention, and the composition comprises more than about 5% (by wt) hydrocodone bitartrate or hydromorphone hydrochloride.

In certain embodiments of the invention, magnesium stearate is added during or after the curing step in order to avoid the tablets sticking together. In certain such embodiments, the magnesium stearate is added at the end of the curing process before or during the cooling of the tablets. Other anti-tacking agents that could be used would be talc, silica, fumed silica, colloidal silica dioxide, calcium stearate, carnauba wax, long chain fatty alcohols and waxes, such as stearic acid and stearyl alcohol, mineral oil, paraffin, micro crystalline cellulose, glycerin, propylene glycol, and polyethylene glycol. Additionally or alternatively, the coating can be started at the high temperature to avoid sticking.

In certain embodiments, wherein the curing step is carried out in a coating pan, the sticking of tablets can be avoided (or sticking tablets can be separated) by increasing the pan speed during or after the curing step, in the latter case for example before or during the cooling of the tablets. The pan speed may be increased up to a speed where all tablets are separated or no sticking occurs.

In certain embodiments of the invention, an initial film coating or a fraction of a film coating is applied prior to performing the curing step d as described above. This film coating provides an "overcoat" for the extended release matrix formulations or tablets to function as an anti-tacking agent, i.e. in order to avoid the formulations or tablets sticking together. In certain such embodiments, the film coating which is applied prior to the curing step is an Opadry film coating. After the curing step d), a further film coating step can be performed.

The present invention encompasses also any multi-/ or bilayer solid oral extended release formulation obtainable by a process according to any process as described above.

In further particular embodiments of the invention, the solid oral extended release pharmaceutical dosage form according to any one of the above embodiments is administered to a patient in need thereof for the treatment of pain, said dosage form comprising an opioid analgesic.

In further particular embodiments, the invention relates to methods of treatment using the above-disclosed solid oral extended release pharmaceutical dosage forms comprising the extended release matrix formulation described in any of the above embodiments.

In a further particular embodiment, the extended release matrix formulations of the invention are used in the manufacture of a medicament for the treatment of pain, wherein the extended release matrix formulation comprises an opioid analgesic.

## TAMPER RESISTANCE

In certain particular embodiments, the present invention is directed to a solid oral extended release pharmaceutical dosage form comprising an extended release matrix formulation in the form of a multi-/ or bilayer tablet as described herein, wherein the tablet can be at least flattened without breaking, characterized by a thickness of the tablet after the flattening which corresponds to no more than about 60 % of the thickness of the tablet before flattening, and wherein said flattened tablet provides an in-vitro dissolution rate, when measured in a USP Apparatus 1 (basket) at 100 rpm in 900 ml simulated gastric fluid without enzymes (SGF) at 37° C, characterized by the percent amount of active released at 0.5 hours of dissolution that deviates no more than about 30 % points from the corresponding in-vitro dissolution rate of a non-flattened reference tablet.

In certain embodiments, the present invention is directed to a solid oral extended release pharmaceutical dosage form comprising an extended release matrix formulation in the form of a multi-/ or bilayer tablet as described herein, wherein the tablet can at least be flattened without breaking, characterized by a thickness of the tablet after the flattening which corresponds to no more than about 60% of the thickness of the tablet before flattening, and wherein the flattened or non flattened tablet provide an in-vitro dissolution rate, when measured in a USP Apparatus 1 (basket) at 100 rpm in 900 ml simulated gastric fluid without enzymes (SGF) comprising 40% ethanol at 37° C, characterized by the percent amount of active released at 0.5 hours of dissolution that deviates no more than about 30 % points from the corresponding in-vitro dissolution rate measured in a USP Apparatus 1 (basket) at 100 rpm in 900 ml simulated gastric fluid without enzymes (SGF) at 37° C without ethanol, using flattened and non flattened reference tablets, respectively.

In certain embodiments, the invention is directed to solid oral extended release pharmaceutical dosage forms comprising an extended release matrix formulation comprising an active agent, said dosage forms being in the form of a multi- or bilayer tablet, wherein the tablet can at least be flattened without breaking, characterized by a thickness of the tablet after the flattening which corresponds to no more than about 60 % of the thickness of the tablet before flattening, and wherein said flattened tablet provides an in-vitro dissolution rate, when measured in a USP Apparatus 1 (basket) at 100 rpm in 900 ml simulated gastric fluid without enzymes (SGF) at 37° C, characterized by the percent amount of active released at 1, 8 and 24 hours of dissolution that deviates no more than about 30 % points at each of said time points from the corresponding in-vitro dissolution rate of a non-flattened reference tablet.

In certain such embodiments, the tablet can at least be flattened without breaking, characterized by a thickness of the tablet after the flattening which corresponds to no more than about 50 %, or no more than about 40%, or no more than about 30%, or no more than about 20%, or no more than about 16% of the thickness of the tablet before flattening, and wherein said flattened tablet provides an in-vitro dissolution rate, when measured in a USP Apparatus 1 (basket) at 100 rpm in 900 ml simulated gastric fluid without enzymes (SGF) at 37° C, characterized by the percent amount of active released at 1, 8 and 24 hours of dissolution that deviates no more than about 30 % points, or no more than about 20 % points, or no more than about 15 % points, or no more than about 10% points at each of said time points from the corresponding in-vitro dissolution rate of a non-flattened reference tablet.

In a further embodiment, the solid oral extended release matrix formulation according to any of the preceding embodiments has, when subjected to an indentation test, a cracking force of at least about 110 N.

In certain embodiments of the invention the extended release matrix formulation has a cracking force of at least about 110 N, or at least about 120 N, or at least about 130 N, or at least about 140 N, or at least about 150 N, or at least about 160 N, or at least about 170 N, or at least about 180 N, or at least about 190 N, or at least about 200 N.

In a further embodiment, the solid oral extended release matrix formulation, when subjected to an indentation test, has a "penetration depth to crack distance" of at least about 1.0 mm.

In certain embodiments of the invention, the extended release matrix formulation has a "penetration depth to crack" distance of at least about 1.0 mm, or at least about 1.2 mm, or at least about 1.4 mm, or at least about 1.5 mm, or at least about 1.6 mm, or at least about 1.8 mm, or at least about 1.9 mm, or at least about 2.0 mm, or at least about 2.2 mm, or at least about 2.4 mm, or at least about 2.6 mm.

In a further embodiment, the solid oral extended release matrix formulation has a cracking force of at least about 120 N, or at least about 130 N, or at least about 140 N and/or a "penetration depth to crack" distance of at least about 1.2 mm, or at least about 1.4 mm, or at least about 1.5 mm, or at least about 1.6 mm.

In certain such embodiments of the invention, the extended release matrix formulation has a cracking force of at least about 110 N, or at least about 120 N, or at least about 130 N, or at least about 140 N, or at least about 150 N, or at least about 160 N, or at least about 170 N, or at least about 180 N, or at least about 190 N, or at least about 200 N, and/or a "penetration depth to crack" distance of at least about 1.0 mm, or at least about 1.2 mmor at least about 1.4 mm, or at least about 1.5 mm, or at least about 1.6 mm, or at least about 1.8 mm, or at least about 1.9 mm, or at least about 2.0 mm, or at least about 2.2 mm, or at least about 2.4 mm, or at least about 2.6 mm. A combination of any of the aforementioned values of cracking force and "penetration depth to crack" distance is included in the scope of the present invention.

In a further embodiment, solid oral extended release matrix formulations of the invention resist work of at least about 0.06 J without cracking.

In certain such embodiments the extended release matrix formulation when subjected to an indentation test resists work of at least about 0.06 J, or at least about 0.08 J, or at least about 0.09 J, or at least about 0.11 J, or at least about 0.13 J, or at least about 0.15 J, or at least about 0.17 J, or at least about 0.19 J, or at least about 0.21 J, or at least about 0.23 J, or at least about 0.25 J, without cracking.

The parameters "cracking force", "penetration depth to crack distance" and "work" may be determined in an indentation test as described above, using a Texture Analyzer such as the TA-XT2 Texture Analyzer (Texture Technologies Corp., 18 Fairview Road, Scarsdale, NY 10583). The cracking force and/or "penetration depth to crack" distance can be determined using an uncoated or a coated extended release matrix formulation.

In certain embodiments, the extended release matrix formulation is in the form of a multi- or bilayer tablet or multi- or bilayer multi particulates, and the tablet can at least be flattened without breaking, characterized by a thickness of the tablet after the flattening which corresponds to no more than about 60 % of the thickness of the tablet or the individual multiparticulates before flattening. Preferably, the tablet can at least be flattened without breaking, characterized by a thickness of the tablet after the flattening which corresponds to no more than about 50 %, or no more than about 40 %, or no more than about 30%, or no more than about 20%, or no more than about 16 % of the thickness of the tablet before flattening.

Preferably, the flattening of the tablets is performed with a bench press, such as a carver style bench press, or with a hammer, as described above.

In certain such embodiments the extended release matrix formulation is in the form of a multi- or bilayer tablet, and the tablet can at least be flattened without breaking, characterized by a thickness of the tablet after the flattening which corresponds to no more than about 60 % of the thickness of the tablet before flattening, and wherein said flattened tablet provides an in-vitro dissolution rate, when measured in a USP Apparatus 1 (basket) at 100 rpm in 900 ml simulated gastric fluid without enzymes (SGF) at 37° C, characterized by the percent amount of active released at 0.5 hours or at 0.5 and 0.75 hours, or at 0.5, 0.75 and 1 hours, or at 0.5, 0.75, 1 and 1.5 hours, or at 0.5, 0.75, 1, 1.5 and 2 hours of dissolution that deviates no more than about 30 % points at each of said time points from the corresponding in-vitro dissolution rate of a non-flattened reference tablet. Preferably, the tablet can at least be flattened without breaking, characterized by a thickness of the tablet after the flattening which corresponds to no more than about 50 %, or no more than about 40%, or no more than about 30%, or no more than about 20%, or no more than about 16% of the thickness of the tablet before flattening, and wherein said flattened tablet provides an in-vitro dissolution rate, when measured in a USP Apparatus 1 (basket) at 100 rpm in 900 ml simulated gastric fluid without enzymes (SGF) at 37° C, characterized by the percent amount of active released at 0.5 hours, or at 0.5 and 0.75 hours, or at 0.5, 0.75 and 1 hours, or at 0.5, 0.75, 1 and 1.5 hours, or at 0.5, 0.75, 1, 1.5 and 2 hours of dissolution that deviates no more than about 30% points, or no more than about 20 % points, or no more than about 15 % points at each of said time points from the corresponding in-vitro dissolution rate of a non-flattened reference tablet.

Preferably, the tablet hardness test to determine the breaking strength of extended release matrix formulations is performed in a Schleuniger Apparatus as described above. For example, the breaking strength is determined using a Schleuniger 2E /106 Apparatus and applying a force of a maximum of about 196 N, or a Schleuniger Model 6D Apparatus and applying a force of a maximum of about 439 N.

It has been observed that the extended release matrix formulations of the present invention comprising a high molecular weight polyethylene oxide can be flattened to a thickness of between about 15 and about 18 % of the non-flattened thickness, and that the flattened bilayer tablet resumes in part or substantially resumes its initial non-flattened shape or a portion thereof during dissolution, neglecting the swelling that also takes place during dissolution, i.e. the thickness of the bilayer tablet increases and the diameter decreases considerably during dissolution. Without wishing to be bound to any theory, it is believed that the high molecular weight polyethylene oxide has a "form memory" providing the ability to restore the initial form or a portion thereof after deformation, e.g. after flattening, in an environment that allows such restoration, such as an aqueous environment used in dissolution tests. This ability is believed to contribute to the tamper resistance, in particular the alcohol resistance, of the dosage forms of the present invention.

CLINICAL STUDIES

The solid oral extended release pharmaceutical dosage form according to the invention comprising hydrocodone or a pharmaceutically acceptable salt, hydrate or solvate thereof, or mixtures of any of the foregoing, may provide the following invivo parameters.

Such solid oral extended release pharmaceutical dosage form may provide a $C_{24}/C_{max}$ ratio of hydrocodone of about 0.40 to about 1.0 after administration of a single dose, or after administration at steady state. The $C_{24}/C_{max}$ ratio may be about 0.40 to about 0.85, or about 0.40 to about 0.75, or about 0.45 to about 0.70, or about 0.2 to about 0.8, about 0.3 to about 0.7, or about 0.4 to about 0.6.

Such solid oral extended release pharmaceutical dosage form may provide a $T_{max}$ (h) of hydrocodone from about 4 to about 20 hours, or about 6 to about 12 hours, or about 4 to about 10 hours after administration of a single dose, or after administration at steady state.

Such solid oral extended release pharmaceutical dosage form may provide a mean AUC (ng*h/mL) after administration of about 250 to 400 per each 20 mg hydrocodone included in the dosage form, and may also provide a mean $C_{max}$ (ng/mL) after administration of about 10 to about 30 per each 20 mg hydrocodone included in the dosage form.

If such solid oral extended release pharmaceutical dosage form contains about 20 mg hydrocodone or a pharmaceutically acceptable salt thereof, it may provide a mean AUC (ng*h/mL) after administration of about 250 to about 400, or about 270 to about 350, and a mean $C_{max}$ (ng/mL) after administration of about 10 to about 30, about 12 to about 25, about 14 to about 18, or about 12 to about 17.

If such solid oral extended release pharmaceutical dosage form contains about 120 mg hydrocodone or a pharmaceutically acceptable salt thereof, it may provide a mean AUC (ng*h/mL) after administration of about 1500 to about 2400, about 1700 to about 2200, about 1800 to about 2100, or about 1900 to about 2100, and a mean $C_{max}$ (ng/mL) after administration of about 60 to about 180, or about 80 to about 160.

Such solid oral extended release pharmaceutical dosage form may also provides a mean $T_{1/2}$ (h) after administration of about 5 to about 10 h, about 6 to about 9 h, about 7 or about 8h, and a mean $T_{lag}$ (h) after administration of about 0.01 to about 0.2.

In general, the solid oral extended release pharmaceutical dosage form of the invention is administered to a subject patient in the fasted state. The mean AUC (ng*h/mL) after administration in the fed state is preferably less than 20% higher, or less than 16% higher, or less than 12% higher than the AUC (ng*h/mL) after administration in the fasted state. Likewise, the mean $C_{max}$ (ng/mL) after administration in the fed state is preferably less than 80% higher, less than 70% higher, or less than 60% higher than the $C_{max}$ after administration in the fasted state. Additionally, the mean $T_{max}$ (h) after administration in the fed state may be within 35%, or within 30% of the $T_{max}$ (h) after administration in the fasted state; the mean $T_{1/2}$ (h) after administration in the fed state may be within 15% of the $T_{1/2}$ after administration in the fasted state; and the mean $T_{lag}$ (h) after administration in the fed state may be less than 150% higher than the $T_{1/2}$ after administration in the fasted state.

The invention also encompasses the dosage form for use in the method of treatment wherein a bilayer or multi-layer dosage form is administered for treatment of a disease or condition of a patient that requires treatment in particular pain and the use of a multi-/ or bilayer dosage form according to the invention for the manufacture of a medicament for the treatment of a disease or certain condition of a patient that requires treatment in particular pain.

The invention also encompasses the use of a bilayer or multi-layer dosage form of the invention in the manufacture of a medicament to treat a disease or condition of a patient requiring such treatment. In one embodiment, the condition is pain.

**EXAMPLE 1**

Hydrocodone bitartrate bilayer tablets.

Three different bilayer tablets including 20 mg hydrocodone bitartrate were prepared, each possessing a 400 mg active layer and a polyethylene oxide blocking layer of 100 (Example 1A), 200 (Example 1B) and 300 mg (Example 1C), respectively.

The compositions of these tablets are shown in Table 1:

Table 1

| | **Ex. 1A** | **Ex. 1B** | **Ex. 1C** | | |
|---|---|---|---|---|---|
| **Component** | **mg/tablet** | **mg/tablet** | **mg/tablet** | **Function** | Ref.to Standard |
| **Active Layer** | | | | | |
| Hydrocodone bitartrate | 20.0 | 20.0 | 20.0 | Active Ingredient | USP |
| Microcrystalline cellulose | 1.36 | 1.36 | 1.36 | Diluent | NF |
| Hydroxypropylcellulose | 1.36 | 1.36 | 1.36 | Binder | NF |
| Purified water [1] | N/A | N/A | N/A | Solvent | USP |
| Polyethylene oxide (WSR-303) | 375.28 | 375.28 | 375.28 | Release-Controlling Polymer | NF |
| Magnesium stearate | 2.0 | 2.0 | 2.0 | Lubricant | NF |
| **Active Layer Subtotal** | **400.0** | **400.0** | **400.0** | | |
| **Blocking Layer** | | | | | |
| Polyethylene oxide (WSR-303) | 99.5 | 199.0 | 298.5 | Release-Controlling Polymer | NF |
| Magnesium stearate | 0.5 | 1.0 | 1.5 | Lubricant | NF |
| **Blocking Layer Subtotal** | **100.0** | **200.0** | **300.0** | | |
| **Coating** | | | | | |
| Opadry® White Y-5-18024-A | 20.0 | 24.0 | 28.0 | Cosmetic Coat | HSE[2] |
| Purified water [1] | N/A | N/A | N/A | Solvent | USP |
| **Total** | **520.0** | **624.0** | **728.0** | | |

[1] Purified water is used to prepare the hydrocodone bitartrate granulation and the coating suspension. It is not present in the final product.
[2] HSE = in-house standard

Table 2A

| Components of the active layer in weight percent: | | | |
|---|---|---|---|
| | **Ex. 1A** | **Ex. 1B** | **Ex. 1C** |
| **Component** | **% (by wt.)** | **% (by wt.)** | **% (by wt.)** |
| Hydrocodone bitartrate | 5.00 | 5.00 | 5.00 |
| Microcrystalline cellulose | 0.34 | 0.34 | 0.34 |
| Hydroxypropylcellulose | 0.34 | 0.34 | 0.34 |
| Polyethylene oxide (WSR-303) | 93.82 | 93.82 | 93.82 |
| Magnesium stearate | 0.50 | 0.50 | 0.50 |
| **Total** | **100.00** | **100.00** | **100.00** |

Table 2B

| Percentaged composition blocking layer: | | | |
|---|---|---|---|
| | **Ex. 1A** | **Ex. 1B** | **Ex. 1C** |
| **Component** | **% (by wt.)** | **% (by wt.)** | **% (by wt.)** |
| Polyethylene oxide (WSR-303) | 99.50 | 99.50 | 99.50 |
| Magnesium stearate | 0.50 | 0.50 | 0.50 |
| **Total** | **100.00** | **100.00** | **100.00** |

Table 2C

| Ratio active layer / blocking layer: | | | |
|---|---|---|---|
| | **Ex. 1A** | **Ex. 1B** | **Ex. 1C** |
| Weight ratio active layer / blocking layer | 4.00 | 2.00 | 1.33 |

Preparation of blocking layer blend:

1. A "V" blender (Gemco "V" Blender - 2 CU. FT.) with intensifier bar was charged with the polyethylene oxide WSR 303 and the magnesium stearate.
2. Step 1 materials were blended for one minute with the intensifier bar OFF.
3. Step 2 blend was charged into a clean, tared stainless steel or polyethylene lined container.

Preparation of active layer blend:

4. A high-shear granulator (Collette 75 L) was charged with the hydrocodone bitartrate, the microcrystalline cellulose and the hydroxypropylcellulose.
5. Water was added to the mixture with the propeller and chopper on.
6. The wet granulation from step 5 was passed through the coarse screen of a Quadro Comil milling device.
7. The screened granulation from step 6 was dried in a Vector VFC-3 fluid bed dryer.
8. The dried granulation from step 7 was passed through the fine screen of the Quadro Comil.
9. A "V" blender (Gemco 2 CU. FT.) with intensifier bar was charged with the polyethylene oxide WSR 303 and the milled granulation from step 8.

10. Step 9 materials were blended for 7.5 minutes with the intensifier bar OFF.
11. Magnesium stearate was added to the mixture from step 10.
12. Step 11 materials were blended for 1 minute with the intensifier bar OFF.
13. Step 12 blend was charged into a clean, tared stainless steel or polyethylene lined container.

Preparation of bilayer tablets:

14. The blends from step 3 and step 13 were concurrently compressed into bilayer oval tablets on a Karnavati bilayer tablet press (Karnavati UNIK-I) at a rotation speed of 10 rpm. The active blend was loaded into the side one hopper and the active layer weight was adjusted to target 400 mg. Then the blocking layer blend was loaded into the side two hopper and the total tablet weight was adjusted to target. After weight adjustment, the compression run was started and the press was run at 10 rpm.
15. The aqueous Opadry® coating suspension was prepared by adding Opadry® white to the vortex. Once the Opadry® was incorporated in the purified water, the mixing was continued for about an hour prior to use.
16. Approximately 10 kg of the core tablets from step 14 were weighed out and spray-coated with the coating suspension to a target weight gain of about 1.0 % (by wt.) in a perforated 24 inch Compu-Lab pan coater (COMP-U-LAB 24). The tablet bed was warmed by setting the inlet air temperature to 55 °C. Once the exhaust temperature reached 39 °C, the film coating began at a pan speed of 15 rpm and a spray rate of approximately 45 mL/min. Film coating was continued until the target 1% weight gain was achieved.
17. The partially coated tablets from step 16 were cured in the perforated pan coater. The inlet temperature was set to 85 °C at a pan speed of approximately 10 rpm. The tablets were cured at an exhaust temperature of 72 °C for approximately 30 minutes.
18. After curing, the tablets were cooled in the rotating pan by setting the inlet temperature to 22 °C. Cooling was continued until the exhaust temperature was less than 28 °C.
19. The cured tablets from step 18 were spray-coated with the coating suspension to a target final weight gain of 4.0 % (by wt.) in the perforated pan coater at a pan speed of 15 rpm and spray rate of approximately 50 mL/min.
20. The film coated tablets were transferred into a tared polyethylene lined drum.

**EXAMPLE 2**

Hydrocodone Bitartrate Film Coated Bilayer Tablets, 120 mg.

In Example 2, three different bilayer tablets including 120 mg hydrocodone bitartrate were prepared, each possessing a 400 mg active layer and a polyethylene oxide blocking layer of 100 mg (Example 2A), 200 mg (Example 2B) and 300 mg (Example 2C), respectively.

The compositions of Examples 2A, 2B and 2C, respectively are shown in Table 3.

The compositions of Examples 2A, 2B and 2C, respectively are shown in Table 3.

Table 3

| | Ex. 2A | Ex. 2B | Ex. 2C | | |
|---|---|---|---|---|---|
| **Component** | **mg/tablet** | **mg/tablet** | **mg/tablet** | **Function** | Ref. to Standard |
| **Active layer** | | | | | |
| Hydrocodone bitartrate | 120.0 | 120.0 | 120.0 | Active Ingredient | USP |
| Microcrystalline bellulose | 8.16 | 8.16 | 8.16 | Diluent | NF |
| Hydroxypropylcellulose | 8.16 | 8.16 | 8.16 | Binder | NF |
| Purified water [1] | N/A | N/A | N/A | Solvent | USP |
| Polyethylene oxide (WSR-303) | 261.68 | 261.68 | 261.68 | Release-Controlling Polymer | NF |
| Magnesium stearate | 2.0 | 2.0 | 2.0 | Lubricant | NF |
| **Active Layer Subtotal** | **400.0** | **400.0** | **400.0** | | |

(continued)

| Blocking Layer | | | | | |
|---|---|---|---|---|---|
| Polyethylene oxide (WSR-303) | 99.5 | 199.0 | 298.5 | Release-Controlling Polymer | NF |
| Magnesium stearate | 0.5 | 1.0 | 1.5 | Lubricant | NF |
| **Blocking Layer Subtotal** | **100.0** | **200.0** | **300.0** | | |
| **Coating** | | | | | |
| Opadry® White Y-5-18024-A | 20.0 | 24.0 | 28.0 | Cosmetic Coat | HSE[2] |
| Purified water[1] | N/A | N/A | N/A | Solvent | USP |
| **Total** | **520.0** | **624.0** | **728.0** | | |

[1] Purified water is used to prepare the hydrocodone bitartrate granulation and the coating suspension. It is not present in the final product.
[2] HSE = in-house standard

From the overall composition of Examples 2A, 2B and 2C, the amount of the components of the active layer in weight percent and of the blocking layer in weight percent, as well as the percent ratio of the active layer / blocking layer, can be calculated. This is shown in Tables 3A, 3B and 3C, respectively.

Table 3A

| **Components of the active layer in weight percent** | | | |
|---|---|---|---|
| | **Ex. 2A** | **Ex. 2B** | **Ex. 2C** |
| **Component** | **% (by wt.)** | **% (by wt.)** | **% (by wt.)** |
| Hydrocodone bitartrate | 30.00 | 30.00 | 30.00 |
| Microcrystalline cellulose | 2.04 | 2.04 | 2.04 |
| Hydroxypropylcellulose | 2.04 | 2.04 | 2.04 |
| Polyethylene oxide (WSR-303) | 65.42 | 65.42 | 65.42 |
| Magnesium stearate | 0.50 | 0.50 | 0.50 |
| Total | 100.00 | 100.00 | 100.00 |

Table 3B

| **Components of the blocking layer in weight percent** | | | |
|---|---|---|---|
| | **Ex. 2A** | **Ex. 2B** | **Ex. 2C** |
| **Component** | **% (by wt.)** | **% (by wt.)** | **% (by wt.)** |
| Polyethylene oxide (WSR-303) | 99.50 | 99.50 | 99.50 |
| Magnesium stearate | 0.50 | 0.50 | 0.50 |
| Total | 100.00 | 100.00 | 100.00 |

Table 3C

| **Ratio active layer / blocking layer:** | | | |
|---|---|---|---|
| | **Ex. 2A** | **Ex. 2B** | **Ex. 2C** |
| Weight ratio active layer / blocking layer | 4.00 | 2.00 | 1.33 |

The processing steps to manufacture the tablets in Examples 2A, 2B and 2C were as follows:

Batch sizes

2A: 13 kg, 25,000 Tablets
2B: 15.6 kg, 25,000 Tablets
2C: 18.2 kg, 25,000 Tablets

The process conditions for the preparation of the above batches correspond to those used in Example 1.

**EXAMPLE 3**

Hydromorphone hydrochloride film-coated tablets 12 mg.

In Example 3, two different bilayer tablets including 12 mg hydromorphone hydrochloride were prepared, each possessing an active layer of 500 mg (Example 3A) and 400 mg (Example 3B), respectively, and a 200 mg polyethylene oxide blocking layer.

The compositions of the compositions in Examples 3A and 3B, respectively are shown in Table 4.

Table 4

| | **Ex. 3A** | **Ex. 3B** | | |
|---|---|---|---|---|
| **Component** | **mg/tablet** | **mg/tablet** | **Function** | Ref. to Standard |
| **Active layer** | | | | |
| Hydromorphone hydrochloride | 12.0 | 12.0 | Active Ingredient | USP |
| Polyethylene oxide (WSR-303) | 485.5 | 386.0 | Release-Controlling Polymer | NF |
| Magnesium stearate | 2.5 | 2.0 | Lubricant | NF |
| **Active Layer Subtotal** | **500.0** | **400.0** | | |
| **Blocking Layer** | | | | |
| Polyethylene oxide (WSR-303) | 199.0 | 199.0 | Release-Controlling Polymer | NF |
| Magnesium stearate | 1.0 | 1.0 | Lubricant | NF |
| **Blocking Layer Subtotal** | **200.0** | **200.0** | | |
| **Coating** | | | | |
| Opadry II Beige 33G97231 | 28.0 | 24.0 | Cosmetic Coat | HSE[1] |
| Purified water[2] | N/A | N/A | Solvent | USP |
| **Total** | **728.0** | **624.0** | | |
| [1] HSE - In-house standard; [2] Not present in final product | | | | |

From the overall composition of Examples 3A and 3B, the weight percent of composition of the active layer and the blocking layer, respectively, and the ratio of active layer / blocking layer may be calculated. This is shown in Tables 5A, 5B and 5C, respectively.

Table 5A

| **Composition of active layer in weight percent:** | | |
|---|---|---|
| | **Ex. 3A** | **Ex. 3B** |
| **Component** | **% (by wt.)** | **% (by wt.)** |
| Hydromorphone Hydrochloride | 2.40 | 3.00 |
| Polyethylene Oxide (WSR-303) | 97.10 | 96.50 |

(continued)

| Composition of active layer in weight percent: | | |
|---|---|---|
| | **Ex. 3A** | **Ex. 3B** |
| **Component** | **% (by wt.)** | **% (by wt.)** |
| Magnesium Stearate | 0.50 | 0.50 |
| Total | 100.00 | 100.00 |

Table 5B

| Composition of blocking layer in weight percent: | | |
|---|---|---|
| | **Ex. 3A** | **Ex. 3B** |
| **Component** | **% (by wt.)** | **% (by wt.)** |
| Polyethylene Oxide (WSR-303) | **99.50** | 99.50 |
| Magnesium Stearate | 0.50 | 0.50 |
| Total | 100.00 | 100.00 |

Table 5C

| Ratio active layer / blocking layer: | | |
|---|---|---|
| | **Ex. 3A** | **Ex. 3B** |
| Ratio active layer / blocking layer | 2.50 | 2.00 |

The processing steps to manufacture the tablets in Examples 3A and 3B, respectively, were as follows:

Batch sizes:

3A: 14.560 kg, 20,000 Tablets
3B: 13.728 kg, 22,000 Tablets

Preparation of blocking layer blend:

1. A Gemco "V" Blender - 2 CU. FT. with intensifier bar was charged with the polyethylene oxide WSR 303 and the magnesium stearate.
2. Step 1 materials were blended for one minute with the intensifier bar OFF.
3. Step 2 blend was charged into a clean, tared stainless steel or polyethylene lined container.

Preparation of active layer blend:

4. A PK "V" Blender - 16 QT. with intensifier bar was charged with the polyethylene oxide WSR 303 and the hydromorphone hydrochloride.
5. Step 4 materials were blended for 10 minutes with the intensifier bar ON.
6. Magnesium stearate was added to the mixture from step 5.
7. Step 6 materials were blended for one minute with the intensifier bar OFF.
8. Step 7 blend was charged into clean, tared stainless steel or polyethylene lined container.

Preparation of bilayer tablets:

9. The blends from step 3 and step 8 were concurrently compressed into bilayer oval tablets on a Karnavati bilayer press (Karnavati UNIK-I) at a speed of 10 rpm. The active blend was loaded into the side one hopper and the active layer weight was adjusted to target weight. Then the blocking layer blend was loaded into the side two hoppers and the total tablet weight was adjusted to target weight. After weight adjustment, the compression run was started and the press was run at 10 rpm.
10. The aqueous Opadry® coating suspension was prepared by adding Opadry® Beige to the vortex. Once the Opadry® was incorporated in the purified water, the mixing was continued for about an hour prior to use.
11. Approximately 10 kg of the core tablets from step 9 were weighed out and spray-coated with the coating suspension to a target weight gain of about 1.0 % (by wt.) in a perforated 24 inch Compu-Lab pan coater (COMP-U-LAB 24). The tablet bed was warmed by setting the inlet air temperature to 55 °C. Once the exhaust temperature reached 39 °C, the film coating began at a pan speed of 12 rpm and a spray rate of approximately 45 mL/min. Film coating was continued until the target 1% weight gain was achieved.
12. The partially coated tablets from step 11 were cured in the perforated pan coater. The inlet temperature was set to 85 °C at a pan speed of approximately 12 rpm. The tablets were cured at an exhaust temperature of 72 °C for approximately 30 minutes.
13. After curing, the tablets were cooled in the rotating pan by setting the inlet temperature to 22 °C. Cooling was continued until the exhaust temperature was less than 28°C.
14. The cured tablets from step 13 were spray-coated with the coating suspension to a target final weight gain of 4.0 % (by wt.) in the perforated pan coater at a pan speed of 12 rpm and spray rate of approximately 45 mL/min.
15. The tablets were discharged.

**EXAMPLE 4**

Hydromorphone Hydrochloride Film Coated Bilayer Tablets, 32 mg.

In Example 4, two different bilayer tablets including 32 mg hydromorphone hydrochloride were prepared, each possessing an active layer of 500 mg and 400 mg, respectively, and a 200 mg polyethylene oxide blocking layer.

The compositions of the tablets in Examples 4A and 4B, respectively, are shown in Table 6.

Table 6

| | **Ex. 4A** | **Ex. 4B** | | |
|---|---|---|---|---|
| **Component** | **mg/tablet** | **mg/tablet** | **Function** | Ref. to Standard |
| **Active layer** | | | | |
| Hydromorphone hydrochloride | 32.0 | 32.0 | Active Ingredient | USP |
| Polyethylene oxide (WSR-303) | 465.5 | 366.0 | Release-Controlling Polymer | NF |
| Magnesium stearate | 2.5 | 2.0 | Lubricant | NF |
| **Active Layer Subtotal** | **500.0** | **400.0** | | |
| **Blocking Layer** | | | | |
| Polyethylene oxide (WSR-303) | 199.0 | 199.0 | Release-Controlling Polymer | NF |
| Magnesium stearate | 1.0 | 1.0 | Lubricant | NF |
| **Blocking Layer Subtotal** | **200.0** | **200.0** | | |
| **Coating** | | | | |
| Opadry II Beige 33G97430 | 28.0 | 24.0 | Cosmetic Coat | HSE[1] |
| Purified water[2] | N/A | N/A | Solvent | USP |
| **Total** | **728.0** | **624.0** | | |
| [1] HSE - In-house standard; [2] Not present in final product | | | | |

Based on the information presented in Table 6, the amounts of individual components of the active layer composition and the blocking layer composition of Examples 4A and 4B in weight percent as well as the ratio active layer / blocking layer may be calculated. This information is shown in Tables 7A, 7B and 7C, respectively.

Table 7A

| Components of the active layer in weight percent: | | |
|---|---|---|
| | **Ex. 4A** | **Ex. 4B** |
| **Component** | **% (by wt.)** | **% (by wt.)** |
| Hydromorphone hydrochloride | 6.40 | **8.00** |
| Polyethylene oxide (WSR-303) | 93.10 | 91.50 |
| Magnesium stearate | 0.50 | 0.50 |
| Total | 100.00 | 100.00 |

Table 7B

| Components of the blocking layer in weight percent: | | |
|---|---|---|
| | **Ex. 4A** | **Ex. 4B** |
| **Component** | **% (by wt.)** | **% (by wt.)** |
| Polyethylene oxide (WSR-303) | 99.50 | 99.50 |
| Magnesium stearate | 0.50 | 0.50 |
| Total | 100.00 | 100.00 |

Table 7C

| Ratio active layer / blocking layer: | | |
|---|---|---|
| | **Ex. 4A** | **Ex. 4B** |
| Ratio active layer / blocking layer | 2.50 | 2.00 |

The processing steps to manufacture the tablets are described herein below.
The batch sizes for Examples 4A and 4B were:

4A: 14.560 kg, 20,000 Tablets
4B: 13.728 kg, 22,000 Tablets

The process conditions for Examples 4A and 4B were essentially similar to those used in Example 3.

**EXAMPLE 5**

Dissolution of bilayer tablets comprising 20 mg or 120 mg hydrocodone bitartrate.
The dissolution of hydrocodone bitartrate tablets was carried out using USP apparatus I (e.g. Dissolution Apparatus I from Hanson Research equipped with USP 10 mesh baskets) with 10 mesh baskets. A stainless steel spring (e.g. a passivated stainless steel spring, 1-cm outside diameter and 2-cm length, Lee Spring Co. (P/N LC 036G 04 S316) was inserted into each basket containing a tablet. The basket was then rotated at 100 rpm in 900 ml simulated gastric fluid without enzymes (SGF) with a temperature maintained at 37°C. The samples (e.g. sampled by an automated dissolution sampling device equipped with in-residence sampling probes, and in line MinisartCA, 28 mm, 1.2 μm filters (P/N 17593 Q) were analyzed by reversed-phase high performance liquid chromatography (HPLC; e.g. using a Waters Alliance™ 2690/2695 HPLC system with 2487 UV-Vis absorbance detector or 996 photodiode array (PDA) detector) on a Waters SymmetryShield RP 18 (4.6 x 100 mm, 3.5 μm) column maintained at 60 °C using a mobile phase consisting of 31:69 acetonitrile:pH 2.1 of 10 mM sodium dodecyl sulfate and 20 mM sodium phosphate monobasic monohydrate buffer, flow rate 1.0 mL/min, with UV detection at 230 nm.

The test procedure comprises the following steps:

1. Assemble the dissolution apparatus. Adjust the height of all baskets 25 ± 2 mm from the bottom of each dissolution vessel.

2. Tranfer 900 mL of dissolution medium in each vessel. Heat the water bath so that the temperature of dissolution medium in all vessels is within 37.0 °C ± 0.5 °C.

3. Check the temperature of the dissolution medium in each vessel with a thermometer before beginning the test. The temperature of the dissolution medium in each vessel must be 37.0 ± 0.5 °C.

4. Place one tablet into each USP 10 mesh basket and horizontally insert a stainless steel spring in the top of the basket.

5. Attach baskets containing the tablets to the height-adjusted shafts.

6. Rotate the shafts at 100 rpm, and lower the shafts to the predetermined height so that the bottom of the basket is 25 ± 2 mm from the vessel bottom.

7. At the times specified in the instructions or as required, withdraw and filter sufficient amount of sample aliquot from each vessel. Transfer about 1 mL of the samples to each HPLC vial.

8. Inject 10 μl for all solutions. No more than 12 sample solutions should be injected between standard solution injection.

9. Calculate the % hydrocodone bitartrate dissolved for each tablet at each time point as follows:

$$\%Hydrocodone\ Bitartrate\ Dissolved = \frac{A_U}{A_{STD}} x\ C_{STD}\ x \frac{900mL}{1\,tablet} x \frac{100}{LC}$$

<u>where</u>
$A_U$ = Area of the Hydrocodone peak in the sample chromatogram
$A_{STD}$ = Area of the Hydrocodone peak in the standard chromatogram
LC = Label claim for the particular potency (20 or 120 mg)
$C_{STD}$ = Concentration of Hydrocodone bitartrate corrected for purity in the working standard solution, mg/mL,

When using Hydrocodone Bitartrate WRS:

$$C_{STD} = \frac{Wstd}{100mL} x \frac{12\,mL}{100mL} x \frac{\%P_{WRS}}{100}$$

When using the dried Hydrocodone bitartrate USP RS:

$$C_{STD} = \frac{Wstd}{100mL} x \frac{12\,mL}{100mL} x \frac{\%P_{USP}}{100} x\,1.1002$$

Wstd= Weight of Hydrocodone bitartrate RS in mg
$\%P_{WRS}$ = Percent purity of Hydrocodone bitartrate · 2 ½ $H_2O$ WRS (as is)
$\%P_{USP}$= Percent purity of Hydrocodone bitartrate anhydrous USP RS (on dried basis)
1.1002 = Conversion factor from Hydrocodone bitartrate to Hydrogene bitartrate · 2 ½ $H_2O$

When a total of more than 10 mL aliquot is taken from each vessel during the dissolution run, volume correction should be applied in the calculation.

The results of the dissolution tests are shown in Tables 8 A and 8 B (for tablets comprising 20 mg hydrocodone bitartrate) and Table 9 (for tablets comprising 120 mg hydrocodone bitartrate) below.

The time of the measurement and the percentage dissolution (mean as well as minimum or maximum values) are shown for the tablets produced in Examples 1A-1C as well as in Example 2A-2C.

Table 8 A)

| **Time (h)** | **Example 1A** Mean (Min/Max) | | |
|---|---|---|---|
| 0.5 | 12 (12/13) | | |
| 1.5 | 21 (21/22) | | |
| 3.5 | 37 (36/38) | | |
| 7.5 | 65 (63/68) | | |
| 11.5 | 83 (80/88) | | |
| 17.5 | 97 (94/103) | | |
| 23.5 | 101 (92/109) | | |
| | | | |
| **Time (h)** | **Example 1B** Mean (Min/Max) | **non cumulative release** | **non cumulative release/h from hour 2 to 12** |
| 1 | 12 (11/13) | | |
| 2 | 20 (19/21) | | |
| 4 | 35 (33/37) | 15 | 7.5 (hours 2 to 4) |
| 8 | 59 (56/63) | 24 | 6 (hours 4 to 8) |
| 12 | 78 (74/82) | 19 | 4.75 (hours 8 to 12) |
| 18 | 93 (87/98) | | |
| 24 | 100 (95/105) | | |
| Mean hourly release between hour 2 and 12 and corresponding zero order range | | | 5.8 (30%=±1.7) 4.1-7.5 |

Table 8 B)

| **Time (h)** | **Example 1C** Mean (Min/Max) | **non cumulative release** | **non cumulative release/h from hour 2 to 12** |
|---|---|---|---|
| 1 | 12 (11/12) | | |
| 2 | 20 (19/20) | | |
| 4 | 33 (33/13) | 13 | 6.5 (hours 2 to 4) |
| 8 | 55 (54/56) | 22 | 5.5 (hours 4 to 8) |
| 12 | 72 (71/73) | 17 | 4.25 (hours 8 to 12) |
| 18 | 88 (85/90) | | |
| 24 | 101 (98/103) | | |
| Mean hourly release between hour 2 and 12 and corresponding zero order range | | | 5.2 (30%=± 1.6) 3.6-6.8 |

Table 9

| Time (h) | **Example 2A** Mean (Min/Max) | **non cumulative release** | **non cumulative release/h from hour 2 to 12** |
|---|---|---|---|
| 1 | 13 (12/13) | | |
| 2 | 21 (21/22) | | |
| 4 | 37 (36/39) | 16 | 8 (hours 2 to 4) |
| 8 | 64 (63/67) | 27 | 6.75 (hours 4 to 8) |
| 12 | 82 (81/85) | 18 | 4.5 (hours 8 to 12) |
| 18 | 95 (93/96) | | |
| 24 | 98 (97/100) | | |
| Mean hourly release between hour 2 and 12 and corresponding zero order range | | | 6.1 (40% = ± 2.4) 3.7 – 8.5 |

| Time (h) | **Example 2B** Mean (Min/Max) | **non cumulative release** | **non cumulative release/h from hour 2 to 12** |
|---|---|---|---|
| 1 | 13 (13/15) | | |
| 2 | 22 (21/24) | | |
| 4 | 36 (34/38) | 14 | 7 (hours 2 to 4) |
| 8 | 59 (57/63) | 23 | 5.75 (hours 4 to 8) |
| 12 | 78 (75/82) | 19 | 4.75 (hours 8 to 12) |
| 18 | 94 (91/99) | | |
| 24 | 102 (98/107) | | |
| Mean hourly release between hour 2 and 12 and corresponding zero order range | | | 5.6 (30% = ± 1.7) 3.9 – 7.3 |

| Time (h) | Example 2C Mean (Min/Max) | non cumulative release | non cumulative release/h from hour 2 to 12 |
|---|---|---|---|
| 1 | 13 (12/14) | 13 | |
| 2 | 21 (20/22) | 8 | |
| 4 | 34 (33/35) | 13 | 7.5 (hours 2 to 4) |
| 8 | 55 (12/13) | 21 | 5.25 (hours 4 to 8) |
| 12 | 71 (69/72) | 16 | 4 (hours 8 to 12) |
| 18 | 86 (84/88) | | |
| 24 | 94 (93/96) | | |
| Mean hourly release between hour 2 and 12 and corresponding zero order range | | | 5 (50% = ± 2.5) 2.5 – 7.5 |

**EXAMPLE 6**

Dissolution of bilayer tablets comprising 12 mg and 32 mg hydromorphone HCl

The dissolution of hydromorphone hydrochloride tablets was carried out using a modified USP Apparatus I (e.g. Dissolution Apparatus I from Hanson Research equipped with USP 10 mesh baskets) with 10 mesh baskets. The modification to the USP Apparatus I consisted of inserting a stainless steel spring on top of USP 10 mesh baskets. The stainless steel spring (e.g. a passivated stainless steel spring, 1-cm outside diameter and 2-cm length, Lee Spring Co. (P/N LC 036G 04 S316) was inserted into each basket containing a tablet. The basket was then rotated at 75 rpm in 900 ml simulated gastric fluid without enzymes (SGF) with a temperature maintained at 37°C. The samples (e.g. sampled by an automated dissolution sampling device equipped with in-residence sampling probes, and in-line 25,mm Glass fiber 1.0-μm filters (Waters P/N WAT200818) or 10 canula filter (QLA P/N FIL010-01) were analyzed by reversed-phase high performance liquid chromatography (HPLC; e.g. using a Waters Alliance™ 2690/2695 HPLC system with 2487 or 2489 UV-Vis absorbance detector or 996 photodiode array (PDA) detector) on a Waters Novapak C18, 3.9 x 150 mm, 4 μm column kept at 30 °C using a mobile phase consisting of a mixture of acetonitrile, sodium dodecyl sulfate, monobasic sodium phosphate buffer and water with a final pH of 2.9 at a flow rate of 1.5 mL/min, with UV detection at 220 nm.

The test procedure comprises the following steps:

1. Assemble the dissolution apparatus. Adjust the height of all baskets 25 ± 2 mm from the bottom of each dissolution vessel.

2. Tranfer 900 mL of dissolution medium in each vessel. Heat the water bath so that the temperature of dissolution medium in all vessels is within 37.0 °C ± 0.5 °C.

3. Check the temperature of the dissolution medium in each vessel with a thermometer before beginning the test. The temperature of the dissolution medium in each vessel must be 37.0 ± 0.5 °C.

4. Place one tablet into each USP 10 mesh basket and horizontally insert a stainless steel spring on top of the basket.

5. Attach baskets containing the tablets to the height-adjusted shafts.

6. Rotate the shafts at 100 rpm, and lower the shafts to the predetermined height so that the bottom of the basket is 25 ± 2 mm from the vessel bottom.

7. At the times specified in the instructions or as required, withdraw and filter sufficient amount of sample aliquot from each vessel. Transfer about 1 mL of the samples to each HPLC vial. Cap vials before performing the HPLC analysis. When sampling, ensure that the sampling apparatus has a pre-wash cycle of at least 3 mL before sample collection.

8. Inject 20 μl for all solutions. No more than 12 sample solutions should be injected between standard solution injection.

9. Calculate the % hydromorphone hydrochloride dissolved for each tablet at each time point as follows:

The % hydromorphone HCl dissolved for each tablet at each time point was calculated according to the following equation:

$$\%Hydromorphone\ HCl\ Dissolved = \frac{A_U}{A_{STD}}\ x\ C_{STD}\ x\ \frac{900mL}{1\ tablet}\ x\ \frac{100}{LC}$$

where:

$A_U$ =Area of the hydromorphone peak in the sample chromatogram
$A_{STD}$ = Area of the hydromorphone peak in the standard chromatogram
LC =Label claim for the particular potency (12, 16, 24, or 32 mg)
$C_{STD}$ = Concentration of Hydrocodone bitartrate corrected for purity in the working standard solution, mg/mL

$$C_{STD} = \frac{WtStd}{100mL}\ x\ \frac{15\ mL}{200mL}\ x\ \frac{\%purity}{100}$$

When a total of more than 10 mL aliquot is taken from each vessel, volume correction method should be applied for calculation.

The results are shown in Tables 10 and 11 below. The time of the measurement and the percentage dissolution (mean as well as minimum or maximum values) are shown for the tablets produced in Examples 3A and 3B, and for the tablets produced in Examples 4A and 4B, respectively:

Table 10

| **Time (h)** | **Example 3A** Mean (Min/Max) | **non cumulative release** | **non cumulative release/h from hour 2 to 12** |
|---|---|---|---|
| 1 | 8.46 (7.94/8.80) | | |
| 2 | 14.17 (13.53/14.90) | | |
| 4 | 24.12 (23.37/25.06) | 10.0 | 5.0 (hours 2 to 4) |
| 8 | 43.63 (43.03/44.24) | 19.5 | 4.9 (hours 4 to 8) |
| 12 | 60.93 (59.85/62.28) | 17.3 | 4.3 (hours 8 to 12) |
| 18 | 76.61 (78.09/81.43) | | |
| 24 | 90.75 (88.60/92.69) | | |
| 36 | 98.40 (96.03/100.80) | | |
| Mean hourly release between hour 2 and 12 and corresponding zero order range | | | 4.7 (20% = ± 0.9) 3.8 – 5.6 |

| Time (h) | **Example 3B** Mean (Min/Max) | **non cumulative release** | **non cumulative release/h from hour 2 to 12** |
|---|---|---|---|
| 1 | 9.45 (8.93/9.77) | | |
| 2 | 16.22 (15.49/17.25) | | |
| 4 | 27.32 (25.56/28.82) | 11.1 | 5.6 (hours 2 to 4) |
| 8 | 47.53 (45.03/49.28) | 20.2 | 5.1 (hours 4 to 8) |
| 12 | 64.96 (62.76/67.21) | 17.4 | 4.4 (hours 8 to 12) |
| 18 | 82.51 (80.50/84.70) | | |
| 24 | 91.94 (88.98/94.54) | | |
| 36 | 97.54 (95.12/99.78) | | |
| Mean hourly release between hour 2 and 12 and corresponding zero order range | | | 4.9 (20% = ±1) 3.9 – 5.9 |

Table 11

| Time (h) | Example 4A Mean (Min/Max) | non cumulative release | non cumulative release/h from hour 2 to 12 |
|---|---|---|---|
| 1 | 9.00 (8.67/9.45) | | |
| 2 | 15.27 (14.72/15.73) | | |
| 4 | 26.13 (25.15/26.66) | 10.9 | 5.6 (hours 2 to 4) |
| 8 | 45.71 (44.73/46.49) | 19.6 | 4.9 (hours 4 to 8) |
| 12 | 63.47 (62.37/64.33) | 17.8 | 4.6 (hours 8 to 12) |
| 18 | 83.01 (82.16/84.25) | | |
| 24 | 94.37 (93.41/94.96) | | |
| 36 | 103.12 (101.99/104.69) | | |
| Mean hourly release between hour 2 and 12 and corresponding zero order range | | | 4.8 (20% = ± 1) 3.8 – 5.8 |

| Time (h) | Example 4B Mean (Min/Max) | non cumulative release | non cumulative release/h from hour 2 to 12 |
|---|---|---|---|
| 1 | 10.03 (9.68/10.44) | | |
| 2 | 16.80 (16.35/17.61) | | |
| 4 | 28.07 (27.38/29.11) | 11.3 | 5.7 (hours 2 to 4) |
| 8 | 48.10 (47.00/47.79) | 20.0 | 5 (hours 4 to 8) |
| 12 | 65.80 (65.00/67.83) | 17.7 | 4.4 (hours 8 to 12) |
| 18 | 84.17 83.14/86.08)( | | |
| 24 | 94.44 (93.06/96.67) | | |
| 36 | 101.09 (99.73/103.29) | | |
| Mean hourly release between hour 2 and 12 and corresponding zero order range | | | 4.9 (20% = ± 1) 3.9 – 5.9 |

EXAMPLE 7

Pharmacokinetics of bilayer tablets comprising 20 mg and 120 mg hydrocodone bitartrate

In Example 7, a randomized, open-label, single-dose, four-treatment, four-period, crossover study in healthy adult male and female subjects was conducted to assess the pharmacokinetic characteristics of six hydrocodone formulations (20 mg of hydrocodone bitartrate, formulations of Examples 1A, 1B, and 1C as well as 120 mg of hydrocodone bitartrate, formulations of Examples 2A, 2B and 2C) in the fasted (all Examples) and fed state (1 B and 2B).

The formulations were each administered orally with 8 oz. (240 mL) water as a single dose in the fasted or fed state.

As this study was conducted in healthy human subjects, the opioid antagonist naltrexone hydrochloride was administered to minimize opioid-related adverse events.

**Subject selection**

*Screening procedures*

The following screening procedures were performed for all potential subjects at a screening visit conducted within 28 days prior to first dose administration:

- Informed consent.
- Informed consent for optional pharmacogenomic sampling.
- Informed consent for optional hair sampling.
- Weight, height, body mass index (BMI), and demographic data.
- Evaluation of inclusion/exclusion criteria.
- Medical and medication history, including concomitant medication.
- Vital signs (systolic/diastolic blood pressure, pulse rate, respiration rate, oral temperature) after being seated for approximately 5 minutes and $SpO_2$
- Additional vital signs (systolic/diastolic blood pressure, and pulse rate) after standing for approximately 2 minutes.
- HDYF? Inquiry was performed at the same time vital signs are measured.
- Routine physical examination.
- Clinical laboratory evaluations following at least a 4 hour fast (including biochemistry, hematology, and urinalysis).
- 12-lead ECG. QTcF not to exceed 450 msec.
- Screens for hepatitis (including hepatitis B surface antigen [HBsAg], hepatitis C antibody [anti-HCV]).
- Screens for alcohol, cotinine, and selected drugs of abuse.
- Serum pregnancy test, female subjects only; Serum follicle stimulating hormone (FSH) postmenopausal females only;.
- Serum pregnancy test (female subjects only).
- Serum follicle stimulating hormone (FSH) test (postmenopausal females only)

*Inclusion criteria*

- Subjects who met the following criteria were included in the study.
- Provided written informed consent.
- Males and females aged 18 to 50, inclusive.

- Body weight ranging from 50 to 100 kg (110 to 220 lbs) and a BMI 18 to 34 (kg/$m^2$), inclusive.

- Healthy and free of significant abnormal findings as determined by medical history, physical examination, vital signs, and ECG.

- Females of child-bearing potential must be using an adequate and reliable method of contraception (i.e, barrier with additional spermicidal foam or jelly, intra-uterine device, hormonal contraception). Females who are postmenopausal must have been postmenopausal ≥ 1 year and have elevated serum FSH.

- Willing to eat the food supplied during the study.

- Will refrain from strenuous exercise during the entire study. Subjects will not begin a new exercise program nor participate in any unusually strenuous physical exertion.

*Exclusion criteria*

The following criteria excluded potential subjects from the study.

- Females who are pregnant (positive beta human chorionic gonadotropin test) or lactating.

- Current or recent (within 5 years) history of drug or alcohol abuse.

- History or any current conditions that might interfere with drug absorption, distribution, metabolism or excretion.

- Use of an opioid-containing medication in the past 30 days preceding the initial dose in this study.

- History of known sensitivity to hydrocodone, naltrexone or related compounds.

- Any history of frequent nausea or emesis regardless of etiology.

- Any history of seizures or head trauma with sequelae.

- Participation in a clinical drug study during the 30 days preceding the initial dose in this study.

- Any significant illness during the 30 days preceding the initial dose in this study.

- Use of any medication including thyroid hormonal therapy (hormonal contraception is allowed), vitamins, herbal and/or mineral supplements during the 7 days preceding the initial dose.

- Abnormal cardiac conditions including any of the following:

    - QTc interval ≥ 450 msec (calculated using Fridericia's correction) at screening.
    - QTc interval ≥ 480 msec (calculated using Fridericia's correction) during Treatment period.

- Refusal to abstain from food 10 hours preceding and 4 hours following study drug administration and to abstain from caffeine or xanthine containing beverages entirely during each confinement.

- Refusal to abstain from consumption of alcoholic beverages 48 hours prior to initial study drug administration (day 1) and anytime during study.

- History of smoking or use of nicotine products within 45 days of study drug administration or a positive urine cotinine test.

- Blood or blood products donated within 60 days prior to study drug administration or anytime during the study and for 30 days after completion of the study, except as required by this protocol.

- Plasma donated within 14 days prior to study drug administration or any time during the study, except as required by this protocol.

- Positive results of urine drug screen or alcohol screen.
- Positive results of HBsAg, anti-HCV.
- Positive naloxone HCl challenge test.
- Presence of Gilbert's Syndrome, or any known hepatobiliary abnormalities.
- For the optional hair sampling portion of the study only, an insufficient amount of scalp hair to provide an adequate sample.
- The investigator believes the subject to be unsuitable for reason(s) not specifically stated in the exclusion criteria.

Subjects meeting all the inclusion criteria and none of the exclusion criteria were randomized into the study.

Each subject was assigned a unique subject number at screening. Assignment of subject numbers was in ascending order and no numbers were omitted. Subject numbers were used on all study documentation.

**Check-in** Procedures

On Day -1 of Period 1 only, subjects were admitted to the study unit and received a Naloxone HCl challenge test. The results of the test had to be negative for subjects to continue in the study. Vital signs and $SPO_2$ were measured prior to and following the Naloxone HCl.

The following procedures were also performed for all subjects at Check-in for each period:

- Verification of inclusion/exclusion criteria, including verification of willingness to comply with caffeine and xanthine restriction criteria.
- Vital signs (after being seated for approximately 5 minutes) and SpO2.
- HDYF(How do you feel) ? Inquiry was performed at the same time vital signs are measured.
- Clinical laboratory evaluations (day -1, period 1 only) including biochemistry (fasting for at least 4 hours), hematology and urinalysis; Appendix A) were collected after vital signs and $SpO_2$ were measured.
- Screen for alcohol (via urine or blood alcohol or breathalyzer test), cotinine, and selected drugs of abuse (via urine testing). See Appendix A.
- Urine pregnancy test (for all female subjects; Appendix A).
- Concomitant medication monitoring and recording.
- AE monitoring and recording.

For subjects to continue their participation in the study, the results of the drug screen (including alcohol and cotinine) had to be available and negative prior to dosing. In addition, continued compliance with concomitant medication and other restrictions were verified at Check-in and throughout the study in the appropriate source documentation.

**Treatment Period Procedures**

Treatments to be studied were predetermined for each Iteration. Within an Iteration, as data became available, treatments were dropped between cohorts. Dropped treatments were replaced with repeats of remaining treatments.

- Prior to the first dose in period 1, subjects were randomized to a treatment sequence.
- Subjects will received naltrexone HCl tablets (50 mg) with 240 mL of water at -12 h prior to study drug dosing.
- Prior to study drug administration (except period 1), subjects had chemistry (fasting for at least 4 hours), hematology and urinalysis tests performed.

- Subjects were administered the study drug with 240 mL of water as follows:

• <u>For Fasted Treatment:</u>

Following a 10-hour overnight fast, subjects were administered study drug with 240 mL of water. Subjects receiving fasted treatment continued fasting from food for 4 hours following dosing.

• <u>For Fed Treatments</u>:

Following a 10-hour overnight fast, the subjects were fed a standard meal (FDA high-fat breakfast, Appendix E) 30 minutes prior to administration of study drug with 240 mL of water. No food was allowed for at least 4 hours post-dose. It was made very clear to the subjects that all of the meal should be consumed within the designated time-frame.

- Subjects were standing or in an upright sitting position while receiving their dose of study drug.
- Fasting was not required for nondosing study days.
  - Subjects will received naltrexone HCl 50-mg tablets with 240 mL of water at -12, 0, 12, 24, and 36 hours relative to each study drug dosing.
  - For subjects receiving hydrocodone doses of 60 mg or more, $SpO_2$ was monitored continuously beginning prior to dosing and continuing through 24 hours post-dose.
  - Vital signs (after being seated for approximately 5 minutes) and $SpO_2$, were obtained pre-dose and at hour 1, 2, 4, 6, 8, 12, 24, 36, 48, and 72 hour post dose for each period.
  - HDYF (How do you feel) ? Inquiry was performed at the same time vital signs were measured.
  - Subjects will had biochemistry (fasting for at least 4 hours), hematology, and urinalysis tests performed 24 hours post-dose.
  - In addition, 12-lead ECGs were performed for each subject pre-dose and approximately 12, 24 and 48 hours post-dose. If QTcF exceeded 480 msec the subject was discontinued due to the reason of Adverse Event.
  - Blood samples for determining oxycodone plasma concentrations were obtained for each subject at pre-dose and at 0.5, 1, 1.5, 2, 2.5, 3, 3.5, 4, 5, 6, 8, 10, 12, 14, 18 24, 36, 48, and 72 hours post-dose for each period.
  - Subjects were confined to the unit from check-in to the unit on the day before dosing until the time that their 48 h procedures were completed. The subjects returned to the unit for the 72 h procedures.
  - During the study, AEs and concomitant medications were recorded.

In addition, the subjects were informed that it is very important to report any/all episodes of emesis to the study staff immediately and that this information is crucial to the proper conduct and outcome of the trial. The subjects were informed that they would not be penalized in any way due to reporting cases of emesis. The study staff was instructed to carefully document any/all cases of emesis.

The treatment sequences for this study are presented below:

Iteration

- HYD 20 mg, slow release tablet (Ex 1A), fasted state
- HYD 20 mg, medium release tablet(Ex 1B), fasted state
- HYD 20 mg, fast release tablet(Ex 1C), fasted state
- HYD 20 mg, medium release tablet (Ex 1B), fed state

Iteration 2

- HYD 120 mg, slow release tablet (Ex 2A), fasted state

- HYD 120 mg, medium release tablet (Ex 2B), fasted state
- HYD 120 mg, fast release tablet (Ex 2C), fasted state
- HYD 120 mg, medium release tablet (Ex 2B), fed state

Following a review of pharmacokinetic data from Cohort 1 subjects in Iterations 1 and 2, it was determined that two of the four treatments studied in Cohort 1 of each Iteration would not be further studied in Cohort 2: Medium release, fed and Fast release, fasted. These dropped treatments were replaced by repeats of the two remaining treatments: Slow release, fasted and medium release, fasted. See Figure 2.

**Study Completion Procedures**

The following procedures were performed at the study site for all subjects at end-of-study (study completion), 7 to 10 days after receiving their last dose of study drug or upon early discontinuation from the study.

- Concomitant medication evaluation.
- Vital signs (after being seated for approximately 5 minutes) and $SpO_2$.
- HDYF? Inquiry was performed at the same time vital signs are measured.
- Physical examination.
- 12-Lead ECG.
- Clinical laboratory evaluations (including biochemistry [fasted at least 4 hours], hematology, and urinalysis;).
- AE evaluations.
- Serum pregnancy test (for female subjects only;).

The pharmacokinetic results of this study are shown in Table 12 as well as Figures 3 to 6.

**Table 12:** Summary of Draft Plasma Hydrocodone Pharmacokinetic Parameters

| | | Iteration 1: 20 mg | | | Itera ation 2: 120 mg | | | 20 mg | 120 mg |
|---|---|---|---|---|---|---|---|---|---|
| | | Slow Fasted | Medium Fasted | Fast Fasted | Slow Fasted | Medium Fasted | Fast Fasted | Medium Fed | Medium Fed |
| Parameter (Unit) | Statistic | (N=51) | (N=51) | (N=16) | (N=49) | (N=53) | (N=18) | (N=15) | (N=15) |
| AUCt (ng*h/mL) | MEAN | 270 | 274 | 279 | 1762 | 1898 | 1962 | 312 | 2073 |
| | SD | 82 | 86 | 65 | 547 | 502 | 464 | 75 | 454 |
| | MIN | 73 | 66 | 183 | 705 | 781 | 1335 | 183 | 1398 |
| | MAX | 449 | 452 | 421 | 2950 | 3095 | 2748 | 460 | 2872 |
| AUCinf (ng*h/mL) | Mean | 279 | 278 | 283 | 1773 | 1910 | 1971 | 316 | 2082 |
| | SD | 81 | 87 | 65 | 550 | 506 | 468 | 76 | 461 |
| | Min | 76 | 70 | 186 | 711 | 783 | 1337 | 185 | 1398 |
| | Max | 451 | 462 | 423 | 2968 | 3106 | 2784 | 467 | 2905 |
| Cmax (ng/mL) | Mean | 12.2 | 12.8 | 14.9 | 82.6 | 90.0 | 95.8 | 18.8 | 120.2 |
| | SD | 3.7 | 3.9 | 4.1 | 22.1 | 22.9 | 24.8 | 4.6 | 26.0 |
| | Min | 4.8 | 6.8 | 7.2 | 46.4 | 55.7 | 61.8 | 11.9 | 66.6 |
| | Max | 22.3 | 23.4 | 23.4 | 158.0 | 168.0 | 162.0 | 26.9 | 150.0 |
| Tmax (h) | Mean | 7.4 | 7.8 | 7.5 | 6.3 | 8.0 | 8.2 | 10.1 | 10.7 |
| | SD | 3.6 | 3:4 | 2.8 | 2.0 | 3.1 | 3.1 | 1.8 | 3.5 |

(continued)

| Parameter (Unit) | Statistic | Iteration 1: 20 mg Slow Fasted (N=51) | Medium Fasted (N=51) | Fast Fasted (N=16) | Itera ation 2: 120 mg Slow Fasted (N=49) | Medium Fasted (N=53) | Fast Fasted (N=18) | 20 mg Medium Fed (N=15) | 120 mg Medium Fed (N=15) |
|---|---|---|---|---|---|---|---|---|---|
| | Min | 2 | 4 | 5 | 3 | 5 | 5 | 6 | 5 |
| | Median | 6 | 6 | 7 | 6 | 8 | 8 | 10 | 10 |
| | Max | 18 | 18 | 14 | 14 | 18 | 14 | 12 | 18 |
| T1/2 (h) | Mean | 9.7 | 7.7 | 7.6 | 8.4 | 8.1 | 8.1 | 8.8 | 8.9 |
| | SD | 6.3 | 2.5 | 2.3 | 3.3 | 2.8 | 3.4 | 4.5 | 3.5 |
| | Min | 4.6 | 4.6 | 4.4 | 4.1 | 3.9 | 3.9 | 4.4 | 4.7 |
| | Max | 46.1 | 15.5 | 10.9 | 19.9 | 15.5 | 15.9 | 17.2 | 14.6 |
| Tlag (h) | Mean | 0.04 | 0.04 | 0.19 | 0.00 | 0.03 | 0.00 | 0.20 | 0.07 |
| | SD | 0.13 | 0.13 | 0.40 | 0.00 | 0.11 | 0.00 | 0.25 | 0.17 |
| | Min | 0 | 0 | 0 | 0 | 0 | 0 | 0 | 0 |
| | Max | 0.5 | 0.5 | 1.5 | 0 | 0.5 | 0 | 0.5 | 0.5 |
| C24/Cmax | Mean | 0.49 | 0.45 | 0.38 | 0.45 | 0.46 | 0.44 | ND | ND |
| | SD | 0.20 | 0.21 | 0.17 | 0.20 | 0.19 | 0.14 | ND | ND |
| | Min | 0.05 | 0.05 | 0.12 | 0.06 | 0.04 | 0.12 | ND | ND |
| | Max | 0.92 | 0.82 | 0.65 | 0.81 | 0.92 | 0.63 | ND | ND |

ND = Not done

The results in Table 12 show that the exemplified formulations provide the pharmacokinetic characteristics as described and claimed herein.

The present invention is hot to be limited in scope by the specific embodiments disclosed in the examples, which are intended as illustrations of a few aspects of the invention, and any embodiments that are functionally equivalent are within the scope of this invention. Indeed, various modifications of the invention in addition to those shown and described herein will become apparent to those skilled in the art and are intended to fall within the scope of the appended claims.

This application claims priority from U.S. Provisional Application Serial No. 61/467,824, filed March 25, 2011.

## Claims

**1.** A solid oral extended release pharmaceutical dosage form comprising a multi-layered extended release matrix formulation with sandwich-type or half-sandwich-type structure, the extended release matrix formulation comprising

(1) a first composition forming an active agent-containing first layer of the extended release matrix formulation comprising:

(a) at least one polyethylene oxide having, based on rheological measurements, an approximate molecular weight of at least 1,000,000; and
(b) at least one active agent; and

(2) a second composition forming an active agent-free second layer of the extended release matrix formulation comprising at least one polyethylene oxide.

**2.** The solid oral extended release pharmaceutical dosage form according to claim 1, wherein the second composition comprises at least one polyethylene oxide having, based on rheological measurements, an approximate molecular weight of at least 1,000,000.

3. The solid oral extended release pharmaceutical dosage form according to claim 1, wherein the second composition comprises at least one polyethylene oxide having, based on rheological measurements, an approximate molecular weight of less than 1,000,000.

4. The solid oral extended release pharmaceutical dosage form according to any one of claims 1 to 3, wherein the active agent is selected from opioid analgesics.

5. The solid oral extended release pharmaceutical dosage form according to any one of claims 1 to 4, wherein the multi-layered extended release matrix formulation is a bilayer extended release matrix formulation.

6. The solid oral extended release pharmaceutical dosage form according to claim 5, wherein the weight ratio of active agent-containing layer / active agent free layer ranges from about 1 to about 5, or from about 1.5 to about 3, or is about 2 or is about 2.5.

7. The solid oral extended release pharmaceutical dosage form according to anyone of claims 1 to 6, wherein the extended release matrix formulation is thermoformed or subjected to a curing step.

8. The solid oral extended release pharmaceutical dosage form according to claim 7, wherein the extended release matrix formulation is subjected to a curing step at a temperature of at least about 60 °C for a time period of at least about 1 minute.

9. The solid oral extended release pharmaceutical dosage form according to anyone of claims 1 to 8, wherein the active agent-containing layer and the active agent free layer comprise less than 25 % lactose, or essentially no lactose.

10. The solid oral extended release pharmaceutical dosage form according to anyone of claims 1 to 9, wherein the active agent-containing layer and the active agent free layer comprise essentially no hydrogenated castor oil and / or essentially no hydroxypropylmethylcellulose.

11. The solid oral extended release pharmaceutical dosage form of anyone of claims 1 to 10, wherein the dosage provides a dissolution rate, which when measured in a USP Apparatus 1 (basket) at 100 rpm in 900 ml simulated gastric fluid without enzymes (SGF) at 37° C releases the active agent essentially according to a zero order mode.

12. The solid oral extended release pharmaceutical dosage form of anyone of claims 1 to 11, wherein the dosage provides a dissolution rate, which when measured in a USP Apparatus 1 (basket) at 100 rpm in 900 ml simulated gastric fluid without enzymes (SGF) at 37° C is from about 5% to about 15% (by wt.) active released per hour and / or is from about 5% to about 15% (by wt.) active released after 1 hour, and / or is from about 10% to about 30% (by wt.) active released after 2 hours, and / or is from about 20% to about 60% (by wt.) active released after 4 hours and / or is from about 40% to about 100% (by wt.) active released after 8 hours.

13. The solid oral extended release pharmaceutical dosage form of anyone of claims 1 to 12, wherein the first composition comprises at least about 60 % (by wt.), at least about 70 % (by wt.), at least about 80 % (by wt.), at least about 90 % (by wt.) of said polyethylene oxide.

14. The solid oral extended release pharmaceutical dosage form according to any of claims 1 to 13, wherein the opioid analgesic is selected from the group of alfentanil, allylprodine, alphaprodine, anileridine, benzylmorphine, bezitramide, buprenorphine, butorphanol, clonitazene, codeine, desomorphine, dextromoramide, dezocine, diampromide, diamorphone, dihydrocodeine, dihydromorphine, dimenoxadol, dimepheptanol, dimethylthiambutene, dioxaphetyl butyrate, dipipanone, eptazocine, ethoheptazine, ethylmethylthiambutene, ethylmorphine, etonitazene, etorphine, dihydroetorphine, fentanyl and derivatives, hydrocodone, hydromorphone, hydroxypethidine, isomethadone, ketobemidone, levorphanol, levophenacylmorphan, lofentanil, meperidine, meptazinol, metazocine, methadone, metopon, morphine, myrophine, narceine, nicomorphine, norlevorphanol, normethadone, nalorphine, nalbuphene, normorphine, norpipanone, opium, oxycodone, oxymorphone, papaveretum, pentazocine, phenadoxone, phenomorphan, phenazocine, phenoperidine, piminodine, piritramide, propheptazine, promedol, properidine, propoxyphene, sufentanil, tilidine, tramadol, pharmaceutically acceptable salts, hydrates and solvates thereof, mixtures of any of the foregoing.

15. The solid oral extended release pharmaceutical dosage form according to any of claims 1 to 14, wherein the opioid analgesic is selected from the group of hydrocodone and hydromorphone or pharmaceutically acceptable salts, hydrates and solvates thereof, mixtures of any of the foregoing.

**16.** The solid oral extended release pharmaceutical dosage form according to any of claims 1 to 15, wherein the opioid analgesic is hydrocodone bitartrate or hydromorphone hydrochloride.

**17.** The solid oral extended release pharmaceutical dosage form according to any of claims 1 to 16, wherein the dosage form comprises from about 5 mg to about 250 mg of hydrocodone bitartrate or 1 mg to 100 mg of hydromorphone hydrochloride, and / or wherein the dosage form comprises 5 mg, 7.5 mg, 10 mg, 15 mg, 20 mg, 30 mg, 40 mg, 45 mg, 60 mg, or 80 mg, 90 mg, 100 mg, 120 mg or 160 mg of hydrocodone bitartrate or 2 mg, 5 mg, 7.5 mg, 10 mg, 15 mg, 20 mg, 25 mg, 30 mg, 32 mg, or 64 mg of hydromorphone hydrochloride.

**18.** The solid oral extended release pharmaceutical dosage form of claims 16 or 17, wherein the first composition comprises at least about 65 % (by wt.) polyethylene oxide having, based on rheological measurements, an approximate molecular weight of at least 1,000,000.

**19.** A solid oral extended release pharmaceutical dosage form according to claim 1 comprising an extended release matrix formulation, the extended release matrix formulation comprising:

(1) a first composition forming an active agent-containing first layer of said extended release matrix formulation comprising at least 65 % (by wt.) of one polyethylene oxide having, based on rheological measurements, an approximate molecular weight of at least 1,000,000; and
(2) about 5 mg, 10 mg, 15 mg, 20 mg or 40 mg, 60 mg, 80 mg, 100 mg or 120 mg hydrocodone bitartrate.

**20.** A solid oral extended release pharmaceutical dosage form according to claim 1 comprising an extended release matrix formulation, the extended release matrix formulation comprising:

(1) a first composition forming an active agent-containing first layer of said extended release matrix formulation comprising at least 90 % (by wt.) of one polyethylene oxide having, based on rheological measurements, an approximate molecular weight of at least 1,000,000; and
(2) about 5 mg hydrocodone bitartrate.

**21.** A solid oral extended release pharmaceutical dosage form according to claim 1 comprising an extended release matrix formulation, the extended release matrix formulation comprising:

(1) a first composition forming an active agent-containing layer first layer of said extended release matrix formulation comprising at least 90 % (by wt) of one polyethylene oxide having, based on rheological measurements, an approximate molecular weight of at least 1,000,000; and
(2) about 5 mg, 6 mg, 7 mg, 8 mg, 10, mg, 12 mg, 15 mg, 20 mg, 25 mg, 30 mg or 32 mg hydromorphone hydrochloride.

**22.** A solid oral extended release pharmaceutical dosage form according to claims 1 to 21, comprising a second composition forming an active agent-free layer second layer of the pharmaceutical dosage form comprising at least one polyethylene oxide having, based on rheological measurements, approximate molecular weight of at least 1,000,000, wherein said second composition comprises at least about 90 % (by wt.) of polyethylene oxide.

**23.** The solid oral extended release pharmaceutical dosage form according to claims 1 to 22, wherein the extended release matrix formulation when subjected to an indentation test has a cracking force of at least about 110 N.

**24.** The solid oral extended release pharmaceutical dosage form according to claims 1 to 23, wherein the extended release matrix formulation when subjected to an indentation test has a "penetration depth to crack distance" of at least about 1.0 mm.

**25.** The solid oral extended release pharmaceutical dosage form according to claim 23, wherein the extended release matrix formulation has a cracking force of at least about 120 N, at least about 130 N or at least about 140 N and / or a "penetration depth to crack" distance of at least about 1.2 mm, preferably of at least about 1.4 mm, at least about 1.5 mm or at least about 1.6 mm, and / or resists a work of at least about 0.06 J without cracking.

**26.** The solid controlled release dosage form according to any one of preceding claims comprising hydrocodone or a pharmaceutically acceptable salt, hydrate or solvate thereof, or mixtures of any of the foregoing.

**27.** A process of preparing a solid oral extended release pharmaceutical dosage form according to any of claims 1 to 26, comprising at least the steps of:

(a) combining

(1) at least one active agent, and
(2) at least one polyethylene oxide having, based on rheological measurements, an approximate molecular weight of at least 1,000,000, to yield a first composition for an active agent-containing layer first layer;

(b) providing a second composition comprising at least one polyethylene oxide having, based on rheological measurements, an approximate molecular weight of at least 1,000,000 or of less than 1,000,000, to yield a second composition for an active agent-free layer second layer,
(c) shaping the compositions form (a) and (b) to form at least a bilayer extended release matrix formulation; and
(d) curing said extended release matrix formulation comprising at least a curing step at a temperature which is at least the softening temperature of said at least one polyethylene oxide.

**28.** The process of preparing a solid oral extended release pharmaceutical dosage form according to claim 27, wherein said curing step (d) is conducted for a time period of at least about 1 minute.

**29.** The process of claim 27, wherein said curing step (d) is conducted for a time period of at least about 5 minutes, or at least 15 minutes.

**30.** The process of claim 27 to 29, wherein in step (c) the composition is shaped to form an extended release matrix formulation in the form of a tablet, and optionally is shaped by direct compression.

**31.** The process of any one of claims 27 to 30, wherein in step (d) the extended release matrix formulation is subjected to a temperature of at least about 60 °C or at least about 62 °C, preferably at least about 68 °C, at least about 70 °C, at least about 72 °C or at least about 75 °C.

**32.** The process of any one of claims 27 to 31, wherein the extended release matrix formulation is subjected to a temperature of from about 60 °C to about 90 °C, from about 65 °C to about 90 °C or from about 68 °C to about 90 °C.

**33.** The process of any one of claims 27 to 32, wherein the extended release matrix formulation is subjected to a temperature of at least about 62 °C or at least about 68 °C for a time period of from about 1 minute to about 5 hours or from about 5 minutes to about 3 hours, or for a time period of at least about 15 minutes.

**34.** The process of any one of claims 27 to 33, wherein the curing step comprises steps of coating the extended release matrix formulation.

**35.** A process of preparing a solid oral extended release pharmaceutical dosage form in accordance with any of the preceding claims, wherein said pharmaceutical dosage form is a bilayer tablet, comprising at least the steps of:

(a) combining

(1) at least one active agent, and
(2) at least one polyethylene oxide having, based on rheological measurements, an approximate molecular weight of at least 1,000,000, to form a first composition for a first active agent-containing layer;

(b) providing a second composition for a second active agent-free layer layer, comprising at least one polyethylene oxide having, based on rheological measurements, an approximate molecular weight of at least 1,000,000, or less than 1,000,000,
(c) shaping the compositions from (a) and (b) by direct compression to form a bilayer tablet; and
(d)

- transferring said tablets to a coating pan;
- coating said tablets to a first weight gain;

- curing said tablets at a temperature of from about 62 °C to about 90 °C for a time period of at least about 1 minute;
- cooling to a temperature of below about 50 °C; and
- coating said cured tablets to a second final weight gain.

**36.** The process of any one of claims 27 to 35, wherein the active agent is an opioid analgesic.

**37.** The process of claim 36, wherein the opioid analgesic is selected from the group of alfentanil, allylprodine, alphaprodine, anileridine, benzylmorphine, bezitramide, buprenorphine, butorphanol, clonitazene, codeine, desomorphine, dextromoramide, dezocine, diampromide, diamorphone, dihydrocodeine, dihydromorphine, dimenoxadol, dimepheptanol, dimethylthiambutene, dioxaphetyl butyrate, dipipanone, eptazocine, ethoheptazine, ethylmethylthiambutene, ethylmorphine, etonitazene, etorphine, dihydroetorphine, fentanyl and derivatives, hydrocodone, hydromorphone, hydroxypethidine, isomethadone, ketobemidone, levorphanol, levophenacylmorphan, lofentanil, meperidine, meptazinol, metazocine, methadone, metopon, morphine, myrophine, narceine, nicomorphine, norlevorphanol, normethadone, nalorphine, nalbuphene, normorphine, norpipanone, opium, oxycodone, oxymorphone, papaveretum, pentazocine, phenadoxone, phenomorphan, phenazocine, phenoperidine, piminodine, piritramide, propheptazine, promedol, propendine, propoxyphene, sufentanil, tilidine, tramadol, pharmaceutically acceptable salts, hydrates and solvates thereof, mixtures of any of the foregoing.

**38.** The process of claim 37, wherein the opioid analgesic is selected from the group of hydrocodone and hydromorphone or pharmaceutically acceptable salts, hydrates and solvates thereof, mixtures of any of the foregoing.

**39.** The process of claim 37, wherein the opioid analgesic is hydrocodone bitartrate or hydromorphone hydrochloride.

**40.** The process of claim 39, wherein the dosage form comprises from about 0.5 mg to about 1250 mg of hydrocodone bitartrate or from about 1 mg to 100 mg hydromorphone hydrochloride, or at least about 5 mg, 7.5 mg, 10 mg, 15 mg, 20 mg, 30 mg, 40 mg, 45 mg, 60 mg, or 80 mg, 90 mg, 120 mg or 160 mg of hydrocodone bitartrate or at least about 2 mg, 4 mg, 8 mg, 12 mg, 16 mg, 24 mg, 32 mg, 48 mg or 64 mg of hydromorphone hydrochloride.

**41.** The process of any one of claims 27 to 40, wherein the at least one polyethylene oxide has, based on rheological measurements, an approximate molecular weight of from 2,000,000 to 8,000,000, or an approximate molecular weight of 2,000,000, 4,000,000, 7,000,000 or 8,000,000.

**42.** A solid oral extended release pharmaceutical dosage form obtainable by a process according to any one of claims 27 to 41.

**43.** A dosage form according to any one of claims 1 to 26 and 42 for the use in the treatment of pain, wherein the dosage form comprises an opioid analgesic.

**Patentansprüche**

**1.** Feste orale verzögert freisetzende pharmazeutische Darreichungsform, umfassend eine mehrschichtige verzögert freisetzende Matrixformulierung mit einer Struktur vom Sandwich-Typ oder Halb-Sandwich-Typ, die verzögert freisetzende Matrixformulierung umfassend:

(1) eine erste Zusammensetzung, bildend eine Wirkstoff-enthaltende erste Schicht der verzögert freisetzenden Matrixformulierung, umfassend:

(a) mindestens ein Polyethylenoxid aufweisend, basierend auf rheologischen Messungen, ein ungefähres Molekulargewicht von mindestens 1.000.000; und
(b) mindestens einen Wirkstoff; und

(2) eine zweite Zusammensetzung bildend eine Wirkstoff-freie zweite Schicht der verzögert freisetzenden Matrixformulierung umfassend mindestens ein Polyethylenoxid.

**2.** Die feste orale verzögert freisetzende pharmazeutische Darreichungsform gemäß Anspruch 1, wobei die zweite Zusammensetzung mindestens ein Polyethylenoxid aufweisend, basierend auf rheologischen Messungen, ein ungefähres Molekulargewicht von mindestens 1.000.000 umfasst.

3. Die feste orale verzögert freisetzende pharmazeutische Darreichungsform gemäß Anspruch 1, wobei die zweite Zusammensetzung mindestens ein Polyethylenoxid aufweisend, basierend auf rheologischen Messungen, ein ungefähres Molekulargewicht von weniger als 1.000.000 umfasst.

4. Die feste orale verzögert freisetzende pharmazeutische Darreichungsform gemäß einem der Ansprüche 1 bis 3, wobei der Wirkstoff ausgewählt ist aus Opioidanalgetika.

5. Die feste orale verzögert freisetzende pharmazeutische Darreichungsform gemäß einem der Ansprüche 1 bis 4, wobei die mehrschichtige verzögert freisetzende Matrixformulierung eine zweischichtige verzögert freisetzende Matrixformulierung ist.

6. Die feste orale verzögert freisetzende pharmazeutische Darreichungsform gemäß Anspruch 5, wobei das Gewichtsverhältnis von Wirkstoff-enthaltender Schicht / Wirkstoff-freier Schicht von ungefähr 1 bis ungefähr 5, oder von ungefähr 1,5 bis ungefähr 3 reicht, oder ungefähr 2 ist oder ungefähr 2,5 ist.

7. Die feste orale verzögert freisetzende pharmazeutische Darreichungsform gemäß einem der Ansprüche 1 bis 6, wobei die verzögert freisetzende Matrixformulierung thermogeformt ist oder einem Härtungsschritt ausgesetzt wird.

8. Die feste orale verzögert freisetzende pharmazeutische Darreichungsform gemäß Anspruch 7, wobei die verzögert freisetzende Matrixformulierung einem Härtungsschritt ausgesetzt wird bei einer Temperatur von mindestens ungefähr 60 °C für eine Zeitspanne von mindestens ungefähr 1 Minute.

9. Die feste orale verzögert freisetzende pharmazeutische Darreichungsform gemäß einem der Ansprüche 1 bis 8, wobei die Wirkstoff-enthaltende Schicht und die Wirkstoff-freie Schicht weniger als 25% Laktose, oder im Wesentlichen keine Laktose enthalten.

10. Die feste orale verzögert freisetzende pharmazeutische Darreichungsform gemäß einem der Ansprüche 1 bis 9, wobei die Wirkstoff-enthaltende Schicht und die Wirkstoff-freie Schicht im Wesentlichen kein hydriertes Rizinusöl und/oder im Wesentlichen keine Hydroxypropylmethylcellulose enthalten.

11. Die feste orale verzögert freisetzende pharmazeutische Darreichungsform gemäß einem der Ansprüche 1 bis 10, wobei die Dosierung eine Auflösungsgeschwindigkeit bereitstellt, die, wenn sie in einem USP-Apparat 1 (Körbchen) bei 100 Upm in 900 ml simulierter Magenflüssigkeit ohne Enzyme (SGF) bei 37 °C gemessen wird, den Wirkstoff im Wesentlichen gemäß einem Modus nullter Ordnung freisetzt.

12. Die feste orale verzögert freisetzende pharmazeutische Darreichungsform gemäß einem der Ansprüche 1 bis 11, wobei die Dosierung eine Auflösungsgeschwindigkeit bereitstellt, die, wenn sie in einem USP-Apparat 1 (Körbchen) bei 100 Upm in 900 ml simulierter Magenflüssigkeit ohne Enzyme (SGF) bei 37 °C gemessen wird, von ungefähr 5 bis ungefähr 15 Gew.-% freigesetzten Wirkstoff pro Stunde beträgt und/oder von ungefähr 5 bis ungefähr 15 Gew.-% freigesetzten Wirkstoff nach 1 Stunde beträgt, und/oder von ungefähr 10 bis ungefähr 30 Gew.-% freigesetzten Wirkstoff nach 2 Stunden beträgt, und/oder von ungefähr 20 bis ungefähr 60 Gew.-% freigesetzten Wirkstoff nach 4 Stunden beträgt, und/oder von ungefähr 40 bis ungefähr 100 Gew.-% freigesetzten Wirkstoff nach 8 Stunden beträgt.

13. Die feste orale verzögert freisetzende pharmazeutische Darreichungsform gemäß einem der Ansprüche 1 bis 12, wobei die erste Zusammensetzung mindestens ungefähr 60 Gew.-%, mindestens ungefähr 70 Gew.-%, mindestens ungefähr 80 Gew.-%, mindestens ungefähr 90 Gew.-% des Polyethylenoxids umfasst.

14. Die feste orale verzögert freisetzende pharmazeutische Darreichungsform gemäß einem der Ansprüche 1 bis 13, wobei das Opioidanalgetikum ausgewählt ist aus der Gruppe von Alfentanil, Allylprodin, Alphaprodin, Anileridin, Benzylmorphin, Bezitramid, Buprenorphin, Butorphanol, Clonitazen, Codein, Desomorphin, Dextromoramid, Dezocin, Diampromid, Diamorphon, Dihydrocodein, Dihydromorphin, Dimenoxadol, Dimepheptanol, Dimethylthiambuten, Dioxaphetylbutyrat, Dipipanon, Eptazocin, Ethoheptazin, Ethylmethylthiambuten, Ethylmorphin, Etonitazen, Etorphin, Dihydroetorphin, Fentanyl und Derivate, Hydrocodon, Hydromorphon, Hydroxypethidin, Isomethadon, Ketobemidon, Levorphanol, Levophenacylmorphan, Lofentanil, Meperidin, Meptazinol, Metazocin, Methadon, Metopon, Morphin, Myrophin, Narcein, Nicomorphin, Norlevorphanol, Normethadon, Nalorphin, Nalbuphen, Normorphin, Norpipanon, Opium, Oxycodon, Oxymorphon, Papaveretum, Pentazocin, Phenadoxon, Phenomorphan, Phenazocin, Phenoperidin, Piminodin, Piritramid, Propheptazin, Promedol, Properidin, Propoxyphen, Sufentanil, Tilidin, Tramadol, pharmazeutisch akzeptable Salze, Hydrate und Solvate davon, sowie sämtliche Mischungen.

**15.** Die feste orale verzögert freisetzende pharmazeutische Darreichungsform gemäß einem der Ansprüche 1 bis 14, wobei das Opioidanalgetikum ausgewählt ist aus der Gruppe von Hydrocodon und Hydromorphon, oder pharmazeutisch akzeptable Salze, Hydrate und Solvate davon, sowie sämtliche Mischungen.

**16.** Die feste orale verzögert freisetzende pharmazeutische Darreichungsform gemäß einem der Ansprüche 1 bis 15, wobei das Opioidanalgetikum Hydrocodonbitartrat oder Hydromorphonhydrochlorid ist.

**17.** Die feste orale verzögert freisetzende pharmazeutische Darreichungsform gemäß einem der Ansprüche 1 bis 16, wobei die Darreichungsform von ungefähr 5 mg bis ungefähr 250 mg Hydrocodonbitartrat oder von 1 mg bis 100 mg Hydromorphonhydrochlorid umfasst, und/oder wobei die Darreichungsform 5 mg, 7,5 mg, 10 mg, 15 mg, 20 mg, 30 mg, 40 mg, 45 mg, 60 mg, oder 80 mg, 90 mg, 100 mg, 120 mg oder 160 mg Hydrocodonbitartrat oder 2 mg, 5 mg, 7,5 mg, 10 mg, 15 mg, 20 mg, 25 mg, 30 mg, 32 mg, oder 64 mg Hydromorphonhydrochlorid umfasst.

**18.** Die feste orale verzögert freisetzende pharmazeutische Darreichungsform gemäß Anspruch 16 oder 17, wobei die erste Zusammensetzung mindestens ungefähr 65 Gew.-% Polyethylenoxid aufweisend, basierend auf rheologischen Messungen, ein ungefähres Molekulargewicht von mindestens 1.000.000 umfasst.

**19.** Feste orale verzögert freisetzende pharmazeutische Darreichungsform gemäß Anspruch 1, umfassend eine verzögert freisetzende Matrixformulierung, die verzögert freisetzende Matrixformulierung umfassend:

(1) eine erste Zusammensetzung bildend eine Wirkstoff-enthaltende erste Schicht der verzögert freisetzenden Matrixformulierung umfassend mindestens ungefähr 65 Gew.-% eines Polyethylenoxids aufweisend, basierend auf rheologischen Messungen, ein ungefähres Molekulargewicht von mindestens 1.000.000; und
(2) ungefähr 5 mg, 10 mg, 15 mg, 20 mg, oder 40 mg, 60 mg, 80 mg, 100 mg, oder 120 mg Hydrocodonbitartrat.

**20.** Feste orale verzögert freisetzende pharmazeutische Darreichungsform gemäß Anspruch 1, umfassend eine verzögert freisetzende Matrixformulierung, die verzögert freisetzende Matrixformulierung umfassend:

(1) eine erste Zusammensetzung bildend eine Wirkstoff-enthaltende erste Schicht der verzögert freisetzenden Matrixformulierung umfassend mindestens ungefähr 90 Gew.-% eines Polyethylenoxids aufweisend, basierend auf rheologischen Messungen, ein ungefähres Molekulargewicht von mindestens 1.000.000; und
(2) ungefähr 5 mg Hydrocodonbitartrat.

**21.** Feste orale verzögert freisetzende pharmazeutische Darreichungsform gemäß Anspruch 1, umfassend eine verzögert freisetzende Matrixformulierung, die verzögert freisetzende Matrixformulierung umfassend:

(1) eine erste Zusammensetzung bildend eine Wirkstoff-enthaltende erste Schicht der verzögert freisetzenden Matrixformulierung umfassend mindestens ungefähr 90 Gew.-% eines Polyethylenoxids aufweisend, basierend auf rheologischen Messungen, ein ungefähres Molekulargewicht von mindestens 1.000.000; und
(2) ungefähr 5 mg, 6 mg, 7 mg, 8 mg, 10, mg, 12 mg, 15 mg, 20 mg, 25 mg, 30 mg oder 32 mg Hydromorphonhydrochlorid.

**22.** Feste orale verzögert freisetzende pharmazeutische Darreichungsform gemäß Ansprüchen 1 bis 21, umfassend eine zweite Zusammensetzung bildend eine Wirkstoff-freie Schicht zweite Schicht der verzögert freisetzenden Matrixformulierung umfassend mindestens ein Polyethylenoxid aufweisend, basierend auf rheologischen Messungen, ein ungefähres Molekulargewicht von mindestens 1.000.000 umfasst, wobei die zweite Zusammensetzung mindestens ungefähr 90 Gew.-% Polyethylenoxid umfasst.

**23.** Die feste orale verzögert freisetzende pharmazeutische Darreichungsform gemäß Ansprüchen 1 bis 22, wobei die verzögert freisetzenden Matrixformulierung, wenn sie einem Eindrücktest (indentation test) unterzogen wird, eine Rissbelastung (cracking force) von mindestens ungefähr 110 N aufweist.

**24.** Die feste orale verzögert freisetzende pharmazeutische Darreichungsform gemäß Ansprüchen 1 bis 23, wobei die verzögert freisetzenden Matrixformulierung, wenn sie einem Eindrücktest unterzogen wird, eine "Eindringtiefe bis zum Riss-Abstand" ("penetration depth to crack distance") von mindestens ungefähr 1,0 mm aufweist.

**25.** Die feste orale verzögert freisetzende pharmazeutische Darreichungsform gemäß Anspruch 23, wobei die verzögert freisetzenden Matrixformulierung eine Rissbelastung von mindestens ungefähr 120 N, mindestens ungefähr 130 N oder mindestens ungefähr 140 N und/oder eine "Eindringtiefe bis zum Riss-Abstand" von mindestens ungefähr 1,2 mm, bevorzugt mindestens ungefähr 1,4 mm, mindestens ungefähr 1,5 mm oder mindestens ungefähr 1,6 mm aufweist und/oder einer Arbeit von mindestens ungefähr 0,06 J ohne Reißen widersteht.

**26.** Die feste kontrolliert freisetzende Darreichungsform gemäß einem der vorangehenden Ansprüche, umfassend Hydrocodon oder pharmazeutisch akzeptable Salze, Hydrate oder Solvate davon, oder sämtliche Mischungen.

**27.** Verfahren zur Herstellung einer festen oralen verzögert freisetzenden pharmazeutischen Darreichungsform gemäß einem der Ansprüche 1 bis 26, umfassend wenigstens die Schritte aus:

(a) Kombinieren

(1) mindestens eines Wirkstoffs, und
(2) mindestens eines Polyethylenoxids aufweisend, basierend auf rheologischen Messungen, ein ungefähres Molekulargewicht von mindestens 1.000.000,
um eine erste Zusammensetzung für eine Wirkstoff-enthaltende erste Schicht zu erhalten;

(b) Bereitstellen einer zweite Zusammensetzung umfassend mindestens ein Polyethylenoxid aufweisend, basierend auf rheologischen Messungen, ein ungefähres Molekulargewicht von mindestens 1.000.000 oder von weniger als 1.000.000, um eine zweite Zusammensetzung für eine Wirkstoff-freie zweite Schicht zu erhalten,
(c) Formen der Zusammensetzungen aus (a) und (b), um mindestens eine zweischichtige verzögert freisetzende Matrixformulierung zu bilden; und
(d) Härten der verzögert freisetzenden Matrixformulierung, umfassend mindestens einen Härtungsschritt bei einer Temperatur, die mindestens der Erweichungstemperatur des mindestens einen Polyethylenoxids entspricht.

**28.** Das Verfahren zur Herstellung einer festen oralen verzögert freisetzenden pharmazeutischen Darreichungsform gemäß Anspruch 27, wobei der Härtungsschritt (d) für eine Zeitspanne von mindestens ungefähr 1 Minute durchgeführt wird.

**29.** Das Verfahren gemäß Anspruch 27, wobei der Härtungsschritt (d) für eine Zeitspanne von mindestens ungefähr 5 Minuten, oder von mindestens ungefähr 15 Minuten durchgeführt wird.

**30.** Das Verfahren gemäß Ansprüchen 27 bis 29, wobei in Schritt (c) die Zusammensetzung geformt wird, um eine verzögert freisetzende Matrixformulierung in Form einer Tablette zu bilden, und optional durch direkte Komprimierung geformt wird.

**31.** Das Verfahren gemäß einem der Ansprüche 27 bis 30, wobei in Schritt (d) die verzögert freisetzende Matrixformulierung einer Temperatur von mindestens ungefähr 60 °C oder mindestens ungefähr 62 °C, bevorzugt mindestens ungefähr 68 °C, mindestens ungefähr 70 °C, mindestens ungefähr 72 °C oder mindestens ungefähr 75 °C ausgesetzt wird.

**32.** Das Verfahren gemäß einem der Ansprüche 27 bis 31, wobei die verzögert freisetzende Matrixformulierung einer Temperatur von ungefähr 60 °C bis ungefähr 90 °C, von ungefähr 65 °C bis ungefähr 90 °C oder von ungefähr 68 °C bis ungefähr 90 °C ausgesetzt wird.

**33.** Das Verfahren gemäß einem der Ansprüche 27 bis 32, wobei die verzögert freisetzende Matrixformulierung einer Temperatur von mindestens ungefähr 62 °C oder mindestens ungefähr 68 °C für eine Zeitspanne von ungefähr 1 Minute bis ungefähr 5 Stunden oder von ungefähr 5 Minuten bis ungefähr 3 Stunden, oder für eine Zeitspanne von mindestens ungefähr 15 Minuten ausgesetzt wird.

**34.** Das Verfahren gemäß einem der Ansprüche 27 bis 33, wobei der Härtungsschritt Beschichtungsschritte der verzögert freisetzenden Matrixformulierung umfasst.

**35.** Verfahren zur Herstellung einer festen oralen verzögert freisetzenden pharmazeutischen Darreichungsform gemäß einem der vorangehenden Ansprüche, wobei die pharmazeutische Darreichungsform eine zweischichtige Tablette ist, umfassend wenigstens die Schritte aus:

(a) Kombinieren

(1) mindestens eines Wirkstoffs, und
(2) mindestens eines Polyethylenoxids aufweisend, basierend auf rheologischen Messungen, ein ungefähres Molekulargewicht von mindestens 1.000.000,
um eine erste Zusammensetzung für eine erste Wirkstoff-enthaltende Schicht zu bilden;

(b) Bereitstellen einer zweiten Zusammensetzung für eine zweite Wirkstoff-freie Schicht, umfassend mindestens ein Polyethylenoxid aufweisend, basierend auf rheologischen Messungen, ein ungefähres Molekulargewicht von mindestens 1.000.000 oder von weniger als 1.000.000 ,
(c) Formen der Zusammensetzungen aus (a) und (b) durch direkte Komprimierung, um eine zweischichtige Tablette zu bilden; und
(d)

- Überführen der Tabletten in eine Beschichtungspfanne;
- Beschichten der Tabletten bis zu einer ersten Gewichtszunahme;
- Härten der Tabletten bei einer Temperatur von ungefähr 62 °C bis ungefähr 90 °C für eine Zeitspanne von mindestens ungefähr 1 Minute;
- Abkühlen auf eine Temperatur von unter ungefähr 50 °C; und
- Beschichten der gehärteten Tabletten bis zu einer zweiten finalen Gewichtszunahme.

**36.** Das Verfahren gemäß einem der Ansprüche 27 bis 35, wobei der Wirkstoff ein Opioidanalgetikum ist.

**37.** Das Verfahren gemäß Anspruch 36, wobei das Opioidanalgetikum ausgewählt ist aus der Gruppe von Alfentanil, Allylprodin, Alphaprodin, Anileridin, Benzylmorphin, Bezitramid, Buprenorphin, Butorphanol, Clonitazen, Codein, Desomorphin, Dextromoramid, Dezocin, Diampromid, Diamorphon, Dihydrocodein, Dihydromorphin, Dimenoxadol, Dimepheptanol, Dimethylthiambuten, Dioxaphetylbutyrat, Dipipanon, Eptazocin, Ethoheptazin, Ethylmethylthiambuten, Ethylmorphin, Etonitazen, Etorphin, Dihydroetorphin, Fentanyl und Derivate, Hydrocodon, Hydromorphon, Hydroxypethidin, Isomethadon, Ketobemidon, Levorphanol, Levophenacylmorphan, Lofentanil, Meperidin, Meptazinol, Metazocin, Methadon, Metopon, Morphin, Myrophin, Narcein, Nicomorphin, Norlevorphanol, Normethadon, Naloiphin, Nalbuphen, Normorphin, Norpipanon, Opium, Oxycodon, Oxymorphon, Papaveretum, Pentazocin, Phenadoxon, Phenomorphan, Phenazocin, Phenoperidin, Piminodin, Piritramid, Propheptazin, Promedol, Properidin, Propoxyphen, Sufentanil, Tilidin, Tramadol, pharmazeutisch akzeptable Salze, Hydrate und Solvate davon, sowie sämtliche Mischungen.

**38.** Das Verfahren gemäß Anspruch 37, wobei das Opioidanalgetikum ausgewählt ist aus der Gruppe von Hydrocodon und Hydromorphon, oder pharmazeutisch akzeptable Salze, Hydrate und Solvate davon, sowie sämtliche Mischungen.

**39.** Das Verfahren gemäß Anspruch 37, wobei das Opioidanalgetikum Hydrocodonbitartrat oder Hydromorphonhydrochlorid ist.

**40.** Das Verfahren gemäß Anspruch 39, wobei die Darreichungsform von ungefähr 0,5 mg bis ungefähr 1250 mg Hydrocodonbitartrat oder von ungefähr 1 mg bis 100 mg Hydromorphonhydrochlorid, oder mindestens ungefähr 5 mg, 7,5 mg, 10 mg, 15 mg, 20 mg, 30 mg, 40 mg, 45 mg, 60 mg, oder 80 mg, 90 mg, 120 mg oder 160 mg Hydrocodonbitartrat oder mindestens ungefähr 2 mg, 4 mg, 8 mg, 12 mg, 16 mg, 24 mg, 32 mg, 48 mg oder 64 mg Hydromorphonhydrochlorid umfasst.

**41.** Das Verfahren gemäß einem der Ansprüche 27 bis 40, wobei das mindestens eine Polyethylenoxid, basierend auf rheologischen Messungen, ein ungefähres Molekulargewicht von 2.000.000 bis 8.000.000, oder ein ungefähres Molekulargewicht von 2.000.000, 4.000.000, 7.000.000 oder 8.000.000 aufweist.

**42.** Feste orale verzögert freisetzende pharmazeutische Darreichungsform, erhältlich durch ein Verfahren gemäß einem der Ansprüche 27 bis 41.

43. Feste orale verzögert freisetzende pharmazeutische Darreichungsform gemäß einem der Ansprüche 1 bis 26 und 42 zur Verwendung für die Behandlung von Schmerz, wobei die Darreichungsform ein Opioidanalgetikum umfasst.

## Revendications

1. Forme posologique pharmaceutique orale solide à libération prolongée, comprenant une formulation de matrice multicouche à libération prolongée à structure du type en sandwich ou du type en demi-sandwich, la formulation de matrice à libération prolongée comprenant:

   (1) une première composition formant une première couche contenant un agent actif de la formulation de matrice à libération prolongée, comprenant:

      (a) au moins un poly(oxyde d'éthylène) ayant, d'après des mesures rhéologiques, un poids moléculaire approximatif d'au moins 1 000 000; et
      (b) au moins un agent actif; et

   (2) une seconde composition formant une seconde couche dépourvue d'agent actif de la formulation de matrice à libération prolongée, comprenant au moins un poly(oxyde d'éthylène).

2. Forme posologique pharmaceutique orale solide à libération prolongée suivant la revendication 1, dans laquelle la seconde composition comprend au moins un poly(oxyde d'éthylène) ayant, d'après des mesures rhéologiques, un poids moléculaire approximatif d'au moins 1 000 000.

3. Forme posologique pharmaceutique orale solide à libération prolongée suivant la revendication 1, dans laquelle la seconde composition comprend au moins un poly(oxyde d'éthylène) ayant, d'après des mesures rhéologiques, un poids moléculaire approximatif inférieur à 1 000 000.

4. Forme posologique pharmaceutique orale solide à libération prolongée suivant l'une quelconque des revendications 1 à 3, dans laquelle l'agent actif est choisi parmi des analgésiques opioïdes.

5. Forme posologique pharmaceutique orale solide à libération prolongée suivant l'une quelconque des revendications 1 à 4, dans laquelle la formulation de matrice multicouche à libération prolongée est une formulation de matrice bicouche à libération prolongée.

6. Forme posologique pharmaceutique orale solide à libération prolongée suivant la revendication 5, dans laquelle le rapport pondéral couche contenant l'agent actif/couche dépourvue d'agent actif va d'environ 1 à environ 5, ou d'environ 1,5 à environ 3, ou est égal à environ 2 ou est égal à environ 2,5.

7. Forme posologique pharmaceutique orale solide à libération prolongée suivant l'une quelconque des revendications 1 à 6, dans laquelle la formulation de matrice à libération prolongée est thermoformée ou soumise à une étape de durcissement.

8. Forme posologique pharmaceutique orale solide à libération prolongée suivant la revendication 7, dans laquelle la formulation de matrice à libération prolongée est soumise à une étape de durcissement à une température d'au moins environ 60 °C pendant une période de temps d'au moins environ 1 minute.

9. Forme posologique pharmaceutique orale solide à libération prolongée suivant l'une quelconque des revendications 1 à 8, dans laquelle la couche contenant l'agent actif et la couche dépourvue d'agent actif comprennent moins de 25% de lactose, ou ne comprennent pratiquement pas de lactose.

10. Forme posologique pharmaceutique orale solide à libération prolongée suivant l'une quelconque des revendications 1 à 9, dans laquelle la couche contenant l'agent actif et la couche dépourvue d'agent actif ne comprennent pratiquement pas d'huile de ricin hydrogénée et/ou pratiquement pas d'hydroxypropylméthylcellulose.

11. Forme posologique pharmaceutique orale solide à libération prolongée suivant l'une quelconque des revendications 1 à 10, dans laquelle la posologie fournit une vitesse de dissolution qui, lorsqu'elle est mesurée dans un appareil USP 1 (à panier) à 100 tr/min dans 900 ml de fluide gastrique simulé sans enzymes (SGF) à 37 °C, libère l'agent actif essentiellement suivant un mode d'ordre zéro.

**12.** Forme posologique pharmaceutique orale solide à libération prolongée suivant l'une quelconque des revendications 1 à 11, dans laquelle la posologie fournit une vitesse de dissolution qui, lorsqu'elle est mesurée dans un appareil USP 1 (à panier) à 100 tr/min dans 900 ml de fluide gastrique synthétique sans enzymes (SGF) à 37 °C, est d'environ 5% à environ 15% (en poids) d'agent actif libéré par heure et/ou est d'environ 5% à environ 15% (en poids) d'agent actif libéré au bout d'1 heure, et/ou est d'environ 10% à environ 30% (en poids) d'agent actif libéré au bout de 2 heures, et/ou est d'environ 20% à environ 60% (en poids) d'agent actif libéré au bout de 4 heures, et/ou est d'environ 40% à environ 100% (en poids) d'agent actif libéré au bout de 8 heures.

**13.** Forme posologique pharmaceutique orale solide à libération prolongée suivant l'une quelconque des revendications 1 à 12, dans laquelle la première composition comprend au moins environ 60% (en poids), au moins environ 70% (en poids), au moins environ 80% (en poids), au moins environ 90% (en poids) dudit poly(oxyde d'éthylène).

**14.** Forme posologique pharmaceutique orale solide à libération prolongée suivant l'une quelconque des revendications 1 à 13, dans laquelle l'analgésique opioïde est choisi dans le groupe comprenant l'alfentanil, l'allylprodine, l'alphaprodine, l'aniléridine, la benzylmorphine, le bézitramide, la buprénorphine, le butorphanol, le clonitazène, la codéine, la désomorphine, le dextromoramide, la dézocine, le diampromide, la diamorphone, la dihydrocodéine, la dihydromorphine, le diménoxadol, le dimépheptanol, le diméthylthiambutène, le butyrate de dioxaphétyle, la dipipanone, l'eptazocine, l'éthoheptazine, l'éthylméthylthiambutène, l'éthylmorphine, l'étonitazène, l'étorphine, la dihydroétorphine, le fentanyl et ses dérivés, l'hydrocodone, l'hydromorphone, l'hydroxy-péthidine, l'isométhadone, la kétobémidone, le lévorphanol, le lévophénacylmorphane, le lofentanil, la mépéridine, le meptazinol, la métazocine, la méthadone, le métopon, la morphine, la myrophine, la narcéine, la nicomorphine, le norlévorphanol, la norméthadone, la nalorphine, le nalbuphène, la normorphine, la norpipanone, l'opium, l'oxycodone, l'oxymorphone, le papaveretum, la pentazocine, la phénadoxone, le phénomorphane, la phénazocine, la phénopéridine, la piminodine, le piritramide, la propheptazine, le promédol, la propéridine, le propoxyphène, le sufentanil, la tilidine, le tramadol, leurs sels, hydrates et produits de solvatation pharmaceutiquement acceptables et des mélanges de n'importe lesquels des agents précités.

**15.** Forme posologique pharmaceutique orale solide à libération prolongée suivant l'une quelconque des revendications 1 à 14, dans laquelle l'analgésique opioïde est choisi dans le groupe comprenant l'hydrocodone et l'hydromorphone ou leurs sels, hydrates et produits de solvatation pharmaceutiquement acceptables et des mélanges de n'importe lesquels des agents précités.

**16.** Forme posologique pharmaceutique orale solide à libération prolongée suivant l'une quelconque des revendications 1 à 15, dans laquelle l'analgésique opioïde est le bitartrate d'hydrocodone ou le chlorhydrate d'hydromorphone.

**17.** Forme posologique pharmaceutique orale solide à libération prolongée suivant l'une quelconque des revendications 1 à 16, ladite forme posologique comprenant environ 5 mg à environ 250 mg de bitartrate d'hydrocodone ou 1 mg à 100 mg de chlorhydrate d'hydromorphone, et/ou la forme posologique comprenant 5 mg, 7,5 mg, 10 mg, 15 mg, 20 mg, 30 mg, 40 mg, 45 mg, 60 mg, ou 80 mg, 90 mg, 100 mg, 120 mg ou 160 mg de bitartrate d'hydrocodone ou 2 mg, 5 mg, 7,5 mg, 10 mg, 15 mg, 20 mg, 25 mg, 30 mg, 32 mg ou 64 mg de chlorhydrate d'hydromorphone.

**18.** Forme posologique pharmaceutique orale solide à libération prolongée suivant la revendication 16 ou 17, dans laquelle la première composition comprend au moins environ 65% (en poids) de poly(oxyde d'éthylène) ayant, d'après des mesures rhéologiques, un poids moléculaire approximatif d'au moins 1 000 000.

**19.** Forme posologique pharmaceutique orale solide à libération prolongée suivant la revendication 1, comprenant une formulation de matrice à libération prolongée, la formulation de matrice à libération prolongée comprenant:

(1) une première composition formant une première couche contenant un agent actif de ladite formulation de matrice à libération prolongée comprenant au moins 65% (en poids) d'un poly(oxyde d'éthylène) ayant, d'après des mesures rhéologiques, un poids moléculaire approximatif d'au moins 1 000 000, et
(2) environ 5 mg, 10 mg, 15 mg, 20 mg ou 40 mg, 60 mg, 80 mg, 100 mg ou 120 mg de bitartrate d'hydrocodone.

**20.** Forme posologique pharmaceutique orale solide à libération prolongée suivant la revendication 1, comprenant une formulation de matrice à libération prolongée, la formulation de matrice à libération prolongée comprenant:

(1) une première composition formant une première couche contenant un agent actif de ladite formulation de matrice à libération prolongée comprenant au moins 90% (en poids) d'un poly(oxyde d'éthylène) ayant, d'après des mesures rhéologiques, un poids moléculaire approximatif d'au moins 1 000 000, et
(2) environ 5 mg de bitartrate d'hydrocodone.

**21.** Forme posologique pharmaceutique orale solide à libération prolongée suivant la revendication 1, comprenant une formulation de matrice à libération prolongée, la formulation de matrice à libération prolongée comprenant:

(1) une première composition formant une première couche contenant un agent actif de ladite formulation de matrice à libération prolongée comprenant au moins 90% (en poids) d'un poly(oxyde d'éthylène) ayant, d'après des mesures rhéologiques, un poids moléculaire approximatif d'au moins 1 000 000, et
(2) environ 5 mg, 6 mg, 7 mg, 8 mg, 10, mg, 12 mg, 15 mg, 20 mg, 25 mg, 30 mg ou 32 mg de chlorhydrate d'hydromorphone.

**22.** Forme posologique pharmaceutique orale solide à libération prolongée suivant les revendications 1 à 21, comprenant une seconde composition formant une seconde couche dépourvue d'agent actif de la forme posologique pharmaceutique comprenant au moins un poly(oxyde d'éthylène) ayant, d'après des mesures rhéologiques, un poids moléculaire approximatif d'au moins 1 000 000, dans laquelle ladite seconde composition comprend au moins environ 90% (en poids) de poly(oxyde d'éthylène).

**23.** Forme posologique pharmaceutique orale solide à libération prolongée suivant les revendications 1 à 22, dans laquelle la formulation de matrice à libération prolongée, lorsqu'elle est soumise à un test d'indentation, a une force de fissuration d'au moins environ 110 N.

**24.** Forme posologique pharmaceutique orale solide à libération prolongée suivant les revendications 1 à 23, dans laquelle la formulation de matrice à libération prolongée, lorsqu'elle est soumise à un test d'indentation, a une "distance de profondeur de pénétration jusqu'à la fissuration" d'au moins environ 1,0 mm.

**25.** Forme posologique pharmaceutique orale solide à libération prolongée suivant la revendication 23, dans laquelle la formulation de matrice à libération prolongée a une force de fissuration d'au moins environ 120 N, d'au moins environ 130 N ou d'au moins environ 140 N et/ou une "distance de profondeur de pénétration jusqu'à la fissuration" d'au moins environ 1,2 mm, de préférence d'au moins environ 1,4 mm, d'au moins environ 1,5 mm ou d'au moins environ 1,6 mm et/ou résiste à un travail d'au moins environ 0,06 J sans fissuration.

**26.** Forme posologique solide à libération contrôlée suivant l'une quelconque des revendications précédentes, comprenant de l'hydrocodone ou un de ses sels, hydrates ou produits de solvatation pharmaceutiquement acceptables, ou des mélanges de n'importe lesquels des agents précédents.

**27.** Procédé de préparation d'une forme posologique pharmaceutique orale solide à libération prolongée suivant l'une quelconque des revendications 1 à 26, comprenant au moins les étapes de:

(a) combinaison

(1) d'au moins un agent actif, et
(2) d'au moins un poly(oxyde d'éthylène) ayant, d'après des mesures rhéologiques, un poids moléculaire approximatif d'au moins 1 000 000, pour obtenir une première composition pour une première couche contenant un agent actif;

(b) fourniture d'une seconde composition comprenant au moins un poly(oxyde d'éthylène) ayant, d'après des mesures rhéologiques, un poids moléculaire approximatif d'au moins 1 000 000 ou inférieur à 1 000 000, pour obtenir une seconde composition pour une seconde couche dépourvue d'agent actif;
(c) façonnage des compositions de (a) et (b) pour former au moins une formulation de matrice bicouche à libération prolongée; et
(d) durcissement de ladite formulation de matrice à libération prolongée comprenant au moins une étape de durcissement à une température qui est égale à au moins la température de ramollissement dudit au moins un poly(oxyde d'éthylène).

**28.** Procédé de préparation d'une forme posologique pharmaceutique orale solide à libération prolongée suivant la revendication 27, dans lequel ladite étape de durcissement (d) est mise en oeuvre pendant une période de temps d'au moins environ 1 minute.

**29.** Procédé suivant la revendication 27, dans lequel ladite étape de durcissement (d) est mise en oeuvre pendant une période de temps d'au moins environ 5 minutes, ou d'au moins 15 minutes.

**30.** Procédé suivant les revendications 27 à 29, dans lequel, dans l'étape (c), la composition est façonnée pour former une formulation de matrice à libération prolongée sous forme de comprimé, et est éventuellement façonnée par compression directe.

**31.** Procédé suivant l'une quelconque des revendications 27 à 30, dans lequel, dans l'étape (d), la formulation de matrice à libération prolongée est soumise à une température d'au moins environ 60 °C ou d'au moins environ 62 °C, de préférence d'au moins environ 68 °C, d'au moins environ 70 °C, d'au moins environ 72 °C ou d'au moins environ 75 °C.

**32.** Procédé suivant l'une quelconque des revendications 27 à 31, dans lequel la formulation de matrice à libération prolongée est soumise à une température d'environ 60 °C à environ 90 °C, d'environ 65 °C à environ 90 °C ou d'environ 68 °C à environ 90 °C.

**33.** Procédé suivant l'une quelconque des revendications 27 à 32, dans lequel la formulation de matrice à libération prolongée est soumise à une température d'au moins environ 62 °C ou d'au moins environ 68 °C pendant une période de temps d'environ 1 minute à environ 5 heures ou d'environ 5 minutes à environ 3 heures, ou pendant une période de temps d'au moins environ 15 minutes.

**34.** Procédé suivant l'une quelconque des revendications 27 à 33, dans lequel l'étape de durcissement comprend des étapes d'enrobage de la formulation de matrice à libération prolongée.

**35.** Procédé de préparation d'une forme posologique pharmaceutique orale solide à libération prolongée suivant l'une quelconque des revendications précédentes, dans lequel ladite forme posologique pharmaceutique est un comprimé bicouche, comprenant au moins les étapes de:

(a) combinaison

(1) d'au moins un agent actif, et
(2) d'au moins un poly(oxyde d'éthylène) ayant, d'après des mesures rhéologiques, un poids moléculaire approximatif d'au moins 1 000 000, pour former une première composition pour une première couche contenant un agent actif;

(b) fourniture d'une seconde composition pour une seconde couche dépourvue d'agent actif, comprenant au moins un poly(oxyde d'éthylène) ayant, d'après des mesures rhéologiques, un poids moléculaire approximatif d'au moins 1 000 000, ou inférieur à 1 000 000;
(c) façonnage des compositions de (a) et (b) par compression directe pour former un comprimé bicouche; et
(d)

- transfert desdits comprimés à une turbine d'enrobage;
- enrobage desdits comprimés jusqu'à un premier gain de poids;
- durcissement desdits comprimés à une température d'environ 62°C à environ 90°C pendant une période de temps d'au moins environ 1 minute;
- refroidissement à une température inférieure à environ 50 °C; et
- enrobage desdits comprimés durcis jusqu'à un second gain de poids final.

**36.** Procédé suivant l'une quelconque des revendications 27 à 35, dans lequel l'agent actif est un analgésique opioïde.

**37.** Procédé suivant la revendication 36, dans lequel l'analgésique opioïde est choisi dans le groupe comprenant l'alfentanil, l'allylprodine, l'alphaprodine, l'aniléridine, la benzylmorphine, le bézitramide, la buprénorphine, le butorphanol, le clonitazène, la codéine, la désomorphine, le dextromoramide, la dézocine, le diampromide, la diamorphone, la dihydrocodéine, la dihydromorphine, le diménoxadol, le dimépheptanol, le diméthylthiambutène, le butyrate de dioxaphétyle, la dipipanone, l'eptazocine, l'éthoheptazine, l'éthylméthylthiambutène, l'éthylmorphine, l'étonitazène, l'étorphine, la dihydroétorphine, le fentanyl et ses dérivés, l'hydrocodone, l'hydromorphone, l'hydroxypéthidine, l'isométhadone, la kétobémidone, le lévorphanol, le lévophénacylmorphane, le lofentanil, la mépéridine, le meptazinol, la métazocine, la méthadone, le métopon, la morphine, la myrophine, la narcéine, la nicomorphine, le norlévorphanol, la norméthadone, la nalorphine, le nalbuphène, la normorphine, la noipipanone, l'opium, l'oxycodone, l'oxymorphone, le papaveretum, la pentazocine, la phénadoxone, le phénomorphane, la phénazocine, la phénopéridine, la piminodine, le piritramide, la propheptazine, le promédol, la propéridine, le propoxyphène, le sufentanil, la tilidine, le tramadol, leurs sels, hydrates et produits de solvatation pharmaceutiquement acceptables et des mélanges de n'importe lesquels des agents précités.

**38.** Procédé suivant la revendication 37, dans lequel l'analgésique opioïde est choisi dans le groupe comprenant l'hydrocodone et l'hydromorphone ou leurs sels, hydrates et produits de solvatation pharmaceutiquement acceptables et des mélanges de n'importe lesquels des agents précédents.

**39.** Procédé suivant la revendication 37, dans lequel l'analgésique opioïde est le bitartrate d'hydrocodone ou le chlorhydrate d'hydromorphone.

**40.** Procédé suivant la revendication 39, dans lequel la forme posologique comprend environ 0,5 mg à environ 1250 mg de bitartrate d'hydrocodone ou environ 1 mg à 100 mg de chlorhydrate d'hydromorphone, ou au moins environ 5 mg, 7,5 mg, 10 mg, 15 mg, 20 mg, 30 mg, 40 mg, 45 mg, 60 mg, ou 80 mg, 90 mg, 120 mg ou 160 mg de bitartrate d'hydrocodone ou au moins environ 2 mg, 4 mg, 8 mg, 12 mg, 16 mg, 24 mg, 32 mg, 48 mg ou 64 mg de chlorhydrate d'hydromorphone.

**41.** Procédé suivant l'une quelconque des revendications 27 à 40, dans lequel ledit au moins un poly(oxyde d'éthylène) a, d'après des mesures rhéologiques, un poids moléculaire approximatif de 2 000 000 à 8 000 000 ou un poids moléculaire approximatif de 2 000 000, 4 000 000, 7 000 000 ou 8 000 000.

**42.** Forme posologique pharmaceutique orale solide à libération prolongée pouvant être obtenue par un procédé suivant l'une quelconque des revendications 27 à 41.

**43.** Forme posologique suivant l'une quelconque des revendications 1 à 26 et 42, pour une utilisation dans le traitement de la douleur, ladite forme posologique comprenant un analgésique opioïde.

Figure 1

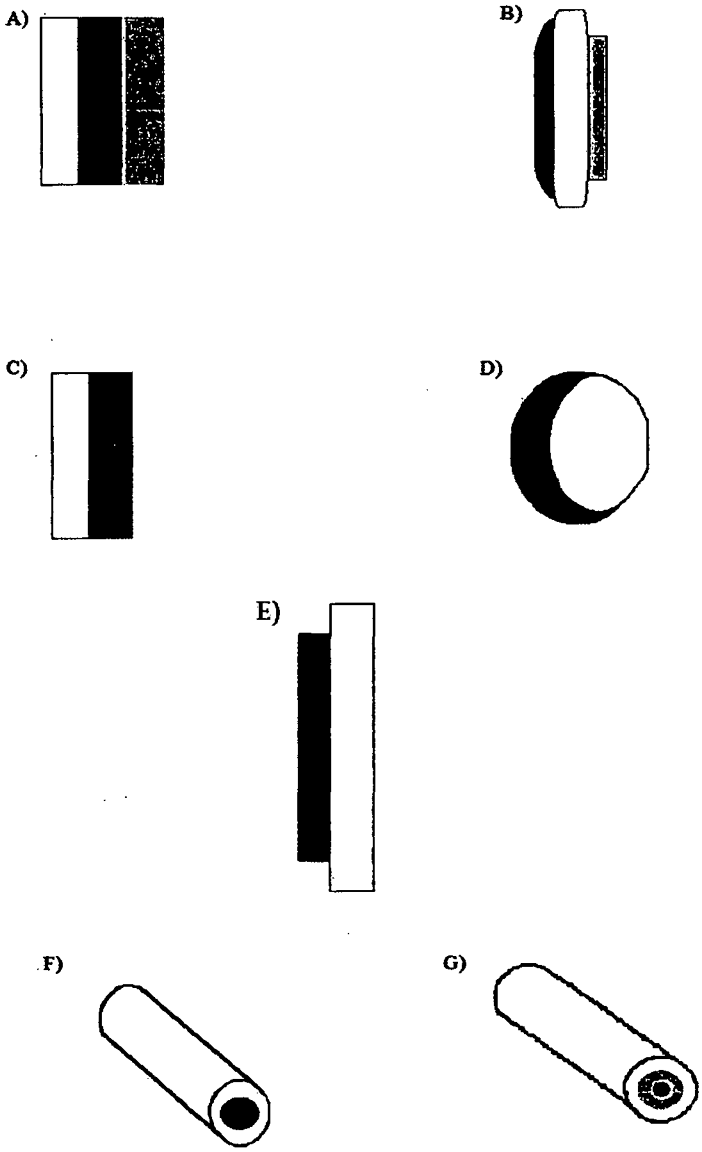

Figure 2

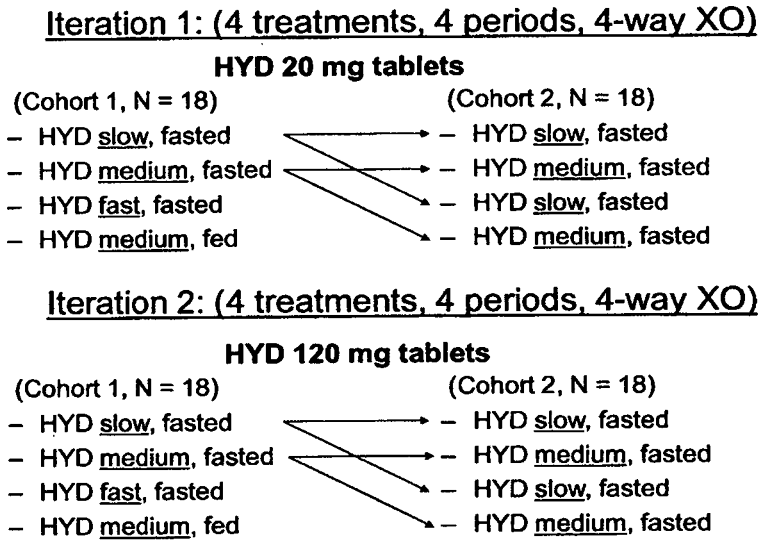
Iteration 1: (4 treatments, 4 periods, 4-way XO)
HYD 20 mg tablets
(Cohort 1, N = 18)
- HYD slow, fasted
- HYD medium, fasted
- HYD fast, fasted
- HYD medium, fed
(Cohort 2, N = 18)
- HYD slow, fasted
- HYD medium, fasted
- HYD slow, fasted
- HYD medium, fasted
Iteration 2: (4 treatments, 4 periods, 4-way XO)
HYD 120 mg tablets
(Cohort 1, N = 18)
- HYD slow, fasted
- HYD medium, fasted
- HYD fast, fasted
- HYD medium, fed
(Cohort 2, N = 18)
- HYD slow, fasted
- HYD medium, fasted
- HYD slow, fasted
- HYD medium, fasted

Figure 3

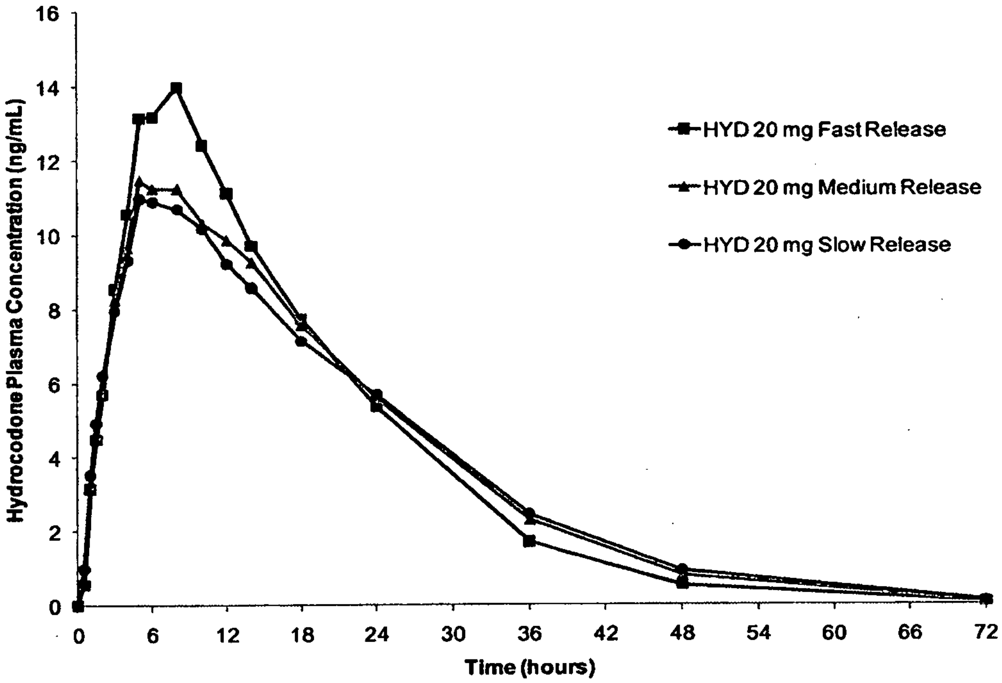

Hydrocodone Plasma Concentration (ng/mL)
16
14
12
10
8
6
4
2
0
0
6
12
18
24
30
36
42
48
54
60
66
72
Time (hours)
HYD 20 mg Fast Release
HYD 20 mg Medium Release
HYD 20 mg Slow Release

Figure 4

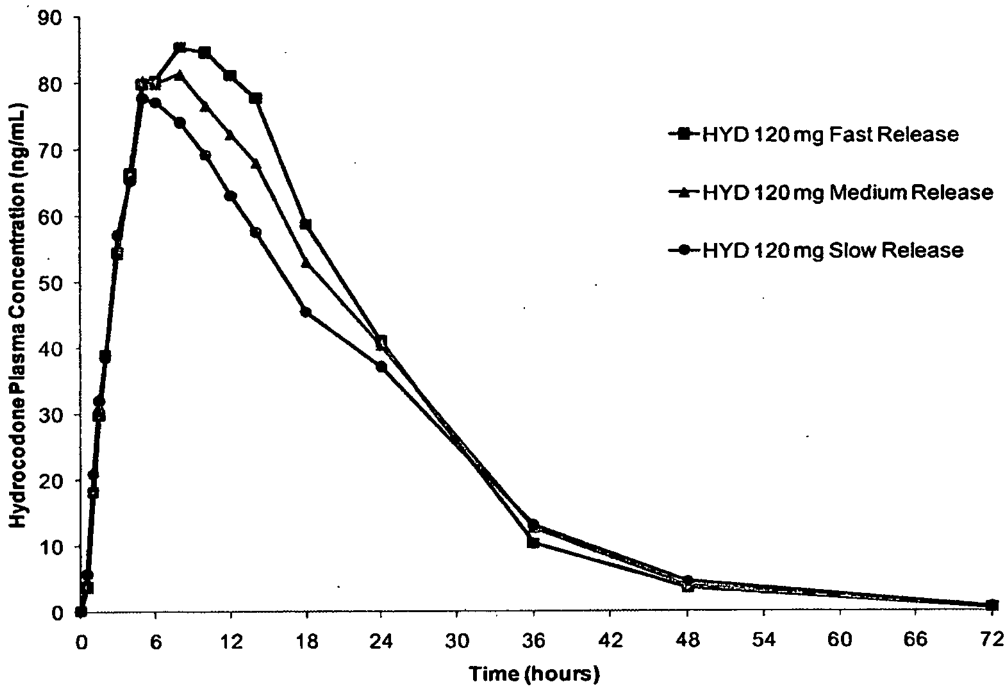
Hydrocodone Plasma Concentration (ng/mL)
90
80
70
60
50
40
30
20
10
0
0
6
12
18
24
30
36
42
48
54
60
66
72
Time (hours)
HYD 120 mg Fast Release
HYD 120 mg Medium Release
HYD 120 mg Slow Release

Figure 5

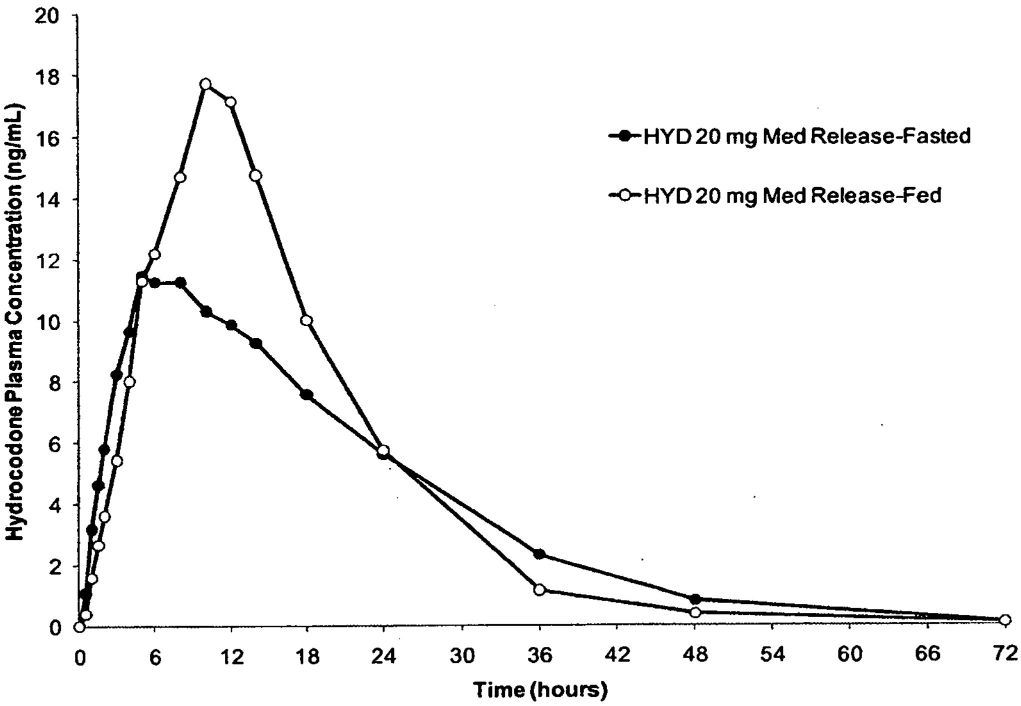
Hydrocodone Plasma Concentration (ng/mL)
20
18
16
14
12
10
8
6
4
2
0
HYD 20 mg Med Release-Fasted
HYD 20 mg Med Release-Fed
0
6
12
18
24
30
36
42
48
54
60
66
72
Time (hours)

Figure 6

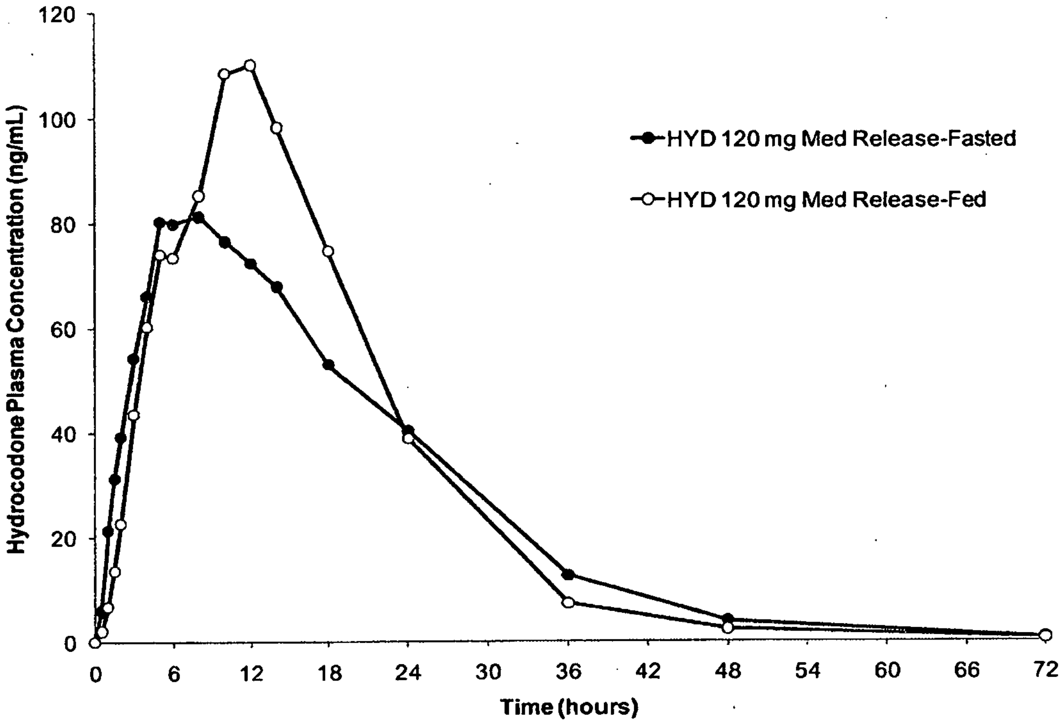
120
100
80
60
40
20
0
Hydrocodone Plasma Concentration (ng/mL)
HYD 120 mg Med Release-Fasted
HYD 120 mg Med Release-Fed
0
6
12
18
24
30
36
42
48
54
60
66
72
Time (hours)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 10056347 B
- US 11825938 B
- US 10056348 B
- US 11698394 B
- US 10057630 B
- US 10057632 B
- US 61467824 A


### Non-patent literature cited in the description


- Oral Solid Dosage Forms. Remington's Pharmaceutical Sciences. 1990, 1633-1665